Europäisches Patentamt
European Patent Office
Office européen des brevets

(19)

(11) Publication number: 0 375 451 A2

(12) # EUROPEAN PATENT APPLICATION

(21) Application number: 89313492.4

(22) Date of filing: 21.12.89

(51) Int. Cl.5: C07K 5/02, A61K 37/64

Claims for the following Contracting State: ES.

The title of the invention has been amended (Guidelines for Examination in the EPO, A-III, 7.3).

(30) Priority: 23.12.88 GB 8830220
06.04.89 GB 8907782
04.12.89 GB 8927210

(43) Date of publication of application:
27.06.90 Bulletin 90/26

(84) Designated Contracting States:
AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Ham, Peter Beecham Pharmaceuticals
Medicinal Res. Ctr. Coldharbour Road The Pinnacles
Fourth Avenue Harlow Essex CM19 5AD(GB)
Inventor: Smith, Stephen Allan Beecham Pharmaceuticals
Medicinal Res. Ctr. Coldharbour Road The Pinnacles
Fourth Avenue Harlow Essex CM19 5AD(GB)

(74) Representative: Jones, Pauline et al
Beecham Pharmaceuticals Patents & Trade Marks Dept. Great Burgh Yew Tree Bottom Road
Epsom Surrey KT18 5XQ(GB)

(54) **Compounds having a renin-inhibiting activity.**

(57) Compounds of formula (I) and pharmaceutically acceptable salts thereof:

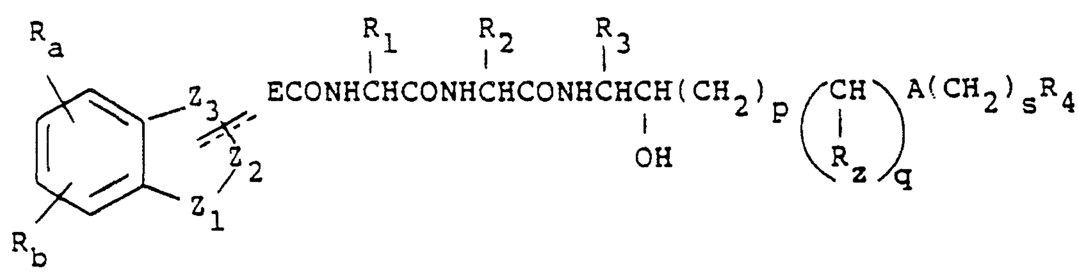

(I)

wherein
$Z_1$, $Z_2$, $Z_3$ and the carbon atoms to which $Z_1$ and $Z_3$ are attached, form a 5-membered non-aromatic heterocyclic ring;
E is absent or is $(CH_2)_n$ or $CH(CH_2)_{n-1}$ wherein n is 1 to 4;
A is -CONH-, -NHCO-, -COO- $-S(O)_r-$ wherein r is 0, 1, or 2, or $-CH_2-$;
p is 0, 1 or 2;
q is 0 or 1;
s is 0, 1, 2, 3 or 4;

EP 0 375 451 A2

$R_z$ is hydrogen, $C_{1-6}$ alkyl or, when A is $-CH_2-$, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl, $C_{3-8}$ cycloalkyl or phenyl; and
$R_4$ is a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and
the dashed line represents an optional bond (when E is present);
which are renin inhibitors, a process for their preparation and their use as pharmaceuticals.

# NOVEL COMPOUNDS

The invention relates to novel compounds having pharmacological activity, to processes for their preparation, to pharmaceutical preparations containing them and to their use in medicine.

Renin is a natural enzyme, disorders in relation to which are implicated in many cases of hypertension. It is released into the blood from the kidney, and cleaves from a blood glycoprotein a decapeptide known as angiotensin-I. Circulating angiotensin-I is cleaved in plasma, and in lung, kidney and other tissues to an octapeptide, angiotensin-II, which raises blood pressure both directly by causing arteriolar constriction and indirectly by stimulating release of the sodium-retaining hormone aldosterone from the adrenal gland and thus causing a rise in extracellular fluid volume. The latter effect is caused by angiotensin-II itself or a heptapeptide cleavage product angiotensin-III.

Inhibitors of renin have been described as useful in the treatment of hypertension.

A group of compounds has now been discovered, which inhibit the enzyme renin and therefore have potential blood pressure lowering activity, useful in the treatment of hypertension.

Compounds having an associated mechanism of action have also been described as possessing anti-retroviral activity and the present compounds may therefore be of potential use in the treatment of diseases caused by retroviruses including human immunodeficiency virus (HIV-1 and 2) and HTLV I and II. They may also be of potential use in the treatment of other cardiovascular disorders, such as congestive heart failure, and have beneficial effects on learning and memory and mood elevation activity, of potential use in the treatment of CNS disorders such as Alzheimer's disease and depression; they may also be of potential use in the treatment of glaucoma.

Accordingly, the present invention provides a compound of formula (I), or a pharmaceutically acceptable salt thereof:

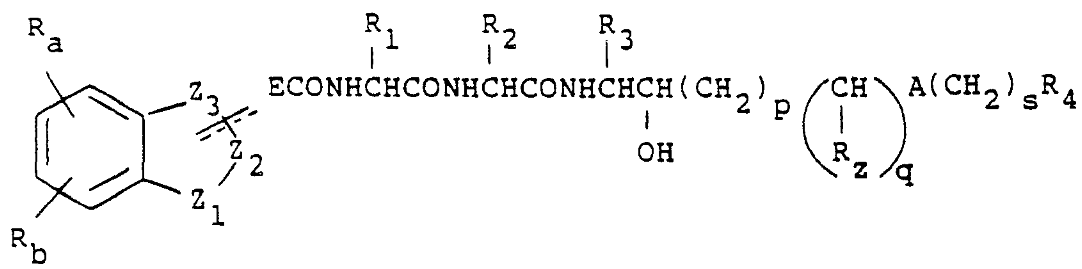

(I)

wherein

$Z_1$, $Z_2$, $Z_3$ and the carbon atoms to which $Z_1$ and $Z_3$ are attached, form a 5-membered non-aromatic heterocyclic ring;

E is absent or is $(CH_2)_n$ or $CH(CH_2)_{n-1}$ wherein n is 1 to 4;

A is -CONH-, -NHCO-, -COO- $-S(O)_r-$ wherein r is 0, 1, or 2, or $-CH_2-$;

p is 0, 1 or 2;

q is 0 or 1;

s is 0, 1, 2, 3 or 4;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;

$R_3$ is $CH_2R_{12}$ wherein $R_{12}$ is $C_{1-6}$ alkyl, $C_{3-8}$ cycloalkyl or phenyl; and

$R_4$ is a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and

the dashed line represents an optional bond (when E is present).

Values of $Z_1$, $Z_2$ and $Z_3$ include wherein one of $Z_1$, $Z_2$ and $Z_3$ is attached to E and is N, CH or C (wherein the optional exocyclic bond is present); the second of $Z_1$, $Z_2$ and $Z_3$ is O, S, SO, $SO_2$ or NR wherein R is hydrogen or $C_{1-6}$ alkyl; and the third is O, S, SO, $SO_2$, NR, $CH_2$ or C=O provided that, one of $Z_1$ and $Z_2$, or one of $Z_2$ and $Z_3$, is $CH_2$, CH or C when the other is S or O, or is $CH_2$, CH, C, NR or N when the other is SO or $SO_2$; and $Z_2$ is SO, $SO_2$ or C=O when one of $Z_1$ and $Z_3$ is N and the other is O, S or NR; or $Z_1$ is CO, $Z_2$ is O and $Z_3$ is CH.

Examples of $Z_1$, $Z_2$ and $Z_3$ therefore include

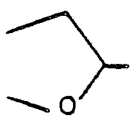 , 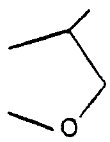 , 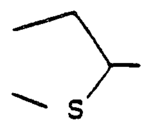 , 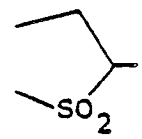 ,

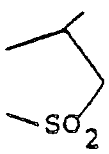 , 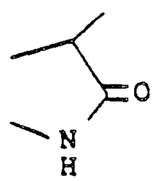 , 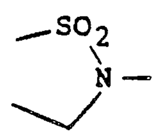 , 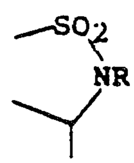 ,

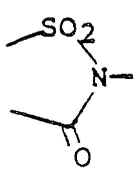 , 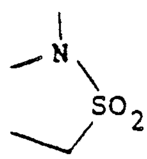 , 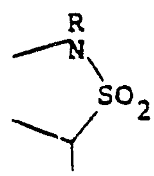 , 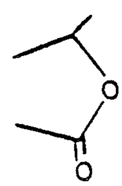 ,

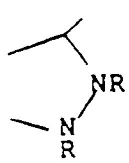 , 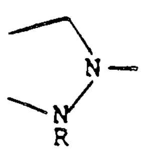 , 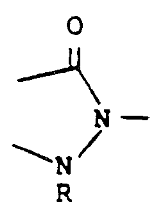 , 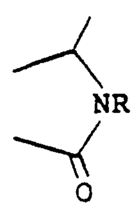 ,

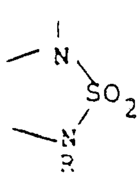 , 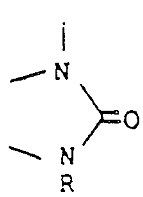 , 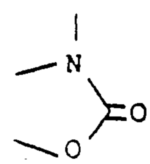 , 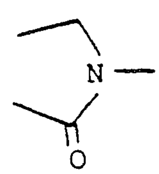

Preferably $Z_1$ is $SO_2$ or O, $Z_2$ is $CH_2$, $Z_3$ is CH and E is attached at $Z_3$; $Z_1$ is $SO_2$ or O, $Z_2$ is CH, $Z_3$ is $CH_2$ and E is attached at $Z_2$; or $Z_1$ is CO, $Z_2$ is N and $Z_3$ is CH and E is attached at $Z_2$.

Suitable examples of $R_a$ and $R_b$ include a moiety selected from hydrogen, halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or

$NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy.

Suitable examples of optionally substituted amino groups in $R_a/R_b$ then include one or two groups independently selected from $C_{1-6}$ alkyl or $C_{1-6}$ alkyl substituted by carboxy, $C_{1-6}$ alkoxycarbonyl, hydroxy or amino group.

Alternatively, $NR_5R_6$ or other substituted amino groups in $R_a/R_b$ may be pyrrolidinyl, piperidinyl, morpholinyl or piperazinyl optionally N-substituted by $C_{1-6}$ alkyl or an N-oxide thereof.

Preferably one of $R_a$ and $R_b$ is hydrogen and the other is $CH_2NH_2$, $CO_2H$, $CH_2NHCH_2CO_2H$ or $SO_2CH_2CO_2H$.

Suitable acyl groups in $R_7$ when $C_{1-6}$ alkyl substituted by acylamino, include $C_{1-7}$ alkanoyl, or optionally substituted benzoyl. Suitable protecting groups in protected amino include benzyloxycarbonyl and t-butyloxycarbonyl.

In regard to values of A and p and q, reference is hereby made to the following literature:-

1. J. Med. Chem. 1988, 31, 1918
(p = 1, q = 0, A = -CONH-)
2. J. Med. Chem. 1987, 30, 1224
(p = 2, q = 0, A = -NHCO-)
3. J. Med. Chem. 1987, 31, 701
(p = 0, q = 0, A = -COO-)
4. J. Med. Chem. 1987, 31, 1839
(p = 1, q = 1, A = -CONH-)
5. J. Med. Chem. 1987, 30, 1729 and refs. therein
(p = 2, q = 1, A = $S(O)_r$)
6. WO 88/05050
(p = 0, q = 1, A = $CH_2$, $R_z$ = OH)
7. EP-A-172346
(p = 0, q = 1, A = $CH_2$, $R_z$ = H)

Suitable values of $R_9$ and $R_{11}$ when aryl include phenyl or naphthyl and when heteroaryl, include a 5- or 6-membered monocyclic or 9- or 10- membered bicyclic of which 5- or 6- membered monocyclic heteroaryl is preferred. In addition, 5- or 6-membered monocyclic or 9- or 10-membered bicyclic heteroaryl preferably contains one, two or three heteroatoms which are independently selected from oxygen, nitrogen and sulphur and which, in the case of there being more than one heteroatom, are the same or different. Examples of 5- or 6-membered monocyclic heteroaryl containing one, two or three heteroatoms which are selected from the class of oxygen, nitrogen and sulphur include furanyl, thienyl, pyrryl, oxazolyl, thiazolyl, imidazolyl and thiadiazolyl, and pyridyl, pyridazyl, pyrimidyl, pyrazolyl and triazolyl. Preferred examples of such groups include furanyl, thienyl, pyrryl and pyridyl, in particular 2- and 3-furyl, 2- and 3-pyrryl, 2- and 3-thienyl, and 2-, 3- and 4-pyridyl. Examples of 9- or 10-membered bicyclic heteroaryl containing one, two or three heteroatoms which are selected from the class of oxygen, nitrogen and sulphur include benzofuranyl, benzothienyl, indolyl and indazolyl, quinolyl and isoquinolyl, and quinazolyl. Examples of such groups include 2- and 3-benzofuranyl, 2- and 3-benzothienyl, and 2- and 3-indolyl, and 2- and 3-quinolyl and, for $R_{11}$, 4-benzimidazolyl.

Suitable examples of groups or atoms for optional substitution of $R_5$ when benzyl and $R_9$ and $R_{11}$ include one, two or three substituents independently selected from $C_{1-4}$ alkyl, $C_{1-4}$ alkoxy, halo (such as fluoro, chloro, bromo), hydroxy, nitro, and cyano.

Preferred examples of $R_9$ include phenyl and naphthyl, and preferred examples of $R_{11}$ when aryl or heteroaryl include phenyl, imidazol-4-yl and -2-yl, pyrazol-1-yl and 4-methylpyrazol-1-yl.

Suitable examples of $R_4$ when monocyclic heteroaryl include those listed for $R_9$ and $R_{11}$, preferably 3-pyridyl or an N-oxide thereof; or 1-imidazolyl.

When $R_4$ is a saturated heterocyclic ring, suitable examples include piperidinyl, pyrrolidinyl, piperazinyl and morpholinyl; optionally N-substituted by $C_{1-6}$ alkyl group(s) or as an N-oxide thereof; or $R_4$ may be an N-linked amino acid, such as proline.

Preferably the amino acid residue containing $R_2$ is Leu, $\beta$-pyrazolylalanine or His.

There is a group of compounds within formula (I)
wherein
E is $(CH_2)_n$ wherein n is 0, 1 or 2;
$Z_1$ is O, S, SO, $SO_2$ or NR wherein R is hydrogen or $C_{1-6}$ alkyl;

$Z_2$ is $CR_xR_y$ wherein $R_x$ is hydrogen and $R_y$ is hydrogen or $R_y$ is attached to E in formula (I), or $R_x$ and $R_y$ together are an oxo group;
$Z_3$ is $CH_2$ or is CH attached to E in formula (I);
$R_{11}$ is $C_{1-6}$ alkyl, $C_{3-8}$ cycloalkyl, or optionally substituted aryl or heteroaryl; and
$R_4$ is a saturated or unsaturated heterocyclic ring linked through carbon, hydroxy, $C_{1-6}$ alkoxy, $C_{1-6}$ alkanoyloxy, amino, $C_{1-7}$ alkanoylamino, amino substituted by one or two $C_{1-6}$ alkyl groups, or $C_{1-6}$ alkylsulphonyl, or (when s is 2 to 4) $R_4$ is a saturated or unsaturated heterocyclic ring linked through nitrogen; and
the remaining variables are as defined in formula (I).

Abbreviations used herein may be as follows:

| Amino Acid Residue | Abbreviations |
|---|---|
| histidine | His |
| isoleucine | Ile |
| leucine | Leu |
| 1-naphthylalanine | NAla |
| norleucine | Nle |
| phenylalanine | Phe |

The "-amino acid components are in the (S)-configuration (or L-form).

In a particular aspect, the present invention provides a compound of formula (IA) or a pharmaceutically acceptable salt thereof:

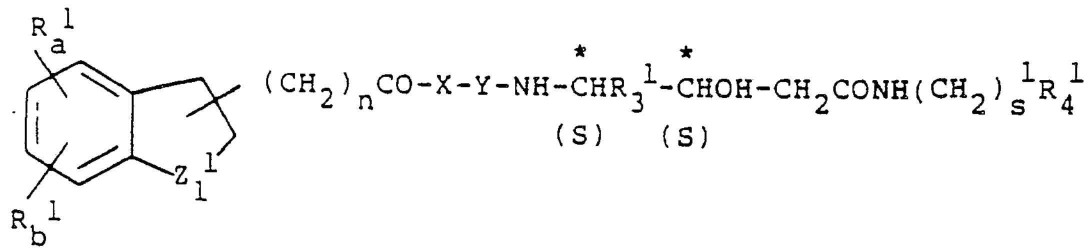

(IA)

wherein
X is Phe or NAla;
Y is Leu, Ile, Nle or His;
$Z_1{}^1$ is O, S, SO or $SO_2$;
n is 0, 1, 2 or 3;
$s^1$ is 2 or 3;
$R_a{}^1$ and $R_b{}^1$ are independently selected from hydrogen, and a moiety as listed hereinbefore for suitable examples of $R_a$ and $R_b$;
$R_3{}^1$ is cyclohexylmethyl; and
$R_4{}^1$ is 1-imidazolyl or 4-morpholinyl.

Often, in formula (IA), X is Phe and Y is Leu.

Suitable values for alkyl groups in $R_a$, $R_b$, $R_z$, R and $R_2$ to $R_{12}$ in formulae (I) and (IA) include methyl, ethyl, n- and iso-propyl, n-, sec-, iso- and tert-butyl, n-, iso-, sec-, tert- and neo-pentyl. $C_{3-8}$ cycloalkyl groups are cyclopropyl, cyclobutyl, cyclopentyl, cyclohexyl, cycloheptyl and cyclooctyl.

Suitable values for $R_a$ and $R_b$ halo include fluoro, chloro, bromo and iodo, preferably chloro or bromo.

The right hand sequence in formula (I) and (IA) is preferably 4(S)-amino-5-cyclohexyl-3-(S)-hydroxypentanoic acid (ACHPA), from the amino acid containing $R_3$ or $R_3{}^1$ to $NH(CH_2)_sR_4$.

Preferably n is 1, 2 or 3.

Preferably E, including the $(CH_2)_nCO$- linkage, is attached at the 3-position with respect to $Z_1$.

Pharmaceutically acceptable salts include acid addition salts which may be, for example, salts with inorganic acids such, for example, as hydrochloric acid, hydrobromic acid, orthophosphoric acid or

sulphuric acid, or organic acids such, for example, as methanesulphonic acid, toluenesulphonic acid, acetic acid, trifluoroacetic acid, propionic acid, lactic acid, citric acid, tartaric acid, fumaric acid, malic acid, succinic acid, salicylic acid or acetylsalicylic acid.

Pharmaceutically acceptable salts may also include alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine.

It will be appreciated that the compounds of the invention may exist as solvates, such as hydrates, and these are included whenever, a compound of formula (I) or a salt thereof, is herein referred to.

The compounds of formula (I) wherein E is attached to $Z_1/Z_2/Z_3$ when CH, and (IA) have at least one asymmetric centre in addition to those attached to $R_1$ and $R_2$ (in X and Y) and those indicated in formula (IA), and are therefore capable of existing in more than one stereoisomeric form. The invention extends to each of these forms and to mixtures thereof. Preferably, the configuration at the carbon atoms bearing $R_1$, $R_2$ and $R_3$ is the (S)-configuration.

It will be appreciated that, when the optional bond depicted in formula (I) is present, the compounds are capable of existing in E and Z (trans and cis) forms. The invention extends to each of these forms and to mixtures thereof.

The compounds of the invention are preferably in pharmaceutically acceptable form. By pharmaceutically acceptable form is meant, inter alia, of a pharmaceutically acceptable level of purity excluding normal pharmaceutical additives such as diluents and carriers, and including no material considered toxic at normal dosage levels. A pharmaceutically acceptable level of purity will generally be at least 50% excluding normal pharmaceutical additives, preferably 75%, more preferably 90% and still more preferably 95%.

A compound of the invention may be prepared by those methods known in the art for the synthesis of compounds of analogous structure, such as peptides, and in this regard reference is made, by way of illustration only, to the following literature reference: S.R. Pettit, "Synthetic Peptides", (Elsevier Scientific Publishing Co. 1976).

The present invention also provides a compound of the present invention which has been prepared synthetically.

A compound of the present invention may, for example, be formed by the sequential coupling of appropriate amino acids with the acid of formula (II):

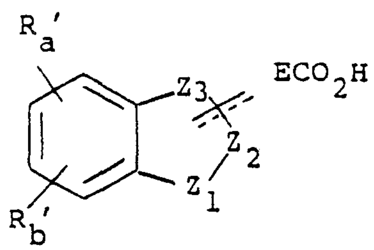

(II)

wherein $R_a'$ and $R_b'$ are $R_a$ and $R_b$ respectively or groups or atoms convertible thereto; or by the initial preparation and subsequent coupling of peptide subunits with the acid of formula (II), the subunits themselves prepared in stepwise manner; in either case classical solution chemistry methods analogous to those used in peptide synthesis, may be employed.

The coupling reactions may be effected by, for example, activating the reacting carboxyl group of the ingoing acid of formula (II) or amino acid, and reacting this with the amino group of the substrate unit. Details of suitable, optional activating and protecting (masking) groups and of suitable reaction conditions (for the coupling reactions and for the introduction and removal of protecting groups) giving, preferably, the minimum of racemisation, may be found in the above-referenced literature.

It will be appreciated that, when A is other than -CONH- or -COO-, the C-terminus group is other than an amino acid, in which case the coupling is carried out with an appropriate other precursor, as described in the aforementioned literature references 2., 5., 6. and 7.

Accordingly, the present invention further provides a process for the preparation of a compound of formula (I) or a pharmaceutically acceptable salt thereof which comprises reacting a reagent of the formula (III):

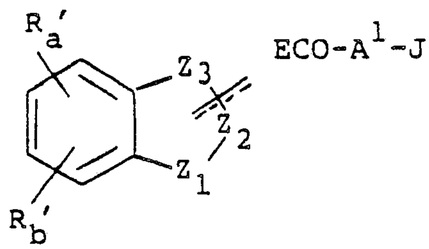

(III)

wherein $R_a'$ and $R_b'$ are $R_a$ and $R_b$ respectively or groups or atoms convertible thereto; $A^1$ is absent or represents an appropriate amino acid or dipeptide unit, J is OH or a leaving group; and the remaining variables are as hereinbefore defined; with a reagent of the formula (IV):

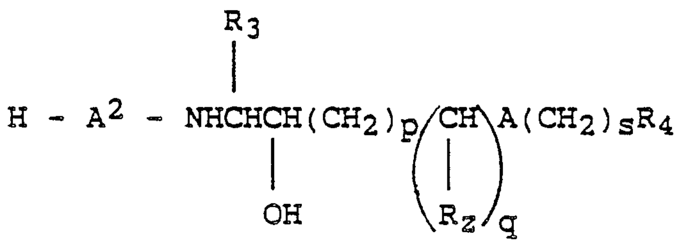

(IV)

wherein
$A^2$ is absent or represents an appropriate amino acid or dipeptide unit such that $A^1$ + $A^2$ is $-NHCHR_1CONHCHR_2CO-$and the remaining variables are as hereinbefore defined; and thereafter, if desired or necessary deprotecting (within $A^1$ or $A^2$) of the products, and/or converting $Z_1$, $Z_2$ or $Z_3$ to other $Z_1$, $Z_2$ or $Z_3$, $R_a'/R_b'$ to $R_a$ and/or $R_b$ and/or forming a pharmaceutically acceptable salt thereof.

Suitable examples of J when a leaving group include halo, such as chloro or bromo, and other suitable groups which are displaceable by an amino nucleophile, such as $C_{1-6}$ alkoxycarbonyloxy.

It is generally preferred, especially when $A^1$ is not absent, however, that J is OH, and a suitable coupling reagent or dehydrating catalyst is used to effect the reaction, usually carbodiimides such as dicyclohexylcarbodiimide and especially those described in the Descriptions and Examples hereinafter.

Deprotection of $A^1$ and/or $A^2$ takes place conventionally, in accordance with the particular protecting group(s) employed.

Pharmaceutically acceptable salts may be formed conventionally.

$Z_1$, $Z_2$ or $Z_3$ when S or SO may be converted to SO or $SO_2$ respectively (or S to $SO_2$) by conventional oxidation methods, such as those described hereinafter for conversion (vi) for $R_a/R_b$.

It will be apparent that compounds of the formula (I) containing an $R_a'/R_b'$ group which is other than $R_a/R_b$ and which is convertible to an $R_a/R_b$ group, are useful novel intermediates. A number of such conversions is possible, not only for the final product compounds of formula (I) when $R_a'/R_b'$ are other than $R_a/R_b$, but also within $R_a/R_b$, and also for their intermediates as follows:

(i) a hydrogen substituent is convertible to a nitro substituent by nitration;

(ii) a nitro substituent is convertible to an amino substituent by reduction;

(iii) a $C_{1-7}$ acylamino substituent is convertible to an amino substituent by deacylation;

(iv) an amino substituent is convertible to a $C_{1-7}$ acylamino substituent by acylation with a carboxylic acid derivative;

(v) a hydrogen substituent is convertible to a halogen substituent by halogenation;

(vi) an $C_{1-6}$ alkylthio or a $C_{1-6}$ alkylsulphinyl substituent is convertible to a $C_{1-6}$ alkylsulphinyl or $C_{1-6}$ alkylsulphonyl substituent respectively, by oxidation;

(vii) an amino, aminosulphonyl, or $NHCONH_2$ substituent is convertible to a corresponding substituent which is substituted by one or two alkyl groups, by N-alkylation;

(viii) an amino substituent is convertible to a group $NHCONH_2$, by reaction with potassium cyanate and acid;

(ix) a hydrogen substituent is convertible to an aminosulphonyl substituent by treatment with $ClSO_3H$ followed by $HNR_5R_6$.

(x) a cyano substituent may be converted to an aminomethyl substituent by reduction.

(xi) a bromo substituent may be converted to a cyano substituent by reaction with copper (I) cyanide.

Conversions (i) to (xi) are only exemplary and are not exhaustive of the possibilities. It will be appreciated that it is often desirable to carry out these conversions at an earlier stage, in the intermediate acid of formula (II), especially in regard to conversion (iii).

In regard to (i), nitration is carried out in accordance with known procedures.

In regard to (ii), the reduction is carried out with a reagent suitable for reducing nitroanisole to aminoanisole.

In regard to (iii), deacylation is carried out by treatment with a base, such as an alkali metal hydroxide.

In regard to (iv), the acylation is carried out with an acylating agent, such as the corresponding acid or acid chloride. Formylation is carried out with the free acid.

In regard to (v), halogenation is carried out with conventional halogenating agents.

In regard to (vi), oxidation is carried out at below ambient temperatures in a non-aqueous solvent, such as a chlorinated hydrocarbon, in the presence of an organic peracid, such as 3-chloroperoxybenzoic acid, or in water in the presence of a soluble strong inorganic oxidant, such as an alkali metal permanganate or in aqueous hydrogen peroxide.

In regard to (vii), alkylation is carried out with a corresponding alkylating agent such as the chloride or bromide under conventional conditions, or in the case of amino, reductive amination is suitable.

In regard to (viii), conversion to a ureido derivative is carried out by reaction with potassium cyanate in methanol, at ambient temperature.

In regard to (ix), the mixing with $ClSO_3H$ takes place at low temperatures around 0°C and allowed to warm to ambient temperature. The subsequent reaction with the amine takes place at ambient temperature, in a solvent such as ethanol.

In regard to (x), the reduction takes place by reaction with sodium borohydride/cobalt chloride as described in Tetrahedron Letters 1969 52 pp 4555-58; or using platinum oxide/hydrogen.

In regard to (xi), the reaction takes place under conventional conditions.

Compounds of the general formulae (II) and (III) may themselves be prepared by standard techniques analogous to those described above.

The acids of formula (II) are either known compounds or are prepared by analogous methods to these and for structurally similar known compounds, for example, as in the Descriptions hereinafter; or in EP-A-350163 (Beecham Group p.l.c.).

Acids of formula (II) wherein E is attached to $Z_1/Z_2/Z_3$ when CH and n is 1-4 may be prepared from the corresponding acids wherein n is n-1, from the corresponding malonic acid derivative, which may then be converted to the corresponding n derivative by conventional methods of homologation, such as the Grignard and nitrile methods.

Acids of formula (II) wherein E is attached to $Z_1/Z_2/Z_3$ when N, and n is 1, 2 or 3, may be prepared by conventional nucleophilic substitution, employing the corresponding $Z_1/Z_2/Z_3$ derivative when NH.

The preparation of the amino acid of formula (V):

$$H_2N-\overset{*}{C}HR_3{}^1-\overset{*}{C}HOH-CH_2CO_2H \qquad (V)$$

(S) (S)

is described in J. Med. Chem 1985, 28, 1779-1790.

As regards the appropriate intermediates wherein A is other than -CONH-, reference is hereby made to the literature references 2., 3., 5., 6. and 7 listed hereinbefore.

It will be appreciated that protected forms of compounds of the present invention are novel intermediates and form an aspect of the invention.

Particularly suitable methods of preparation of the compounds of the present invention are as described in the Descriptions and Examples hereinafter. The couplings may be carried out sequentially beginning by coupling the acid of formula (II) with, for example, phenylalanine or 1-naphthylalanine, followed by coupling with Y, for example, leucine or histidine, and finally, coupling with the amino acid of formula (V). In a preferred aspect, however, either the acid of formula (II) is coupled with a tripeptide unit formed between the amino acid of formula (V); leucine, histidine or other $R_2$ containing amino acid; and phenylalanine or naphthylalanine; or alternatively the acid of formula (II) is coupled with phenylalanine or naphthylalanine and this is coupled with a dipeptide unit formed between the $R_2$ containing amino acid and the amino acid of formula (V).

As mentioned previously the compounds of the invention have been found to be renin inhibitors, and therefore they are of potential use in the treatment of hypertension. They may also be of potential use in the other diseases and disorders hereinbefore referred to.

The present invention also provides a pharmaceutical composition which comprises a compound of formula (I) or a pharmaceutically acceptable salt thereof, and a pharmaceutically acceptable carrier. In particular, the present invention provides an anti-hypertensive pharmaceutical composition which comprises an anti-hypertensive effective amount of a compound of formula (I) or a pharmaceutically acceptable salt thereof, and a pharmaceutically acceptable carrier.

The compositions are preferably adapted for oral administration. However, they may be adapted for other modes of administration, for example parenteral administration for patients suffering from heart failure.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot beaccomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

The present invention further provides a method of prophylaxis or treatment of hypertension in mammals including man, which comprises administering to the suffering mammal an anti-hypertensively effective amount of a compound of formula (I) or a pharmaceutically acceptable salt thereof.

An effective amount will depend on the relative efficacy of the compounds of the present invention, the severity of the hypertension being treated and the weight of the sufferer. However, a unit dose form of a composition of the invention may contain from 0.1 to 500 mg of a compound of the invention and more usually from 1 to 100 mg, for example 2 to 50 mg such as 2, 3, 4, 5, 10, 20 or 30mg. Such compositions may be administered from 1 to 6 times a day, more usually from 2 to 4 times a day, in a manner such that the daily dose is from 1 to 1000mg for a 70 kg human adult and more particularly from 5 to 500 mg.

No toxicological effects are indicated at the aforementioned dosage ranges.

The present invention further provides a compound of formula (I) or a pharmaceutically acceptable salt thereof for use in the treatment or prophylaxis of hypertension.

The following tabulations and structures show the compounds and intermediates which are prepared.

The following Descriptions relate to the preparation of intermediates and the following Examples relate to the preparation of compounds of the invention.

In the following, the abbreviations used are:

| Abbreviation | Definition |
|---|---|
| ACHPAA | 4 (S)-amino-5-cyclohexyl-3 (S)-hydroxypentanoic acid isobutylamide |
| ACHPA | 4 (S)-amino-5-cyclohexyl-3 (S)-hydroxypentanoic acid |
| BOC | tert-butoxycarbonyl |
| CBZ | benzyloxycarbonyl |
| Phth | phthaloyl |
| Me | methyl |
| Et | ethyl |
| Ph | phenyl |
| GABA | gamma-aminobutyric acid |
| Pro | proline |
| TFA | trifluoroacetate |

TABLE 1

$$R_p\text{-}N(R_q)\text{-}(CH_2)_n\text{-}R_r$$

| Compound No. | $R_p$ | $R_q$ | n | $R_r$ | salt/solvate |
|---|---|---|---|---|---|
| D1(a) | H | BOC-ACHPA | 3 | 1-imidazolyl | |
| D1(b) | H | CBZ-PheLeu-ACHPA | 3 | 1-imidazolyl | $0.5H_2O$ |
| D2(a) | H | BOC-ACHPA | 2 | 4-morpholinyl | |
| D2(b) | H | CBZ-PheLeu-ACHPA | 2 | 4-morpholinyl | $0.5H_2O$ |
| D3(a) | H | BOC-ACHPA | 3 | 2-oxopyrrolidin-1-yl | |
| D3(b) | H | CBZ-PheLeu-ACHPA | 3 | 2-oxopyrrolidin-1-yl | $0.5H_2O$ |
| D4(a) | H | BOC-ACHPA | 2 | 2-pyridyl | |
| D4(b) | H | CBZ-PheLeu-ACHPA | 2 | 2-pyridyl | $0.5H_2O$ |
| D5(a) | H | BOC-ACHPA | 2 | OH | |
| D5(b) | H | CBZ-PheLeu-ACHPA | 2 | OH | |
| D6 | H | BOC-ACHPA | 2 | $NHCOCH_3$ | |
| D7 | H | BOC-ACHPA | 3 | $CO_2Et$ | |
| D8(a) | H | BOC | 3 | (S)-$CH(CO_2CH_2Ph)NH$-CBZ | |
| D8(b) | H | BOC-ACHPA | 3 | (S)-$CH(CO_2CH_2Ph)NH$-CBZ | |

TABLE 1 (Contd.)

$$R_p-N(R_q)-(CH_2)_n-R_r$$

| Compound No. | $R_p$ | $R_q$ | n | $R_r$ | salt/solvate |
|---|---|---|---|---|---|
| D9(a) | H | BOC | 4 | (S)-CH($CO_2CH_2Ph$)NH-CBZ | |
| D9(b) | H | BOC-ACHPA | 4 | (S)-CH($CO_2CH_2Ph$)NH-CBZ | |
| D12(a) | H | CBZ-PheLeu-ACHPA | 3 | $CO_2Et$ | |
| D12(b) | H | PheLeu-ACHPA | 3 | $CO_2Et$ | HCl |
| D13(a) | H | BOC-PheLeu-ACHPA | 3 | 1-imidazolyl | |
| D13(b) | H | PheLeu-ACHPA | 3 | 1-imidazolyl | 2TFA |
| D14(a) | Phth | | 2 | 1-imidazolyl | |
| D14(b) | H | H | 2 | 1-imidazolyl | 2HCl |
| D14(c) | H | BOC-ACHPA | 2 | 1-imidazolyl | |
| D15(a) | Phth | | 4 | 1-imidazolyl | |
| D15(b) | H | H | 4 | 1-imidazolyl | 2HCl |
| D15(c) | H | BOC-ACHPA | 4 | 1-imidazolyl | |
| D17(a) | H | H | 4 | $CO_2Me$ | HCl |
| D17(b) | H | BOC-ACHPA | 4 | $CO_2Me$ | |

<u>TABLE 1</u> (Contd.)

$$R_p-\overset{\displaystyle R_q}{\overset{|}{N}}-(CH_2)_n-R_r$$

<table>
<tr><th>Compound No.</th><th>Rp</th><th>Rq</th><th>n</th><th>Rr</th><th>salt/solvate</th></tr>
<tr><td>D18(a)</td><td colspan="2">Phth</td><td>4</td><td>4-methylpiperazin-1-yl</td><td></td></tr>
<tr><td>D18(b)</td><td>H</td><td>H</td><td>4</td><td>4-methylpiperazin-1-yl</td><td>3HCl</td></tr>
<tr><td>D18(c)</td><td></td><td>BOC-ACHPA</td><td>4</td><td>4-methylpiperazin-1-yl</td><td></td></tr>
<tr><td>D19(a)</td><td colspan="2">Phth</td><td>3</td><td>4-methylpiperazin-1-yl</td><td></td></tr>
<tr><td>D19(b)</td><td>H</td><td>H</td><td>3</td><td>4-methylpiperazin-1-yl</td><td>3HCl</td></tr>
<tr><td>D19(c)</td><td></td><td>BOC-ACHPA</td><td>3</td><td>4-methylpiperazin-1-yl</td><td></td></tr>
<tr><td>D20(a)</td><td colspan="2">Phth</td><td>2</td><td>4-methylpiperazin-1-yl</td><td></td></tr>
<tr><td>D20(b)</td><td>H</td><td>H</td><td>2</td><td>4-methylpiperazin-1-yl</td><td>3HCl</td></tr>
<tr><td>D20(c)</td><td>H</td><td>BOC-ACHPA</td><td>2</td><td>4-methylpiperazin-1-yl</td><td></td></tr>
<tr><td>D21</td><td>H</td><td>BOC-ACHPA</td><td>3</td><td>$NMe_2$</td><td></td></tr>
<tr><td>D23(a)</td><td>H</td><td>BOC</td><td>3</td><td>$CO_2H$</td><td></td></tr>
<tr><td>D23(b)</td><td>H</td><td>BOC</td><td>3</td><td>$CO_2CH_2Ph$</td><td></td></tr>
<tr><td>D23(c)</td><td>H</td><td>BOC-ACHPA</td><td>3</td><td>$CO_2CH_2Ph$</td><td></td></tr>
<tr><td>D23(d)</td><td>H</td><td>H-ACHPA</td><td>3</td><td>$CO_2CH_2Ph$</td><td>TFA</td></tr>
<tr><td>D23(e)</td><td>H</td><td>BOC-PheLeu-ACHPA</td><td>3</td><td>$CO_2CH_2Ph$</td><td></td></tr>
</table>

TABLE 1 (Contd.)

$$R_p-N(R_q)-(CH_2)_n-R_r$$

| Compound No. | $R_p$ | $R_q$ | n | $R_r$ | salt/solvate |
|---|---|---|---|---|---|
| D24(a) | H | BOC | 2 | $CO_2H$ | |
| D24(b) | H | BOC | 2 | $CO_2CH_2Ph$ | |
| D24(c) | H | BOC-ACHPA | 2 | $CO_2CH_2Ph$ | |
| D25(a) | H | BOC | 2 | (S)-$CH(CO_2H)$NH-CBZ | |
| D25(b) | H | BOC | 2 | (S)-$CH(CO_2CH_2Ph)$NH-CBZ | |
| D25(c) | H | BOC-ACHPA | 2 | (S)-$CH(CO_2CH_2Ph)$NH-CBZ | |
| D26 | H | PheLeu-ACHPA | 3 | 1-imidazolyl | |
| D33(a) | —— | Phth —— | 3 | $CO_2Et$ | |
| D34(a) | —— | Phth —— | 3 | Pro-OMe | |
| D34(b) | H | H | 3 | Pro-OMe | 2HCl |
| D34(c) | H | BOC-ACHPA | 3 | Pro-OMe | |
| D35(a) | H | H | 2 | 3-pyridyl | |
| D35(b) | H | BOC-ACHPA | 2 | 3-pyridyl | |
| D36(a) | H | H | 2 | 4-pyridyl | 2TFA |
| D36(b) | H | BOC-ACHPA | 2 | 4-pyridyl | |

**TABLE 2**

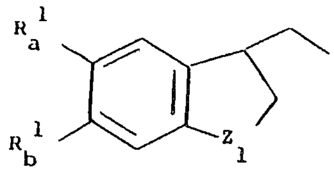 CO-Phe-Y-ACHPA-NH-$(CH_2)_n$-$R_s$

| Compound No. | $R_a^1$ | $R_b^1$ | $Z_1$ | Y | n | $R_s$ | salt/solvate |
|---|---|---|---|---|---|---|---|
| D8(c) | H | H | $SO_2$ | Leu | 3 | (S)-CH($CO_2CH_2Ph$)NH-CBZ | $0.5H_2O$ |
| D9(c) | H | H | $SO_2$ | Leu | 4 | (S)-CH($CO_2CH_2Ph$)NH-CBZ | $H_2O$ |
| D25(d) | H | H | $SO_2$ | Leu | 2 | (S)-CH($CO_2CH_2Ph$)NH-CBZ | |
| D31(f) | $MeO_2CCH_2N(BOC)CH_2$ | H | O | Leu | 3 | 1-imidazolyl | |
| D31(g) | $HO_2CCH_2N(BOC)CH_2$ | H | O | Leu | 3 | 1-imidazolyl | |
| E1(a) | H | H | $SO_2$ | Leu | 3 | 1-imidazolyl | $H_2O$ |
| E1(b) | H | H | $SO_2$ | Leu | 3 | 1-imidazolyl | HCl |
| E2 | H | H | $SO_2$ | Leu | 2 | 4-morpholinyl | $H_2O$ |
| E3 | H | H | $SO_2$ | Leu | 3 | 2-oxopyrrolidin-1-yl | $1.5H_2O$ |
| E4 | H | H | $SO_2$ | Leu | 2 | 2-pyridyl | $H_2O$ |
| E5 | H | H | $SO_2$ | Leu | 2 | OH | $H_2O$ |
| E6 | H | H | $SO_2$ | Leu | 2 | $NHCOCH_3$ | $2H_2O$ |

TABLE 2 (Contd.)

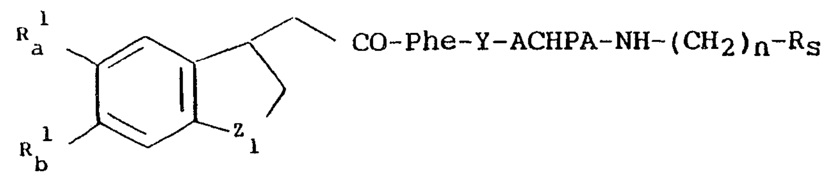

| Compound No. | $R_a^1$ | $R_b^1$ | $Z_1$ | Y | n | $R_S$ | salt/solvate |
|---|---|---|---|---|---|---|---|
| E7 | H | H | $SO_2$ | Leu | 3 | $CO_2Et$ | |
| E8(a) | H | H | $SO_2$ | Leu | 3 | $CO_2H$ | $1.5H_2O$ |
| E8(b) | H | H | $SO_2$ | Leu | 3 | $CO_2Na$ | |
| E9 | H | H | $SO_2$ | Leu | 3 | (S)-$CH(NH_2)CO_2H$ | $3H_2O.HCl$ |
| E10 | H | H | $SO_2$ | Leu | 4 | (S)-$CH(NH_2)CO_2H$ | $2H_2O.HCl$ |
| E11 | H | BOC-$NHCH_2$ | $SO_2$ | Leu | 3 | $CO_2Et$ | |
| E12 | BOC-$NHCH_2$ | H | O | Leu | 3 | 1-imidazolyl | $0.5H_2O$ |
| E13 | $H_2NCH_2$ | H | O | Leu | 3 | 1-imidazolyl | $H_2O.2TFA$ |
| E14 | H | H | $SO_2$ | Leu | 4 | 1-imidazolyl | $2H_2O.HCl$ |
| E15 | H | H | $SO_2$ | Leu | 2 | 1-imidazolyl | $2H_2O.HCl$ |
| E16 | $PhCH_2O_2C$ | H | O | Leu | 3 | 1-imidazolyl | |
| E17 | $HO_2C$ | H | O | Leu | 3 | 1-imidazolyl | $3H_2O.HCl$ |
| E18 | H | H | $SO_2$ | Leu | 4 | $CO_2Me$ | $0.5H_2O$ |

TABLE 2 (Contd.)

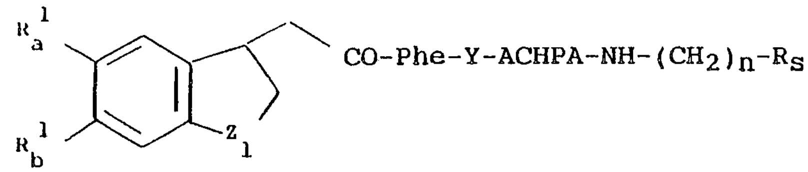

| Compound No. | $R_a^1$ | $R_b^1$ | $Z_1$ | Y | n | $R_S$ | salt/solvate |
|---|---|---|---|---|---|---|---|
| E19 | H | H | $SO_2$ | Leu | 4 | $CO_2H$ | |
| E20 | H | H | $SO_2$ | Leu | 4 | 4-methylpiperazin-1-yl | 2HCl |
| E21 | H | H | $SO_2$ | Leu | 3 | 4-methylpiperazin-1-yl | 2HCl |
| E22 | H | H | $SO_2$ | Leu | 2 | 4-methylpiperazin-1-yl | $2.5H_2O$ |
| E23 | H | H | $SO_2$ | Leu | 3 | $N(CH_3)_2$ | $1.5H_2O$ |
| E27 | H | H | $SO_2$ | Leu | 2 | $CO_2CH_2Ph$ | |
| E28 | H | H | $SO_2$ | Leu | 2 | $CO_2H$ | $1.5H_2O$ |
| E29 | H | H | $SO_2$ | Leu | 2 | (S)-$CH(NH_2)CO_2H$ | $1.5H_2O$.HCl |
| E30 | H | H | $SO_2$ | Leu | 2 | 2-pyridyl-N-oxide | $0.5H_2O.0.5CHCl_3$ |
| E31 | H | $O_2N$ | $SO_2$ | Leu | 3 | 1-imidazolyl | |
| E32(a) | H | $H_2N$ | $SO_2$ | Leu | 3 | 1-imidazolyl | |
| E32(b) | H | $H_2N$ | $SO_2$ | Leu | 3 | 1-imidazolyl | 2HCl |
| E35 | H | BOC-$NHCH_2$ | $SO_2$ | Leu | 3 | $CO_2CH_2Ph$ | |

**TABLE 2** (Contd.)

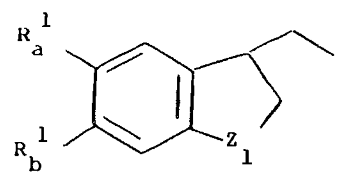 $CO-Phe-Y-ACHPA-NH-(CH_2)_n-R_S$

| Compound No. | $R_a^1$ | $R_b^1$ | $Z_1$ | Y | n | $R_S$ | salt/solvate |
|---|---|---|---|---|---|---|---|
| E36 | H | BOC-$NHCH_2$ | $SO_2$ | Leu | 3 | $CO_2H$ | |
| E37 | H | $H_2NCH_2$ | $SO_2$ | Leu | 3 | $CO_2H$ | TFA |
| E38 | BOC-$NHCH_2$ | H | O | Leu | 3 | $CO_2CH_2Ph$ | |
| E39 | BOC-$NHCH_2$ | H | O | Leu | 3 | $CO_2H$ | |
| E40 | $H_2NCH_2$ | H | O | Leu | 3 | $CO_2H$ | $0.5H_2O$.TFA |
| E42 | BOC-$NHCH_2$ | H | $SO_2$ | Leu | 3 | 1-imidazolyl | $2H_2O$ |
| E43 | $H_2NCH_2$ | H | $SO_2$ | Leu | 3 | 1-imidazolyl | $H_2O$.3TFA |
| E44 | $HO_2CCH_2NHCH_2$ | H | O | Leu | 3 | 1-imidazolyl | 3TFA |
| E45 | BOC-$NHCH_2$ | H | $SO_2$ | His | 3 | $CO_2CH_2Ph$ | |
| E46 | BOC-$NHCH_2$ | H | $SO_2$ | His | 3 | $CO_2H$ | |
| E47 | $H_2NCH_2$ | H | $SO_2$ | His | 3 | $CO_2H$ | $2H_2O$.3TFA |
| E48 | BOC-$NHCH_2$ | H | $SO_2$ | His | 3 | 1-imidazolyl | |
| E49 | $H_2NCH_2$ | H | $SO_2$ | His | 3 | 1-imidazolyl | $CH_2Cl_2$.3TFA |

TABLE 2 (Contd.)

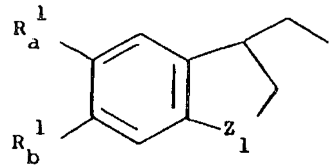

CO-Phe-Y-ACHPA-NH-$(CH_2)_n$-$R_s$

| Compound No. | $R_a^1$ | $R_b^1$ | $Z_1$ | Y | n | $R_s$ | salt/solvate |
|---|---|---|---|---|---|---|---|
| E51 | H | BOC-$NHCH_2$ | $SO_2$ | Leu | 3 | 1-imidazolyl | $2H_2O$ |
| E52 | H | $H_2NCH_2$ | $SO_2$ | Leu | 3 | 1-imidazolyl | 3TFA |
| E54 | H | H | $SO_2$ | Leu | 3 | Pro-OMe | $2H_2O.0.5CHCl_3$ .HCl |
| E55(a) | H | H | $SO_2$ | Leu | 2 | 3-pyridyl | |
| E55(b) | H | H | $SO_2$ | Leu | 2 | 3-pyridyl | HCl |
| E56 | H | H | $SO_2$ | Leu | 2 | 3-pyridyl-N-oxide | |
| E57 | H | H | $SO_2$ | Leu | 2 | 4-pyridyl | HCl |
| E58 | H | H | $SO_2$ | Leu | 2 | 4-pyridyl-N-oxide | |
| E59 | H | H | $SO_2$ | Leu | 3 | 2-imidazolyl | HCl |
| E60 | H | H | $SO_2$ | Leu | 3 | 2-pyridyl | HCl |
| E61 | H | H | $SO_2$ | Leu | 3 | 2-pyridyl-N-oxide | |
| E62 | H | H | $SO_2$ | Leu | 3 | Pro-OH | HCl |
| E63 | H | H | $SO_2$ | Leu | 4 | piperazin-1-yl | 2HCl |

TABLE 3

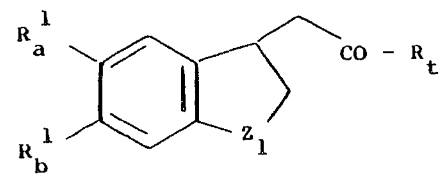

| Compound No. | $R_a^1$ | $R_b^1$ | $Z_1$ | $R_t$ | salt/solvate |
|---|---|---|---|---|---|
| D10(b) | H | H | $SO_2$ | OH | |
| D10(c) | H | $O_2N$ | $SO_2$ | OH | |
| D10(d) | H | $O_2N$ | $SO_2$ | OEt | |
| D10(e) | H | $H_2N$ | $SO_2$ | OEt | |
| D10(f) | H | Br | $SO_2$ | OEt | |
| D10(g) | H | NC | $SO_2$ | OEt | |
| D10(h) | H | BOC-$NHCH_2$ | $SO_2$ | OEt | |
| D10(i) | H | BOC-$NHCH_2$ | $SO_2$ | OH | |
| D11(a) | OHC | H | O | OEt | |
| D11(b) | NC | H | O | OEt | |
| D11(c) | BOC-$NHCH_2$ | H | O | OEt | |
| D11(d) | BOC-$NHCH_2$ | H | O | OH | |
| D16(a) | $HO_2C$ | H | O | OEt | |

TABLE 3 (Contd.)

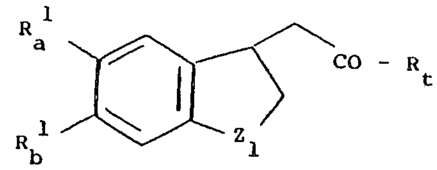

| Compound No. | $R_a^1$ | $R_b^1$ | $Z_1$ | $R_t$ | salt/solvate |
|---|---|---|---|---|---|
| D16(b) | $PhCH_2O_2C$ | H | O | OEt | |
| D16(c) | $PhCH_2O_2C$ | H | O | OH | |
| D27 | H | H | $SO_2$ | Phe-Leu-OH | |
| D30(e) | NC | H | $SO_2$ | OEt | |
| D30(f) | $BOC-NHCH_2$ | H | $SO_2$ | OEt | |
| D30(g) | $BOC-NHCH_2$ | H | $SO_2$ | OH | |
| D31(a) | OHC | H | O | OH | |
| D31(b) | OHC | H | O | OtBu | |
| D31(c) | $MeO_2CCH_2NHCH_2$ | H | O | OtBu | |
| D31(d) | $MeO_2CCH_2NHCH_2$ | H | O | OH | TFA |
| D31(e) | $MeO_2CCH_2N(BOC)CH_2$ | H | O | OH | |
| D32(a) | $BOC-NHCH_2$ | H | $SO_2$ | Phe-His-OMe | |
| D32(b) | $BOC-NHCH_2$ | H | $SO_2$ | Phe-His-OH | (HCl) |

## Miscellaneous Structures (descriptions)

D10(a)

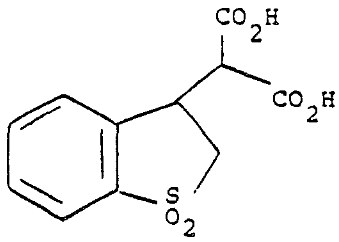

D22(a)

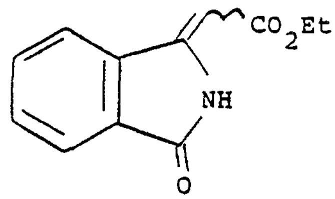

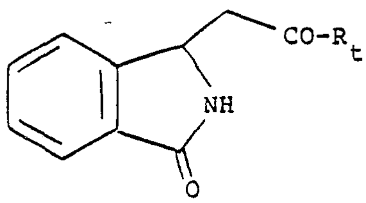

$R_t$ = OEt D22(b)

$R_t$ = OH D22(c)

$R_t$ = PheLeuOCH$_2$Ph D22(d)

$R_t$ = PheLeuOH D22(e)

D28(a)

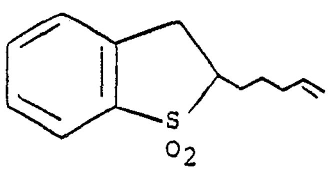

D28(b)

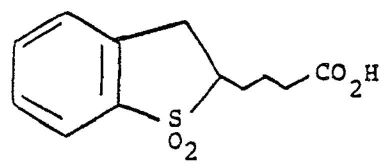

D28(c)

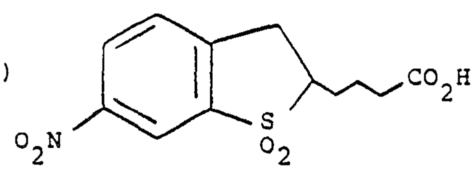

D29(a)

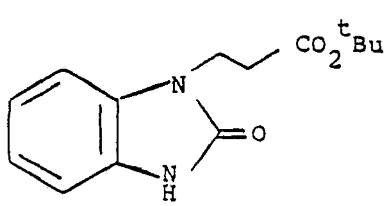

D29(b)

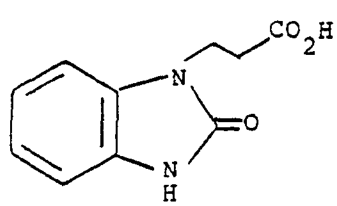

Miscellaneous Structures (descriptions) continued

D30(a)

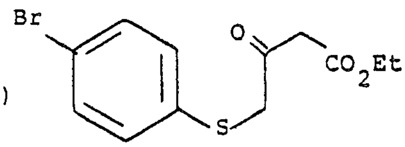

D30(b)

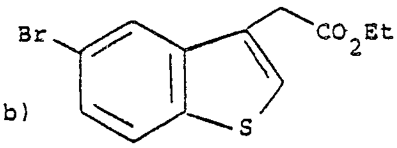

D30(c)

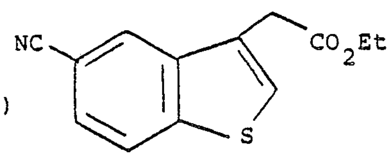

D30(d)

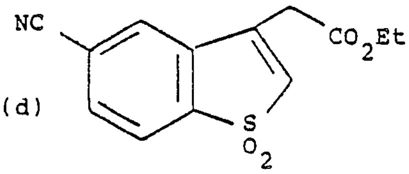

D33(b)

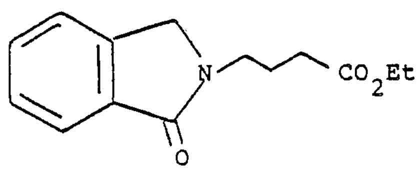

D33(c)

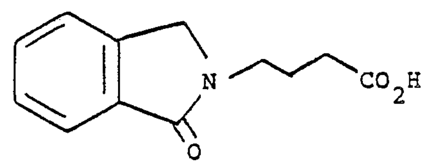

## Miscellaneous Structures (examples)

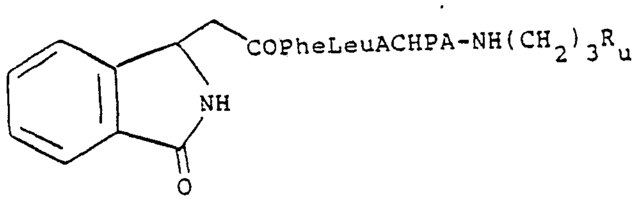

$R_u$ = $CO_2CH_2Ph$ E24

$R_u$ = $CO_2H$ E25(a)

$R_u$ = $CO_2Na$ E25(b)

$R_u$ = 1-imidazolyl E26(a)

$R_u$ = 1-imidazolyl .$HCl.2H_2O$ E26(b)

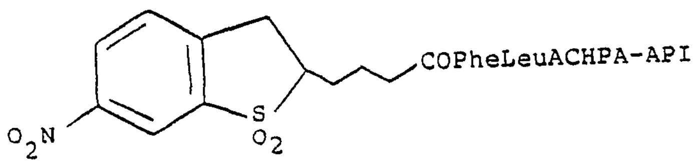

E33

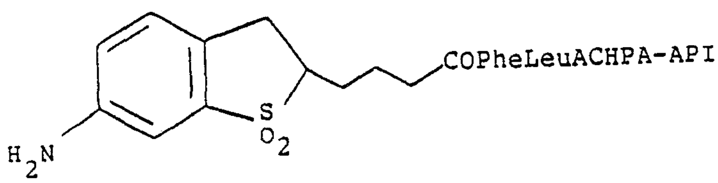

E34(a)

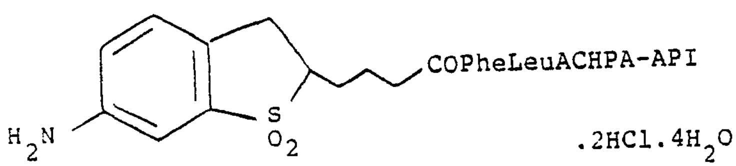

.$2HCl.4H_2O$

E34(b)

## Miscellaneous Structures (examples) continued

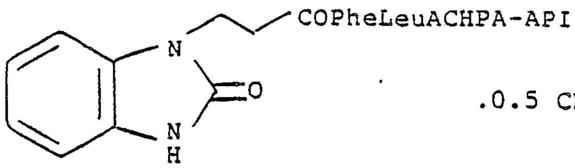

.0.5 $CHCl_3$

E41

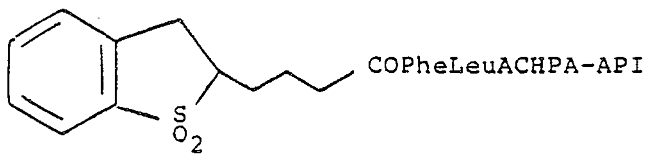

E50(a)

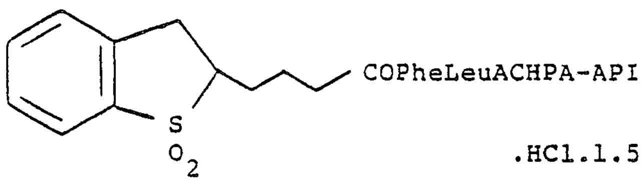

.HCl.1.5$H_2O$

E50(b)

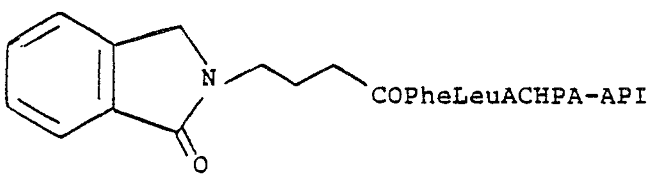

E53

API = 3-(1-imidazolyl)propylamide

Description 1

a) BOC-ACHPA 3-(1-imidazolyl)propylamide

BOC-ACHPA-OH (J. Med. Chem. 1985, 28, 1779-1790.) (0.207g), 1-hydroxybenzotriazole (HOBT) (0.098g) and 1-(3-aminopropyl)imidazole (0.086 ml) were stirred at 0°C in dry dimethylformamide (DMF) (5 ml). 1-(3-dimethylaminopropyl)-3-ethylcarbodiimide hydrochloride (DEC) (0.139g) was added, and the mixture was stirred for 16h, warmed to ambient temperature, diluted with ethyl acetate (50 ml), washed with 5% sodium hydrogen carbonate solution, water, and brine; dried ($Na_2SO_4$) and evaporated. This gave the title compound (0.177g) as an oil.

b) CBZ-Phe-Leu-ACHPA 3-(1-imidazolyl)propylamide .$0.5H_2O$

BOC-ACHPA 3-(1-imidazolyl)propylamide (0.177g) was dissolved in dichloromethane (4 ml), and cooled in ice. Trifluoroacetic acid (4 ml) was added. The mixture was swirled and left to stand at ambient temperature for 0.5h, and then evaporated, dissolved in dry dimethylformamide (3 ml) and cooled in ice. CBZ-Phe-Leu-OH (0.19g) and HOBT (0.062g) were stirred at 0°C in dry DMF (3 ml), and DEC (0.088g) was added. This mixture was stirred at 0°C for 15 min, and then diisopropylethylamine (DIPEA) (0.22 ml) and the amine trifluoroacetate solution, prepared above, were added. After stirring for 16h, with warming to ambient temperature, the work up procedure of Description 1(a) was followed. The crude product was chromatographed on silica gel using methanol/chloroform (0-10% methanol, gradient). This gave the title compound (0.195g) as a white solid.

NMR (δ) ($CDCl_3$): 0.75-0.1 (7H, m), 1.05-2.5 (20H, m), 2.9-3.4 (4H, m), 3.9-4.1 (4H, m), 4.15-4.5 (2H, m), 5.0-5.15 (2H, m), 5.45 (b), 6.35-6.65 (2H, m), 6.9-7.1 (3H, m), 7.1-7.45 (10H, m), 7.5-7.8 (1H, m).
Analysis: $C_{40}H_{56}N_6O_6.0.5H_2O$ requires C,66.2; H,7.9; N,11.6%. Found: C,65.9; H,7.7; N,11.3%.
M.S. (m/z) (FAB) (M+1)=717 (consistent with m.w.=716).

Description 2

a) BOC-ACHPA 2-(4-morpholinyl)ethylamide

This material was formed from BOC-ACHPA-OH (0.23g) and 2-(aminoethyl)morpholine (0.11 ml), following the procedure of Description 1(a). This gave the title compound (0.195g) as an oil.

b) CBZ-Phe-Leu-ACHPA 2-(4-morpholinyl)ethylamide .$0.5H_2O$

This material was formed from BOC-ACHPA 2-(4-morpholinyl)ethylamide (0.195g) following the procedure of Description 1(b). The crude product was chromatographed on silica gel using methanol/chloroform (0-10% methanol, gradient). This gave the title compound (0.163g) as a white solid.

NMR (δ) ($CDCl_3$): 0.75-1.0 (7H, m), 1.05-2.2 (16H, m), 2.2-2.35 (2H, m), 2.35-2.6 (6H, m), 3.0-3.15 (2H, m), 3.15-3.55 (2H, m), 3.7 (4H, m), 3.85-4.05 (2H, m), 4.2-4.45 (2H, m), 4.95-5.15 (2H, m), 5.3 (d) and 5.65 (d) (1H), 6.15-6.6 (3H, m), 7.1-7.4 (10H, m).
Analysis: $C_{40}H_{59}N_5O_7.0.5H_2O$ requires C,65.7; H,8.3; N,9.6%. Found: C,65.7; H,8.1; N,9.3%.
M.S. (m/z) (FAB) (M+1)=722 (consistent with m.w.=721).

Description 3

a) BOC-ACHPA 3-(2-oxopyrrolidin-1-yl)propylamide

This material was formed from BOC-ACHPA-OH (0.22 g) and 1-(3-aminopropyl)-2-pyrrolidinone (0.11 ml), following the procedure of Description 1(a). This gave the title compound (0.227 g) as an oil.

b) CBZ-Phe-Leu-ACHPA 3-(2-oxopyrrolidin-1-yl)propylamide .$0.5H_2O$

This material was formed from BOC-ACHPA 3-(2-oxopyrrolidin-1-yl)propylamide (0.227 g), following the procedure of Description 1(b). The crude product was chromatographed on silica gel using methanol/chloroform (0-10% methanol, gradient). This gave the title compound (0.273 g) as a yellow solid.

NMR (δ) ($DMSOd_6$): 0.7-1.1 (10H, m), 1.1-1.95 (m), 1.95-2.1 (2H, m), 2.1-2.4 (5H, m), 2.8-2.95 (1H, m), 3.0-3.2 (3H, m), 3.2-3.35 (2H, m), 3.35-3.6 (m), 3.95-4.05 (2H, m), 4.3-4.65 (2H, m), 4.95-5.15 (3H, m), 7.2-7.9 (13H, m), 8.2-8.4 (1H, m).
Analysis: $C_{41}H_{59}N_5O_7.0.5H_2O$ requires C, 66.3; H, 8.1; N, 9.4%. Found: C, 66.5; H, 8.1; N, 9.0%.
M.S. (m/z) (FAB) (M+1) = 734 (consistent with m.w. = 733).

Description 4

a) BOC-ACHPA 2-(2-pyridyl)ethylamide

This material was formed from BOC-ACHPA-OH (0.20 g) and 2-(2-aminoethyl)pyridine (0.08 ml), following the procedure of Description 1(a). This gave the title compound (0.183 g).

b) CBZ-Phe-Leu-ACHPA 2-(2-pyridyl)ethylamide .$0.5H_2O$

This material was formed from BOC-ACHPA 2-(2-pyridyl)ethylamide (0.183 g) following the procedure of Description 1(b). The crude product was chromatographed on silica gel using methanol/chloroform (0-5% methanol, gradient). This gave the title compound (0.205 g).

NMR (δ) ($CDCl_3$): 0.75-1.05 (7H, m), 1.1-2.1 (15H, m), 2.15-2.4 (2H, m), 2.95-3.15 (4H, m), 3.35-3.8 (2H, m), 3.8-4.05 (2H, m), 4.2-4.45 (2H, m), 4.95-5.15 (2H, m), 5.2-5.5 (1H, m), 6.15-6.3 (1H, m), 6.4-6.75 (1H, m), 6.8-7.05 (1H, m), 7.1-7.4 (13H, m), 7.55-7.6 (1H, m), 8.45-8.55 (1H, m).

Analysis: $C_{41}H_{55}N_5O_6.0.5H_2O$ requires C,.68.1; H, 7.8; N, 9.7%. Found: C, 68.3; H, 7.6; N, 9.7%.

M.S. (m/z) (FAB) (M+1) = 714 (consistent with m.w = 713).

Description 5

a) BOC-ACHPA 2-hydroxyethylamide

This material was formed from BOC-ACHPA-OH (0.20g) and ethanolamine (0.04 ml), following the procedure of Description 1(a). This gave the title compound (0.134 g).

b) CBZ-Phe-Leu-ACHPA 2-hydroxyethylamide.

This material was formed from BOC-ACHPA 2-hydroxyethylamide (0.134 g) following the procedure of Description 1(b). The crude product was chromatographed on silica gel using methanol/chloroform (0-10% methanol, gradient). This gave the title compound (0.134 g).

NMR (δ) ($CDCl_3$): 0.65-1.05 (8H, m), 1.05-1.3 (4H, m), 1.3-1.95 (10H, m), 2.15-2.3 (1H, m), 2.3-2.5 (1H, m), 2.8-3.05 (1H, m), 3.05-3.8 (8H, m), 3.85-4.1 (2H, m), 4.15-4.3 (1H, m), 4.35-4.55 (1H, m), 4.85-5.15 (2H, m), 6.5-6.7 (1H, m), 6.75-6.9 (1H, d), 6.95-7.45 (10H, m), 7.45-7.8 (1H, m).

Analysis: $C_{36}H_{52}N_4O_7$ requires C, 66.2; H, 8.0; N, 8.6%. Found: C, 66.0; H, 7.6; N, 8.5%.

M.S. (m/z) (FAB) (M+1) = 653 (consistent with m.w = 652).

Description 6

a) BOC-ACHPA 2-(acetylamino)ethylamide.

This material was formed from BOC-ACHPA-OH (0.201g) and N-acetylethylenediamine (0.145 g), following the procedure of Description 1(a). This gave the title compound (0.182 g) as an orange oil.

Description 7

BOC-ACHPA-NH$(CH_2)_3CO_2Et$

This material was formed from BOC-ACHPA-OH (0.255 g) and ethyl 4-aminobutanoate hydrochloride (0.149 g), following the procedure of Description 1(a). This gave the title compound (0.253 g) as an yellow

solid.

Description 8

a) Nα-CBZ-Nδ-BOC-Orn benzyl ester

Nα-CBZ-Nδ-BOC-Orn-OH (0.5 g) and anhydrous potassium carbonate (0.21 g) were stirred in dry DMF (8 ml). Benzyl bromide (0.18 ml) was added, and the mixture was stirred for 16h, diluted with ethyl acetate (100 ml), washed with water and brine, dried ($MgSO_4$) and evaporated. The crude product was chromatographed on silica gel using chloroform. This gave the title compound (0.612 g) as a pale brown oil.

NMR (δ) ($CDCl_3$): 1.35-1.5 (11H, m), 1.6-1.75 (1H, m), 1.8-1.95 (1H, m), 3.05-3.15 (2H, q), 4.35-4.5(2H, m), 5.05-5.1 (2H, s), 5.15-5.2 (2H, s), 5.3-5.4 (1H, m), 7.25-7.4 (10H, s).

b) (S)-BOC-ACHPA-NH$(CH_2)_3$CH(NH-CBZ)$CO_2CH_2$Ph

This material was formed from BOC-ACHPA-OH (0.20g) and Nα-CBZ-Nδ-BOC-Orn benzyl ester (0.32 g), following the procedure of Description 1(b). This gave the title compound (0.432 g) as a yellow oil.

NMR(δ)($CDCl_3$): 0.75-1.05 (1H, m), 1.1-1.4 (4H, m), 1.4-1.95 (21H, m), 2.2-2.5 (2H, m), 3.15-3.3 (2H, m), 3.5-3.65 (1H, m), 3.85-3.95 (1H, m), 4.35-4.45 (1H, m), 4.7-4.85 (1H, m), 5.05-5.1 (2H, s), 5.15-5.2 (2H, s), 5.45-5.6 (1H, m), 6.15-6.3 (1H, bs), 7.25-7.45 (11H, m).

c) (2,3-Dihydro-1,1-dioxobenzothiophene-3-yl)acetyl-Phe-Leu-ACHPA-NH$(CH_2)_3$-(S)-CH(NH-CBZ)$CO_2CH_2$Ph .0.5$H_2O$

This material was formed from (S)-BOC-ACHPA-NH$(CH_2)_3$CH(NH-CBZ)$CO_2CH_2$Ph (0.43 g) and (2,3-dihydro-1,1-dioxobenzothiophene-3-yl)acetyl-Phe-Leu-OH (0.352 g) (Description 27) following the method of Description 1(b). The crude product was chromatographed on silica gel using methanol/chloroform (0-5% methanol, gradient). This gave the title compound (0.546 g) as a white solid.

NMR(δ)($CD_3OD$): 0.7-0.85 (4H, m), 0.85-1.05 (4H, m), 1.1-1.45 (6H, m), 1.45-1.9 (12H, m), 2.2-2.35 (2H, m), 2.4-2.65 (1H, m), 2.7-2.85 (2H, m), 2.85-3.15 (3H, m), 3.4-3.65 (1H, m), 3.8-3.95 (0.5H, m), 3.95-4.05 (2.5H, m), 4.1-4.3 (2H, m), 4.35-4.45 (0.5H, m), 4.45-4.6 (2H, m), 5.0-5.1 (2H, m), 5.1-5.2 (2H, m), 7.2-7.4 (15H, m), 7.4-7.55 (2H, m), 7.55-7.7 (2H, m).

Analysis: $C_{56}H_{71}N_5O_{11}S.0.5H_2O$ requires C, 65.2; H,7.0; N, 6.8%. Found: C, 65.2; H, 7.0; N, 6.8%.

M.S. (m/z) (FAB) (M+1) = 1022 (consistent with m.w. = 1021).

Description 9

a) Nα-CBZ-Nε-BOC-Lys benzyl ester.

This material was formed from Nα-CBZ-Nε-BOC-Lys-OH (0.51 g) following the procedure of Description 8(a). The crude product was chromatographed on silica gel using chloroform/petroleum ether (b.p. 60-80°C) (50-100% chloroform, gradient). This gave the title compound (0.603 g) as a yellow oil.

NMR (δ) ($CDCl_3$): 1.2-1.5 (14H, m), 1.6-1.9 (1H, m), 2.95-3.15 (2H, m), 4.3-4.6 (2H, m), 5.0-5.1 (2H, s), 5.1-5.2 (2H, d), 5.3-5.45 (1H, m), 7.2-7.5 (10H, s).

b) (S)-BOC-ACHPA-NH$(CH_2)_4$CH(NH-CBZ)$CO_2CH_2$Ph

This material was formed from BOC-ACHPA-OH (0.20 g) and Nα-CBZ-Nε-BOC-Lys benzyl ester (0.33 g), following the procedure of Description 1(b). This gave the title compound (0.421 g) as a yellow oil.

NMR (δ) ($CDCl_3$): 0.6-1.0 (3H, m), 1.05-1.5 (19H, m), 1.55-1.95 (6H, m), 2.1-2.45 (2H, m), 3.1-3.25 (2H, m), 3.5-3.65 (0.5H, m), 3.85-4.0 (0.5H, m), 4.3-4.45 (1H, m), 4.7-4.8 (0.5H, m), 5.0-5.2 (5H, m), 5.4-5.6 (1H,

m), 6.05-6.2 (0.5H, m), 7.25-7.4 (12H, m).

c) (2,3-Dihydro-1,1-dioxobenzothiophene-3-yl)acetyl-Phe-Leu-ACHPA-NH($CH_2$)4-(S)-CH(NH-CBZ)-$CO_2CH_2Ph.H_2O$

This material was formed from (S)-BOC-ACHPA-NH($CH_2$)$_4$CH(NH-CBZ)$CO_2CH_2$Ph (0.42g),following the method of Description 8(c). The crude product was chromatographed on silica gel using methanol/chloroform (0-5% methanol, gradient). This gave the title compound (0.50 g) as a yellow foam.

NMR (δ) ($CD_3OD$): 0.65-0.85 (3H, m), 0.85-1.05 (4H, m), 1.1-1.9 (21H, m), 2.2-2.65 (2.5H, m), 2.7-2.9 (1.5H, m), 2.9-3.25 (4H, m), 3.4-3.65 (0.5H, m), 3.8-3.9 (0.5H, m), 3.9-4.05 (2H, m), 4.1-4.3 (1.5H, m), 4.3-4.4 (0.5H, m), 4.45-4.65 (2H, m), 5.0-5.25 (5H, m), 7.1-7.4 (15H, m), 7.4-7.55 (2H, m), 7.55-7.7 (2H, m).

Analysis: $C_{57}H_{73}N_5O_{11}S.H_2O$ requires C, 64.9; H, 7.2; N, 6.6%. Found: C, 65.0; H, 7.0; N, 6.6%.

M.S. (m/z) (FAB) (M+1) = 1036 (consistent with m.w. = 1035)

Description 10

a) 2-(2,3-Dihydro-1,1-dioxobenzothiophene-3-yl)malonic acid

To a solution of diethyl 2-(2,3-dihydro-1,1-dioxobenzothiophene-3-yl)malonate (11.6g) (J. Am. CHem. Soc. 1950, 72, 1985.) in ethanol (150 ml) was added sodium hydroxide (4g) in water (30 ml). The mixture was stirred at reflux for 4h, cooled and the ethanol was removed under reduced pressure. The residue was diluted with water (20 ml) and was extracted with ether (3x100 ml) and dichloromethane. The aqueous layer was acidified to pH 1 with 5M hydrochloric acid and was extracted with ether (3x80 ml) and dichloromethane (3x80 ml). The combined extracts (3x ether + 3x dichloromethane) were dried over sodium sulphate and the solvent was removed under reduced pressure to give the title compound (6.2g). NMR (δ) ($DMSOd_6$): 3.6-3.9 (2H, m), 4.1-4.3 (2H, m), 7.5-7.9 (4H, m), 13.2 (2H, b).

b) (2,3-Dihydro-1.1-dioxobenzothiophene-3-yl)acetic acid

2-(2,3-Dihydro-1,1-dioxobenzothiophene-3-yl)malonic acid (1.9g) was heated in xylene at reflux for 3h. The mixture was cooled and extracted with saturated sodium hydrogen carbonate (2x50 ml). The combined aqueous extracts were washed with ether (50 ml) and acidified to pH 2 with 5M hydrochloric acid. The mixture was extracted with ether (2x80 ml) and dichloromethane (2x80 ml). The combined extracts were dried ($Na_2SO_4$) and the solvent was removed under reduced pressure to give the title compound (1.4g).

NMR (δ) ($DMSOd_6$): 2.6-2.8 (1H, m), 2.9-3.1 (1H, m), 3.4-3.6 (1H, m), 3.8-4.0 (2H, m), 7.5-7.9 (4H, m), 12.6 (1H, b).

c) (6-Nitro-2,3-dihydro-1,1-dioxobenzothiophene-3-yl)acetic acid

(2,3-Dihydro-1,1-dioxobenzothiophene-3-yl)acetic acid (2.00g) was added portionwise to fuming nitric acid (9 ml), cooled in a water bath; stirring was continued for 4.5h. The solution was poured into water (75 ml) and the precipitated solid filtered off. The filtrate was cooled to 0°C and the filtration repeated. The combined solids were air-dried to give the title compound (1.96g). NMR (δ) ($DMSOd_6$): 2.8-2.9 (1H, m), 3.0-3.1 (1H, dd), 3.6-3.7 (1H, m), 3.9-4.1 (2H, m), 7.9 (1H, m), 8.5 (2H, m), 12.7 (1H, b).

d) Ethyl (6-nitro-2.3-dihydro-1,1-dioxobenzothiophene-3-yl acetate

(6-Nitro-2,3-dihydro-1,1-dioxobenzothiophene-3-yl)acetic acid (26.57g) was taken up in anhydrous ethanol (200 ml) and concentrated sulphuric acid (15 ml) and heated at reflux for 1.5h. After concentration of the solution to half volume, the mixture was poured into water (100 ml) and the precipitated solid filtered off. This was air-dried to give the title compound (27.59g).

NMR (δ) ($CDCl_3$): 1.2-1.3 (3H, t), 2.8-3.0 (2H, m), 3.4-3.5 (1H, dd), 3.8-3.9 (1H, dd), 4.1-4.3 (3H, m), 7.6

(1H, d), 8.5 (1H, m), 8.6 (1H, d).

e) Ethyl (6-amino-2,3-dihydro-1,1-dioxobenzothiophene-3-yl acetate

A solution of ethyl (6-nitro-2,3-dihydro-1,1-dioxobenzothiophene-3-yl)acetate (1.47g) in ethanol (100 ml) was hydrogenated at room temperature and pressure for 4h in the presence of 10% palladium on carbon. The catalyst was removed by filtration through Kieselguhr and the filtrate evaporated to give the title compound (1.2g).

NMR (δ) ($CDCl_3$): 1.2-1.3 (3H, t), 2.6-2.9 (2H, m), 3.2-3.4 (1H, dd), 3.6-3.7 (1H, m), 3.8-3.9 (1H, m), 4.1-4.2 (2H, q), 6.8-7.0 (2H, m), 7.1-7.2 (1H, m).

f) Ethyl (6-bromo-2,3-dihydro-1,1-dioxobenzothiophene-3-yl)acetate

Ethyl (6-amino-2,3-dihydro-1,1-dioxobenzothiophene-3-yl)acetate (10.81g) was taken up in aqueous hydrobromic acid with heating to aid solution. The solution was then cooled to 0°C. A solution of sodium nitrite (2.80g) in water (4.5 ml) was added dropwise (evolution of brown fumes). Once evolution had ended the mixture was allowed to warm to room temperature. Copper bronze (0.5g) was added, leading to immediate effervescence. After heating gently for 30 min the purple solution was poured into ice. Residual copper bronze was filtered off through Kieselguhr and the filtrate extracted with chloroform (3x50 ml). The combined organics were dried ($Na_2SO_4$) and the solvents removed under reduced pressure to give (6-bromo-2,3-dihydro-1,1-dioxobenzothiophene-3-yl)acetic acid (8g). This was heated to reflux in anhydrous ethanol (150 ml) and concentrated sulphuric acid (6 ml) for 1.5h. After cooling, the solution was concentrated to half volume, poured into water and extracted with chloroform (4x50 ml). The combined organic layers were washed with sodium hydrogen carbonate (2x30 ml), dried ($Na_2SO_4$) and the solvents removed to give the title compound (8.13g).

NMR (δ) ($CDCl_3$): 1.2-1.3 (3H, t), 2.7-2.9 (2H, m), 3.3-3.4 (1H, dd), 3.7-3.8 (1H, m), 3.9-4.0 (1H, m), 4.1-4.3 (2H, q), 7.2-7.3 (1H, m), 7.7 (1H, dd), 7.9 (1H, d).

g) Ethyl (6-cyano-2,3-dihydro-1,1-dioxobenzothiophene-3-yl)acetate

Ethyl (6-bromo-2,3-dihydro-1,1-dioxobenzothiophene-3-yl)acetate (13.11g) was taken up in DMF (120 ml) and stirred at room temperature with copper (I) cyanide (6.9g). The resulting solution was refluxed overnight under nitrogen. After cooling, the copper complex was broken down with aqueous 1,2-diaminoethane (500 ml). The resulting blue solution was extracted with ether (5x100 ml). The combined organic extracts were washed with brine (100 ml), and the solvents removed in vacuo to give a beige solid (5.88g). This was purified by chromatography on silica gel using ethyl acetate/petroleum ether (b.p. 60-80°C) (0-30% ethyl acetate, gradient) to afford the title compound (4.98g).

NMR (δ) ($CDCl_3$): 1.2-1.3 (3H, t), 2.8-3.0 (2H, m), 3.3-3.5 (1H, m), 3.7-3.8 (1H, m), 4.0-4.3 (3H, m), 7.6 (1H, d), 7.9 (1H, dd), 8.1 (d).

h) Ethyl (6-(BOC-aminomethyl)-2,3-dihydro-1,1-dioxobenzothiophene-3-yl)acetate

Ethyl (6-cyano-2,3-dihydro-1,1-dioxobenzothiophene-3-yl)acetate (1.63g) and cobalt (II) chloride hexahydrate (2.78g) were stirred in methanol (30ml). Sodium borohydride (0.67g) was added portionwise, and the black suspension so formed was stirred for 1 h, diluted with water (200ml), acidified (5M hydrochloric acid), and then basified with excess aqueous ammonia. This solution was extracted with chloroform, and the extract was dried ($Na_2SO_4$) and evaporated to a brown oil (1.78g). This was dissolved in dichloromethane (30 ml), and di-tert-butyl dicarbonate (1.27g) and triethylamine (0.81 ml) were added. The solution was stirred for 16 h, diluted with chloroform (50 ml), washed with 0.5M hydrochloric acid, dried ($Na_2SO_4$) and evaporated. This crude product was purified by chromatography on silica gel using chloroform. This gave the title compound (1.42g) as a colourless gum.

NMR (δ)d ($CDCl_3$): 1.3 (3H, t), 1.45 (9H, s), 2.75 (1H) and 2.95 (1H) (ABX), 3.35 (1H) and 3.75 (1H) (ABX), 4.0 (1H, m), 4.2 (2H, q), 4.4 (2H, d), 5.0 (1H, b), 7.4 (1H, d), 7.55 (1H, d), 7.65 (1H, s).

i) (6-(BOC-aminomethyl)-2,3-dihydro-1,1-dioxobenzothiophene-3-yl)acetic acid

Ethyl (6-(BOC-aminomethyl)-2,3-dihydro-1,1-dioxobenzothiophene-3-yl)acetate (1.37g) was dissolved in ethanol (20 ml), and sodium hydroxide (10% aqueous solution, 2.0 ml) was added. The mixture was swirled, and left to stand for 2 h, diluted with water (200 ml), acidified (5M hydrochloric acid), and extracted with chloroform. The extract was dried ($Na_2SO_4$) and evaporated to give the title compound (1.25g) as a white foam.

NMR (δ) ($CDCl_3$): 1.5 (9H, s), 2.8-3.05 (2H, ABX), 3.4 (1H, dd), 3.75 (1H, dd), 4.0 (1H, b), 4.4 (2H, bd), 5.0 (b), 7.4 (1H, d), 7.55 (1H, d), 7.65 (1H, s).

Description 11

a) Ethyl (5-formyl-2,3-dihydrobenzofuran-3-yl)acetate

To ethyl (2,3-dihydrobenzofuran-3-yl)acetate (10.55g) in dry DMF (10.5 ml) was added cautiously, with stirring, phosphoryl chloride (55 ml). This mixture was stirred at reflux for 5 h, cooled, and poured cautiously into water (750 ml) with stirring, maintaining temperature below 80°C. This solution was cooled and extracted with ether (3x150 ml), and the extract was washed with 10% sodium carbonate solution (100 ml), dried ($Na_2SO_4$) and evaporated under reduced pressure to give a brown oil (7.94g). This was chromatographed on silica gel using ether/petroleum ether (b.p. 60-80°C) (10-50% ether, gradient), giving recovered ethyl (2,3-dihydrobenzofuran-3-yl)acetate (2.59g), and the title compound (5.15g), contaminated with ca.25% of an unidentified, dihydrobenzofuran-derived, impurity.

NMR (δ) ($CDCl_3$): 1.3 (3H,t), 2.5-2.9 (2H, ABX), 3.9 (1H,m), 4.2 (2H,q), 4.4 (1H,dd), 4.9 (1H, t), 6.9 (1H,d), 7.7 (2H,m), 9.85 (1H,s).

b) Ethyl (5-cyano-2,3-dihydrobenzofuran-3-yl)acetate

Ethyl (5-formyl-2,3-dihydrobenzofuran-3-yl)acetate (0.92g) and hydroxylamine hydrochloride (0.27g) were stirred in ethanol (20 ml) for 5 h. The solution was concentrated and poured into water. The crude product was extracted into chloroform, dried ($Na_2SO_4$) and evaporated. Elution through silica gel using chloroform gave a waxy solid (0.80g). This was stirred in dry dioxan (6 ml) with triethylamine (1.8 ml), and cooled in ice. Trifluoroacetic anhydride (0.50 ml) was added over 6 min, and the mixture was stirred for 6 h, warming to ambient temperature. 2M hydrochloric acid (100 ml) was added, and the product was extracted into chloroform. The extract was washed with 5% sodium hydrogen carbonate solution, dried ($Na_2SO_4$) and evaporated. Residual aldehyde was removed by treatment with sodium borohydride (0.05g) in methanol (10 ml). The crude product was chromatographed on silica gel using ether/petroleum ether (b.p. 60-80°C) (10-50% ether, gradient). This gave the title compound as a white solid (0.52g), still contaminated with ca.30% of the unidentified impurity of Description 11(a).

NMR (δ) ($CDCl_3$): 1.3 (3H, t), 2.55-2.8 (2H, ABX), 3.9 (1H, m), 4.2 (2H, q), 4.35 (1H, dd), 4.85 (1H, t), 6.85 (1H, d), 7.4-7.5 (2H, m).

c) Ethyl (5-(BOC-aminomethyl)-2,3-dihydrobenzofuran-3-yl)acetate

This material was formed from ethyl (5-cyano-2,3-dihydrobenzofuran-3-yl)acetate (2.92g), following the procedure of Description 10(h). The intermediate amine was purified by extraction from ether into 0.5 M hydrochloric acid, followed by basification and extraction into chloroform, prior to conversion to the carbamate. This gave, without the need for chromatographic purification, the title compound (1.79g) as an amber oil.

NMR (δ) ($CDCl_3$): 1.3 (3H, t), 1.45 (9H, s), 2.55 (1H) and 2.8 (1H)(ABX), 3.85 (1H, m), 4.15-4.3 (5H, m), 4.75 (1H, t), 6.75 (1H, d), 7.0-7.1 (2H, m).

d) (5-(BOC-aminomethyl)-2,3-dihydrobenzofuran-3-yl)acetic acid

This material was formed from ethyl (5-(BOC-amino methyl)-2,3-dihydrobenzofuran-3-yl)acetate (1.69g), following the procedure of Description 10(i). This gave the title compound (1.34g) as a white foam.

NMR (δ) ($CDCl_3$): 1.45 (9H, s), 2.65 (1H) and 2.85 (1H) (ABX), 3.85 (1H, m), 4.2 (2H, b), 4.3 (1H, dd), 4.75 (1H, t), 6.75 (1H, d), 7.0-7.15 (2H, m).

Description 12

a) CBZ-Phe-Leu-ACHPA-$NH(CH_2)_3CO_2Et$

This material was formed from BOC-ACHPA-$NH(CH_2)_3CO_2Et$ (0.80g) (Description 7) following the procedure of Description 1(b). The crude product was chromatographed on silica gel using methanol/chloroform (0-5% methanol, gradient), and then crystallised from ether. This gave the title compound (0.99g) as a white powder.

NMR (δ) ($CDCl_3$): 0.75-1.05 (8H, m), 1.05-1.9 (19H, m), 2.2-2.4 (4H, m), 3.1 (2H, m), 3.15-3.3 (2H, m), 4.0 (2H, m), 4.1 (2H, q), 4.2-4.45 (2H, m), 5.05 (2H, m), 5.3 (1H, d), 5.7 (d), 6.2 (d), 6.4 (d), 6.55-6.7 (1H, m), 7.1-7.4 (m).

b) Phe-Leu-ACHPA-$NH(CH_2)_3CO_2Et$ .HCl

CBZ-Phe-Leu-ACHPA-$NH(CH_2)_3CO_2Et$ (0.99g) was hydrogenated in ethanol (25 ml) over 10% palladium on charcoal (10% aqueous paste, 1.6g, added in 3 portions) for 15 h. Catalyst was filtered off, and the filtrate was evaporated, dissolved in ethanol (3 ml), acidified with a slight excess of aqueous hydrochloric acid, diluted with water (50 ml), and freeze-dried. This gave the title compound (0.83g) as a grey solid.

NMR (δ) ($CDCl_3$): 0.7-1.0 (7H, m), 1.0-1.35 (9H, m), 1.35-2.1 (11H, m), 2.2-2.4 (4H, m), 2.9-3.15 (2H, m), 3.2-3.35 (2H, m), 3.8-4.05 (3H, m), 4.1 (2H, q), 4.45 (1H, m), 6.4-6.6 (1H, m), 6.75 (d), 6.85 (d), 7.15-7.4 (7H, m).

Description 13

a) BOC-Phe-Leu-ACHPA 3-(1-imidazolyl)propylamide

This material was formed from BOC-ACHPA 3-(1-imidazolyl)propylamide (1.53g) (Description 1(a)) and BOC-Phe-Leu-OH (1.60g),following the procedure of Description 1(b). The crude product was chromatographed on silica gel using methanol/chloroform (0-10% methanol, gradient). This gave the title compound (1.95g).

NMR (δ) ($CDCl_3$): 0.7-1.05 (8H, m), 1.05-1.9 (2H, m), 2.0 (2H, m), 2.2-2.5 (2H, m), 2.9-3.6 (5H, m), 3.9-4.5 (6H, m), 5.1 (b), 6.4 (b), 6.6 (bd), 7.0 (1H, s), 7.05 (1H, s), 7.15-7.4 (m), 7.65 (1H, s).

b) Phe-Leu-ACHPA 3-(1-imidazolyl)propylamide .2($CF_3CO_2H$)

To a solution of BOC-Phe-Leu-ACHPA 3-(1-imidazolyl)propylamide (0.60g) in dichloromethane (6 ml) was addd trifluoroacetic acid (6 ml). The mixture was stirred for 1 h, and evaporated. Trituration with ether/pentane and filtration then gave the title compound (0.62g) as a white solid.

Description 14

a) N-(2-(1-Imidazolyl)ethyl)phthalimide

N-(2-Bromoethyl)phthalimide (3.14g) and imidazole (0.68g) were stirred at 60°C in dry DMF (10 ml) for

64 h. The mixture was diluted with ethyl acetate (100 ml) and water (100 ml). The layers were separated, the aqueous layer extracted with chloroform and the combined organics dried ($MgSO_4$) and evaporated. The crude product was chromatographed on silica gel using methanol/chloroform (0-2% methanol, gradient). This gave the title compound (1.97g) as a white solid.

NMR (δ) ($CDCl_3$): 4.0-4.1 (2H, t), 4.25-4.35 (2H, t), 6.9-7.0 (1H, s), 7.0-7.1 (1H, s), 7.35-7.45 (1H, s), 7.65-7.8 (2H, m), 7.8-7.9 (2H, m).

b) 1-(2-Aminoethyl)imidazole .2HCl

N-(2-(1-Imidazolyl)ethyl)phthalimide (0.231g) and hydrazine hydrate (0.93 ml) were stirred in ethanol (10 ml) for 1 h. The mixture was evaporatd, and the residue taken up in water (2 ml), acidified with 5 M hydrochloric acid and left to stand overnight. A white precipitate was filtered off and the filtrate freeze-dried. The product was dissolved in methanol, filtered, and evaporated. This gave the title compound (0.158g) as a yellow solid.

NMR (δ) ($CDCl_3$): 3.5-3.6 (2H, m), 4.6-4.7 (2H, m), 7.6-7.7 (1H, m), 7.75-7.8 (1H, m), 9.1-9.2 (1H, m).

c) BOC-ACHPA 2-(1-imidazolyl)ethylamide

This material was formed from BOC-ACHPA-OH (0.225g) and 1-(2-aminoethyl)imidazole .2HCl (0.15g), following the procedure of Description 1(a), but incorporating the addition of DIPEA (0.3 ml) to the reaction mixture. This gave the title compound (0.187g) as a white foam.

NMR (δ) ($CDCl_3$): 0.7-0.85 (2H, m), 1.05-1.55 (15H, m), 1.55-1.85 (5H, m), 2.25-2.5 (2H, m), 3.4-3.8 (6H, m), 3.9-4.0 (1H, m), 4.05-4.2 (1H, m), 4.7-4.9 (1H, m), 6.85-6.95 (1H, s), 6.95-7.05 (1H, s), 7.5-7.6 (1H, s).

Description 15

a) N-(4-(1-Imidazolyl)butyl)phthalimide

This material was formed from N-(4-bromobutyl)phthalimide (2.08g) and imidazole (1.01g), following the procedure of Description 14(a), but with a reaction time of 3 h. The crude product was chromatographed on silica gel using methanol/chloroform (0-5% methanol, gradient). This gave the title compound (1.263g) as a yellow oil.

NMR (δ) ($CDCl_3$): 1.70 (2H, m), 1.85 (2H, m), 3.7 (2H, t), 4.0 (2H, t), 6.9 (1H, s), 7.05 (1H, s), 7.5 (1H, s), 7.7 (2H, m), 7.85 (2H, m).

b) 1-(4-Aminobutyl)imidazole .2HCl

This material was formed from N-(4-(1-imidazolyl)butyl)phthalimide (0.282g), following the procedure of Description 14(b). This gave the title compound (0.22g) as a white solid.

NMR (δ) ($CD_3OD$): 1.7 (2H, m), 2.0 (2H, m), 3.0 (2H, m), 4.35 (2H, m), 7.6 (1H, m), 7.75 (1H, m), 9.05 (1H, m).

c) BOC-ACHPA 4-(1-imidazolyl)butylamide

This material was formed from BOC-ACHPA-OH (0.24g) and 1-(4-aminobutyl)imidazole .2HCl (0.209g), following the procedure of Description 14(c). This gave the title compound (0.364g) as a white foam.

NMR (δ) ($CDCl_3$): 0.9 (2H, m), 1.1-1.9 (24H, m), 2.35 (2H, m), 3.25 (2H, q), 3.5-4.1 (5H, m), 4.8 (1H, d), 6.7 (1H, m), 6.9 (1H, s), 7.05 (1H, s), 7.5 (1H, m).

Description 16

a) Ethyl (5-carboxy-2,3-dihydrobenzofuran-3-yl)acetate

To ethyl (5-formyl-2,3-dihydrobenzofuran-3-yl)acetate (2.00g) (Description 11(a)) in water (100 ml) at 75°C was added, with vigorous stirring, potassium permanganate (2.00g) in water (50 ml), over 10 min. After stirring at 75°C for a further 30 min, 5M HCl (10 ml) was added, followed by sufficient sodium metabisulphite (solid) to give a clear solution, which was cooled and extracted with ethyl acetate (3x50 ml). These combined organic extracts were then extracted with 5% sodium hydrogen carbonate solution (3x30 ml). Acidification of these combined aqueous extracts, extraction into chloroform (3x30 ml), drying ($Na_2SO_4$) and evaporation under reduced pressure gve the title compound (1.01g) as a brown solid.

NMR (δ) ($CDCl_3$): 1.3 (3H,t), 2.55-2.9 (2H,ABX), 3.9 (1H,m), 4.2 (2H,q), 4.4 (1H,dd), 4.9 (1H,t), 6.85 (1H,d), 7.9 (1H,s), 8.0 (1H,d).

b) Ethyl (5-(benzyloxycarbonyl)-2,3-dihydrobenzofuran-3-yl)acetate

Ethyl (5-carboxy-2,3-dihydrobenzofuran-3-yl)acetate (0.39g), anhydrous potassium carbonate (0.33g) and benzyl bromide (0.21 ml) were stirred for 60h in dry DMF (4 ml). The mixture was diluted with ethyl acetate (50 ml), washed twice with water, and with brine; dried ($Na_2SO_4$) and evaporated. The product was chromatographed on silica gel using ether/petroleum ether (b.p. 60-80°) (0-30% ether, gradient). This gave the title compound (0.48g) as a colourless oil.

NMR (δ) ($CDCl_3$): 1.2 (3H,t), 2.7 (2H,ABX), 3.6-4.5 (4H,t,q,m), 4.8 (1H,t), 5.3 (2H,s), 6.7 (1H,d), 7.1-7.4 (5H,s), 7.7-8.0 (2H,m).

c) (5-(Benzyloxycarbonyl)-2,3-dihydrobenzofuran-3-yl)acetic acid

Ethyl (5-(benzyloxycarbonyl)-2,3-dihydrobenzofuran-3-yl)acetate (1.54g) was stirred in dioxan (20 ml). Lithium hydroxide hydrate (0.19g) was added in water (10 ml). After stirring for 16h, the mixture was poured into water (200 ml), acidified (5M HCl), and the product was extracted into chloroform and evaporated. The residue was dissolved in ethyl acetate, and extracted into 5% sodium hydrogen carbonate solution. This extract was acidified (5M HCl), and the product was extracted into chloroform, dried ($Na_2SO_4$) and evaporated, giving the title compound (1.17g) as an off-white solid.

NMR (δ) ($CDCl_3$): 2.65 (1H) and 2.95 (1H) (ABX), 3.9 (1H,m), 4.35 (1H,dd), 4.85 (1H,t), 5.3 (2H,s), 6.8 (1H,d), 7.3-7.5 (5H,m), 7.9-8.0 (2H,m).

Description 17

a) Methyl 5-aminopentanoate .HCl

5-Aminopentanoic acid hydrochloride (5.52g) was dissolved in methanol (20 ml), and cooled in ice. Thionyl chloride (8 ml) was added dropwise with stirring. After stirring for 16h, with warming to ambient temperature, the mixture was evaporated. This gave the title compound (5.95g) as a white solid.

NMR (δ) ($CD_3OD$): 1.7 (4H,m), 2.45 (2H,m), 2.95 (2H,m), 3.7 (3H,s).

b) BOC-ACHPA-NH$(CH_2)_4CO_2Me$

This material was formed from methyl 5-aminopentanoate .HCl (0.14g), following the proceedure of Description 14(c). This gave the title compound (0.31g) as a yellow oil.

NMR (δ) ($CDCl_3$): 0.8-1.05 (2H,m), 1.1-1.9 (24H,m), 2.25-2.45 (5H,m), 3.25 (2H,q), 3.55-3.65 (1H,m), 3.7 (3H,s), 3.95 (1H,m), 4.75 (1H,m), 6.3 (1H,m).

Description 18

a) N-(4-(4-Methylpiperazin-1-yl)butyl)phthalimide

This material was formed from N-(4-bromobutyl)phthalimide (1.28g) and 1-methylpiperazine (1.0 ml), following the procedure of Description 14(a), but at room temperature. This gave the title compound (0.61g) as a yellow oil.

NMR (δ) ($CDCl_3$): 1.55 (2H,m), 1.7 (2H,m), 2.2-2.8 (13H,m), 3.7 (2H,t), 7.7 (2H,m), 7.85 (2H,m).

b) 1-(4-Aminobutyl)-4-methylpiperazine .3HCl

This material was formed from N-(4-(4-methylpiperazin-1-yl)butyl)phthalimide (0.25g), following the procedure of Description 14(b). This gave the title compound (0.29g) as a white solid.

NMR (δ) ($CD_3OD$): 1.7-2.1 (4H,m), 2.95-3.15 (4H,m), 3.3-3.5 (2H,m), 3.55-4.2 (9H,m).

c) BOC-ACHPA 4-(4-methylpiperazin-1-yl)butylamide

This material was formed from 1-(4-aminobutyl)-4-methylpiperazine .3HCl (0.23g), following the procedure of Description 14(c). This gave the title compound (0.33g).

NMR (δ) ($CDCl_3$): 0.8-1.05 (2H,m), 1.1-1.4 (4H,m), 1.4-1.9 (20H,m), 2.3-2.8 (16H,m), 3.3 (2H,m), 3.6 (1H,m), 3.7 (1H,t), 3.95 (1H,m), 4.75 (1H,m).

Description 19

a) N-(3-(4-Methylpiperazin-1-yl)propyl)phthalimide

This material was formed from N-(3-bromopropyl)phthalimide (1.21g) following the procedure of Description 18(a). This gave the title compound (0.66g) as a yellow oil.

NMR (δ) ($CDCl_3$): 1.85 (2H,quintet), 2.2 (3H,s), 2.5 (2H,t), 2.0-2.8 (8H,b), 3.65 (2H,t), 7.7 (2H,m), 7.85 (2H,m).

b) 1-(3-Aminopropyl)-4-methylpiperazine .3HCl

This material was formed from N-(3-(4-methylpiperazin-1-yl)propyl)phthalimide (0.36g), following the procedure of Description 14(b). This gave the title compound (0.34g).

NMR (δ) ($CD_3OD$): 2.25 (2H,m), 3.0-3.2 (4H,m), 3.4-3.8 (6H,m), 3.8-4.1 (5H,m).

c) BOC-ACHPA 3-(4-methylpiperazin-1-yl)propylamide

This material was formed from 1-(3-aminopropyl)-4-methylpiperazine .3HCl (0.21g), following the procedure of Description 14(c). This gave the title compound (0.28g).

NMR (δ) ($CDCl_3$): 0.75-1.05 (2H,m), 1.1-1.4 (6H,m), 1.4-1.55 (9H,m), 1.6-1.9 (7H,m), 2.2-2.3 (4H,m), 2.35-2.7 (10H,m), 3.35 (2H,m), 3.6 (2H,m), 3.95 (3H,m), 4.8 (1H,m).

Description 20

a) N-(2-(4-Methylpiperazin-1-yl)ethyl)phthalimide

This material was formed from N-(2-bromoethyl)phthalimide (3.45g), following the procedure of Description 18(a). The crude product was purified by chromatography on silica gel using methanol/chloroform (0-10% methanol, gradient). This gave the title compound (0.54g) as a cream solid.

NMR (δ) ($CDCl_3$): 2.25 (3H,s), 2.65 (2H,t), 2.3-2.8 (8H,b), 3.8 (2H,t), 7.7 (2H,m), 7.85 (2H,m).

b) 1-(2-Aminoethyl)-4-methylpiperazine .3HCl

This material was formed from N-(2-(4-methylpiperazin-1-yl)ethyl)phthalimide (0.25g), following the procedure of Description 14(b). This gave the title compound (0.25g) as a slightly impure yellow foam.

NMR (δ) ($CD_3OD$): 2.55 (2H,m), 2.75 (2H,m), 2.9 (3H,s), 3.0-3.3 (6H,m), 3.5 (3H,m), 3.8 (1H,m).

c) BOC-ACHPA 2-(4-methylpiperazin-1-yl)ethylamide

This material was formed 1-(2-aminoethyl)-4-methylpiperazine .3HCl (0.25g), following the procedure of Description 14(c). This gave the title compound (0.23g).

NMR (δ) ($CDCl_3$): 0.9 (2H,m), 1.1-1.4 (5H,m), 1.5 (9H,m), 1.6-1.9 (6H,m), 2.2-2.7 (15H,m), 3.3 (1H,m), 3.5 (1H,m), 3.6 (1H,m), 3.8-4.2 (3H,m), 4.8 (1H,m).

Description 21

BOC-ACHPA 3-(dimethylamino)propylamide

This material was formed from BOC-ACHPA-OH (0.23g) and 3-dimethylaminopropylamine (0.1 ml) following the procedure of Description 1(a). This gave the title compound (0.27g) as a yellow oil.

NMR (δ) ($CDCl_3$): 0.75-1.05 (2H,m), 1.1-1.4 (3H,m), 1.4-1.6 (10H,m), 1.6-1.9 (1H,m), 2.25 (6H,s), 2.4 (2H,t), 2.15-2.7 (3H,m), 3.35 (2H,m), 3.6 (1H,m), 3.95 (1H,d), 4.8 (1H,d), 7.35 (1H,m).

Description 22

a) Ethyl (2,3-dihydro-3-oxo-1H-isoindol-1-ylidine)acetate

(Carbethoxymethylene)triphenylphosphorane (4.15g) and phthalimide (0.81g) were heated at reflux in dry toluene (20 ml) under nitrogen for 18h. Solvent was removed in vacuo, and the product was chromatographed on silica gel using ether/petroleum ether (b.p. 60-80°C) (50-70% ether, gradient). This gave the title compound (0.87g) as a light yellow powder. NMR indicates the presence of a single isomer. (W. Flitsch, S.R. Schindler, Syntheesis 1975, 685.)

NMR (δ) ($CDCl_3$): 1.35 (3H,t), 4.3 (2H,q), 5.8 (1H,s), 7.6-7.9 (4H,m), 9.65 (1H,b).

b) Ethyl 2,3-dihydro-3-oxo-1H-isoindole-1-acetate

Ethyl (2,3-dihydro-3-oxo-1H-isoindol-1-ylidine)acetate was hydrogenated over 10% palladium on charcoal (60% aqueous paste, 0.15g) in ethanol (25 ml) for 4h. Catalyst was filtered off, and evaporation of solvent afforded the title compound (0.17g) as a white solid.

NMR (δ) ($DMSOd_6$): 1.15 (3H,t), 2.6 and 2.95 (2H,ABX), 4.1 (2H,q), 4.9 (1H,t), 7.5-7.7 (4H,m), 8.7 (1H,b).

c) 2,3-Dihydro-3-oxo-1H-isoindole-1-acetic acid

This material was formed from ethyl 2,3-dihydro-3-oxo-1H-isoindole-1-acetate (0.17g), following the procedure of Description 10(i). This gave the title compound (0.042g) as a white solid.

NMR (δ) ($DMSOd_6$): 2.5 and 2.8 (2H,ABX), 4.9 (1H,t), 7.4-7.7 (4H,m), 8.6 (1H,s), 12.3-12.8 (1H,b).

d) (2,3-Dihydro-3-oxo-1H-isoindole-1-acetyl)-Phe-Leu benzyl ester

This material was formed from 2,3-dihydro-3-oxo-1H-isoindole-1-acetic acid (0.58g) and Phe-Leu benzyl ester .$CF_3CO_2H$ (1.61g), following the method of Description 14(c). The crude product was chromatographed on silica gel using methanol/chloroform (0-2% methanol, gradient). This gave the title compound (1.02g) as a yellow solid.

NMR (δ) ($CDCl_3$): 0.8 (6H,m), 1.55 (3H,m), 2.05 (1H,s), 2.25 (1H,m), 2.8-3.2 (3H,m), 4.5 (1H,m), 4.8 (1H,m), 5.1 (1H,m), 5.15 (2H,d), 6.85 (0.5H,d), 7.05 (1H,m), 7.15 (2H,s), 7.15-7.3 (2.5H,m), 7.3 (5H,m), 7.35-7.55 (3H,m), 7.7-7.85 (1.5H,m), 7.9 (0.5H).

e) (2,3-Dihydro-3-oxo-1H-isoindole-1-acetyl)Phe-Leu-OH

This material was formed from (2,3-dihydro-3-oxo-1H-isoindole-1-acetyl-Phe-Leu benzyl ester (1.02g), following the procedure of Description 22(b). This gave the title compound (0.76g).

NMR (δ) ($CD_3OD$): 0.95 (6H,m), 1.6-1.8 (3H,m), 2.5 (1H,m), 2.65 (1H,m), 2.85 (1H,m), 3.15-3.25 (1H,m), 4.5 (1H,t), 4.7-4.85 (1H,m), 7.25 (5H,m), 7.4-7.6 (3H,m), 7.7-7.8 (1H,m), 8.3-8.5 (1H,m).

Description 23

a) 4-(BOC-amino)butanoic acid

4-Aminobutanoic acid (10.44g) was dissolved in a mixture of dioxan (200 ml) and 0.5M aqueous sodium hydroxide (200 ml), and cooled in ice. Di-tert-butyldicarbonate (24g) was added, and the mixture was stirred for 0.5h, warming to ambient temperature. Dioxan was removed under reduced pressure, and ethyl acetate (100 ml) was added. After acidification to pH 3 (5M hydrochloric acid), the mixture was extracted with ethyl acetate. The extract was dried ($Na_2SO_4$) and evaporated in vacuo, giving the title compound (18.3g) as a yellow oil.

NMR (δ) ($CDCl_3$): 1.45 (9H,s), 1.8 (2H,quintet), 2.4 (2H,t), 3.2 (2H,b), 4.7 (1H,b).

b) Benzyl 4-(BOC-amino)butanoate

This material was formed from 4-(BOC-amino)butanoic acid (18.3g), following the procedure of Description 8(a). This gave the title compound (24.4g).

NMR (δ) ($CDCl_3$): 1.45 (9H,s), 1.8 (2H,quintet), 2.4 (2H,t), 3.15 (2H,q), 5.2 (2H,s), 7.35 (5H,s).

c) BOC-ACHPA-$NH(CH_2)_3CO_2CH_2Ph$

This material was formed from BOC-ACHPA-OH (0.93g) and benzyl 4-(BOC-amino)butanoate (1.15g), following the procedure of Description 1(b). This gave the title compound (1.10g).

NMR (δ) ($CDCl_3$): 0.8-2.0 (24H,m), 2.1-2.5 (4H,m), 3.3 (2H,q), 3.6 (1H,m), 3.95 (1H,m), 4.75 (1H,m), 5.1 (2H,s), 6.25 (1H,b), 7.35 (5H,s).

d) ACHPA-$NH(CH_2)_3CO_2CH_2Ph$ .$CF_3CO_2H$

BOC-ACHPA-$NH(CH_2)_3CO_2CH_2Ph$ (1.10g) was dissolved in dichloromethane (20 ml), and trifluoroacetic acid (20 ml) was added. After standing for 0.5h, evaporation in vacuo gave the title compound (1.43g).

NMR (δ) ($CD_3OD$): 0.9 (2H,m), 1.0-1.5 (6H,m), 1.5-1.8 (7H,m), 2.3-2.5 (4H,m), 3.15 (2H,t), 3.9 (1H,m), 5.0 (2H,s), 7.25 (5H,s).

e) BOC-Phe-Leu-ACHPA-$NH(CH_2)_3CO_2CH_2Ph$

This material was formed from ACHPA-$NH(CH_2)_3CO_2CH_2Ph$ .$CF_3CO_2H$ (0.50g) and BOC-Phe-Leu-OH (1.1g), following the procedure of Description 14(c). This gave the title compound.

NMR (δ) ($CDCl_3$): 0.8-2.0 (33H,m), 2.1-2.5 (4H,m), 2.9-3.15 (3H,m), 3.3 (1H,q), 4.0 (1H,m), 4.2-4.65 (3H,m), 5.1 (2H,s), 5.2 and 5.4 (1H,2xb), 6.5-6.7 (2H,m), 6.9 (1H,m), 7.1-8.4 (10H,m).

Description 24

a) 3-(BOC-amino)propanoic acid

This material was formed from β-alanine (1.78g), following the procedure of Description 23(a). This gave the title compound (3.41g) as a white solid.

NMR (δ) ($CDCl_3$): 1.45 (9H,s), 2.55 (2H,m), 3.4 (2H,m), 5.1(b) and 6.35(b) (1H), 10.1 (1H,b).

b) Benzyl 3-(BOC-amino)propanoate

This material was formed from 3-(BOC-amino)propanoic acid (1.45g), following the procedure of Description 8(a). The crude product was chromatographed on silica gel using methanol/chloroform (0-2% methanol, gradient). This gave the title compound (2.1g).

NMR (δ) ($CDCl_3$): 1.45 (9H,s), 2.55 (2H,t), 3.4 (2H,m), 5.05 (1H,b), 5.15 (2H,s), 7.35 (5H,m).

c) BOC-ACHPA-NH$(CH_2)_2CO_2CH_2$Ph

This material was formed from BOC-ACHPA-OH (0.25g) and benzyl 3-(BOC-amino)propanoate (0.29g), following the procedure of Description 1(b). This gave the title compound (0.38g).

NMR (δ) ($CDCl_3$): 0.7-1.05 (2H,m), 1.1-1.4 (4H,m), 1.4-1.55 (9H,m), 1.6-1.9 (7H,m), 2.15-2.5 (3H,m), 2.6 (2H,t), 3.45-3.65 (3H,m), 3.95 (2H,d), 4.75 (1H,d), 5.15 (2H,s), 7.35 (5H,s).

Description 25

a) 2(S)-(CBZ-amino)-4-(BOC-amino)butanoic acid

This material was formed from 2(S)-(CBZ-amino)-4-aminobutanoic acid (0.27g), following the procedure of Description 23(a). This gave the title compound (0.23g) as a colourless oil.

NMR (δ) ($CDCl_3$): 1.35-1.6 (10H,m), 2.0 (1H,m), 2.4 (0.5H,m), 3.1 (2H,m), 3.4 (0.5H,m), 4.4 (1H,m), 5.1 (2H,s), 5.2 (0.5H,m), 5.8 (1H,m), 6.8 (0.5H,m), 7.35 (5H,s).

b) Benzyl 2(S)-(CBZ-amino)-4-(BOC-amino)butanoate

This material was formed from 2(S)-(CBZ-amino)-4-(BOC-amino)butanoic acid (0.31g), following the procedure of Description 8(a). This gave the title compound (0.37g) as a yellow oil.

NMR (δ) ($CDCl_3$): 1.4 (9H,s), 1.55 (0.5H,m), 1.7 (1H,m), 2.05 (1H,m), 3.0 (1H,m), 3.4 (1H,m), 4.5 (1H,m), 5.05 (1H,m), 5.1 (2H,s), 5.15 (2H,s), 5.6 (0.5H,m), 7.3-7.4 (10H,m).

c) (S)-BOC-ACHPA-NH$(CH_2)_2$CH(NH-CBZ)$CO_2CH_2$Ph

This material was formed from BOC-ACHPA-OH (0.23g) and benzyl 2(S)-(CBZ-amino)-4-(BOC-amino)-butanoate (0.35g), following the procedure of Description 1(b). This gave the title compound (0.47g).

NMR (δ) ($CDCl_3$): 0.75-1.05 (2H,m), 1.05-1.35 (6H,m), 1.35-1.6 (10H,m), 1.6-1.9 (6H,m), 2.0-2.45 (3H,m), 3.05 (1H,m), 3.55 (2H,m), 3.95 (1H,d), 4.45 (1H,m), 4.75 (1H,m), 5.1 (2H,s), 5.15 (2H,s), 5.7 (1H,m), 6.55 (1H,m), 7.35 (10H,s).

d) (2,3-Dihydro-1,1-dioxobenzothiophene-3-yl)acetyl-Phe-Leu-ACHPA-NH$(CH_2)_2$-(S)-CH(NH-CBZ)$CO_2CH_2$Ph

This material was formed from (S)-BOC-ACHPA-NH$(CH_2)_2$CH(NH-CBZ)$CO_2CH_2$Ph (0.47g), following the procedure of Description 8(c). The crude product was chromatographed on silica gel using

methanol/chloroform (0-2% methanol, gradient). This gave the title compound (0.43g) as a white foam.

NMR (δ) ($CD_3OD$): 0.7-0.85 (4H,m), 0.85-1.0 (4H,m), 1.05-1.45 (7H,m), 1.45-1.75 (7H,m), 1.75-1.9 (2H,m), 2.1 (1H,m), 2.25 (2H,m), 2.4-2.6 (1H,m), 2.75-2.9 (2H,m), 2.9-3.15 (1H,m), 3.15-3.3 (2H,m), 3.4-3.6 (2H,m), 3.85 (0.5H,m), 3.95 (2.5H,m), 4.2-4.4 (2H,m), 4.55 (0.5H,m), 5.05 (2H,m), 5.1 (2H,m), 7.2-7.4 (15H,m), 7.4-7.55 (2H,m), 7.55-7.7 (2H,m).

Description 26

Phe-Leu-ACHPA 3-(1-imidazolyl)propylamide

This material was formed from CBZ-Phe-Leu-ACHPA 3-(1-imidazolyl)propylamide (3.0g) (Description 1-(b)), following the procedure of Description 22(b). The crude product was chromatographed on silica gel using methanol/chloroform (0-16% methanol, gradient). This gave the title compound (1.4g) as a light yellow powder.

NMR (δ) ($DMSOd_6$): 0.7-1.9 (24H,m), 2.1 (2H,m), 2.55-2.7 (1H,m), 3.0 (3H,m), 3.8 (2H,m), 3.9 (2H,t), 4.3 (1H,q), 4.9 (1H,m), 6.85 (1H,b), 7.1-7.3 (6H,m), 7.4-7.8 (4H,m), 8.2 (1H,d).

Description 27

(2,3-Dihydro-1,1-dioxobenzothiophene-3-yl)acetyl-Phe-Leu-OH

This material was formed from (2,3-dihydro-1,1-dioxobenzothiophene-3-yl)acetic acid (0.50g) (Description 10(b)) and Phe-Leu methyl ester .$CH_3CO_2H$ (0.65g), following the procedure of Description 14-(c), and subsequent hydrolysis following the procedure of Description 10(i). This gave the title compound (0.50g).

NMR (δ) ($CDCl_3$): 0.6-2.0 (9H,m), 2.0-5.1 (9H,m), 6.6-8.1 (11H,m), 9.4 (1H,b).

Description 28

a) 2-(Pent-4-en-1-yl)-2,3-dihydro-1,1-dioxobenzothiophene

2,3-Dihydro-1,1-dioxobenzothiophene (2.50g) was dissolved in THF (20 ml) and cooled to -30° under nitrogen. N-Butyl lithium (1.6M in hexane, 9.3 ml) was added dropwise. After 1h 5-bromopent-1-ene (1.8 ml) was added in one portion and stirring continued for 3h. The solvent was removed under reduced pressure and the residues partitioned between dichloromethane (100 ml) and water (100 ml). The organic layer was washed with brine (2x100 ml) and dried ($Na_2SO_4$). Removal of the solvents gave a crude product which was purified by chromatography on silica gel using methanol/chloroform (0-1% methanol, gradient). This gave the title compound (0.93g) as a slightly coloured oil.

NMR (δ) ($CDCl_3$): 1.6-1.9 (3H,m), 2.0-2.3 (3H,m), 2.9-3.0 (1H,m), 3.4-3.5 (2H,m), 4.9-5.1 (2H,m), 5.7-5.9 (1H,m), 7.3-7.6 (3H,m), 7.7 (1H,m).

b) 4-(2,3-Dihydro-1,1-dioxobenzothiophene-2-yl)butanoic acid

2-(Pent-4-en-1-yl)-2, 3-dihydro-1,1-dioxobenzothiophene (1.26g) was taken up in acetone (50 ml) at 0°C and sodium hydrogen carbonate (0.22g) added in one portion. Potassium permanganate (3.24g) was added over 1.5h. After removal of the acetone under reduced pressure the residues were taken up in water (100 ml) and dilute sulphuric acid (5 ml) and sodium sulphite added. When the solution was clear it was extracted with ethyl acetate (3x50 ml). The combined organic layers were extracted with sodium hydrogen carbonate solution (2x50 ml). The combined aqueous phases were acidified (5M hydrochloric acid) and extracted with ethyl acetate (3x50 ml). After drying ($Na_2SO_4$) solvents were removed in vacuo to give the title compound (0.96g) as a white solid.

NMR (δ) ($DMSOd_6$): 1.6-1.8 (2H,m), 1.8-2.0 (1H,m), 2.3 (2H,m), 2.8-3.0 (1H,m), 3.4-3.7 (2H,m), 7.4-7.8 (4H,m), 12.1 (1H,bs).

c) 4-(6-Nitro-2,3-dihydro-1,1-dioxobenzothiophene-2-yl)butanoic acid

4-(2,3-Dihydro-1,1-dioxobenzothiophene-2-yl)butanoic acid (0.96g) was added portionwise to fuming nitric acid (6 ml) cooled in a waterbath. Stirring was continued for 4h. The solution was poured into water (100 ml) and extracted with ethyl acetate (2x30 ml). The combined organic layers were dried ($Na_2SO_4$) and solvents removed in vacuo. Double recrystallisation from ethyl acetate/ petroleum ether (b.p. 60-80°C) then gave the title compound (0.47g) as a white solid.

NMR (δ) ($DMSOd_6$): 1.6-1.7 (2H,m), 1.9-2.0 (1H,m), 2.3-2.4 (2H,m), 3.0-3.1 (1H,m), 3.6-3.9 (2H,m), 7.8 (1H,d), 8.4-8.6 (2H,m).

Description 29

a) tert-Butyl 2,3-dihydro-2-oxo-1H-benzimidazole-1-propanoate

2-Hydroxybenzimidazole (3.0g), anhydrous potassium carbonate (4.64g), and tert-butyl acrylate (3.4 ml) were stirred in DMF (50 ml) for 16h. The mixture was diluted with ethyl acetate (300 ml), washed well with water and brine, dried ($Na_2SO_4$) and evaporated in vacuo, giving a milky oil. This was taken up in chloroform and filtered, and evaporated again. Recrystallisation from chloroform/petroleum ether (b.p. 60-80°C) then gave the title compound (0.93g) as a white solid.

NMR (δ) ($CDCl_3$): 1.4 (9H,s), 2.7 (2H,t), 4.15 (2H,t), 7.1 (4H,s), 9.5 (1H,b).

b) 2,3-Dihydro-2-oxo-1H-benzimidazole-1-propanoic acid

tert-Butyl 2,3-dihydro-2-oxo-1H-benzimidazole-1-propanoate (0.29g) was dissolved in a mixture of dichloromethane (5 ml) and trifluoroacetic acid (5 ml), and left to stand for 4h. Solvents were then removed in vacuo. Trituration with chloroform and filtration gave the title compound (0.18g) as a white solid.

NMR (δ) ($DMSOd_6$): 2.6 (2H,t), 4.0 (2H,t), 6.95 (3H,m), 7.15 (1H,m), 10.85 (1H,s).

Description 30

a) Ethyl 4-(4-bromophenylthio)acetoacetate

4-Bromothiophenol (5g) was dissolved in dry pyridine (18 ml). To this was added ethyl 4-chloroacetoacetate (3.65 ml) dropwise with stirring. After 15 minutes the reaction mixture was heated at 100° on an oil bath for 0.25h and then cooled to room temperature. Ether (15 ml) was added, then the pyridine dissolved in 5M hydrochloric acid. After separation the aqueous layer further extracted with ether (3x50 ml). The combined organic phases were washed with water (75 ml), brine (35 ml) and dried ($Na_2SO_4$). Removal of the solvent gave the title compound (7.60g).

b) Ethyl (5-brombenzothiophene-3-yl)acetate

Ethyl 4-(4-bromophenylthio)acetoacetate (7.60g) was added to stirred excess polyphosphoric acid and stirring continued at 65° for 4h. After cooling, the reaction mixture was poured into water (200 ml) and extracted with ether (3x200 ml). The combined ethereal extracts were washed with sodium carbonate solution (2x100 ml), dried ($Na_2SO_4$) and the solvent removed under reduced pressure to give the title compound (4g).

NMR (δ) ($CDCl_3$): 1.2-1.3 (3H,t), 3.8 (2H,s), 4.1-4.2 (2H,q), 7.4 (1H,s), 7.5 (1H,dd), 7.7 (1H,d), 7.9 (1H,m).

c) Ethyl (5-cyanobenzothiophene-3-yl)acetate

This material was formed from ethyl (5-bromobenzothiophene-3-yl)acetate (26.42g) following the procedure of Description 10(g). This gave the title compound (16.63g).

NMR (δ) ($CDCl_3$): 1.2-1.3 (3H,t), 3.8-3.9 (2H,s), 4.1-4.3 (2H,q), 7.5-7.6 (3H,m), 7.9-8.0 (1H,d), 8.1 (1H,m).

d) Ethyl (5-cyano-1,1-dioxobenzothiophene-3-yl)acetate

Ethyl (5-cyanobenzothiophene-3-yl)acetate (1.52g) was taken up in acetic acid (10 ml) and stirred with 30% hydrogen peroxide solution (3.8 ml) at room temperature for 5 mins. The mixture was then heated to reflux for 15 mins, cooled and diluted with water (20 ml). The resulting suspension was extracted with chloroform (5x30 ml) and the combined organic phases were dried ($Na_2SO_4$). Removal of the solvent gave the title compound (1.67g).

NMR (δ) ($CDCl_3$): 1.3-1.5 (3H,t), 4.2-4.4 (2H,q), 6.7 (1H,m), 7.8-8.1 (3H,m).

e) Ethyl (5-cyano-2,3-dihydro-1,1-dioxobenzothiophene-3-yl acetate

This material was formed from ethyl (5-cyano-1,1-dioxobenzothiophene-3-yl)acetate (0.50g) following the procedure of Description 22(b), but using ethyl acetate as solvent. This gave the title compound (0.48g).

NMR (δ) ($CDCl_3$): 1.2-1.4 (3H,m), 2.7-3.0 (2H,m), 3.3-3.5 (1H,dd), 3.7-3.8 (1H,dd), 4.0-4.3 (3H,m), 7.8-7.9 (3H,m).

f) Ethyl (5-(BOC-aminomethyl)-2,3-dihydro-1,1-dioxobenzothiophene-3-yl)acetate

This material was formed from ethyl (5-cyano-2,3-dihydro-1,1-dioxobenzothiophene-3-yl)acetate (0.83g), following the procedure of Description 10(h). Purification by chromatography on silica gel using chloroform afforded the title compound (0.34g) as a colourless oil.

NMR (δ) ($CDCl_3$): 1.2 (3H,t), 1.35 (9H,s), 2.75 (1H,dd), 2.95 (1H,dd), 3.4 (1H,dd), 3.6-3.85 (1H,m), 3.9 (1H,m), 4.0-4.25 (4H,m), 7.3-7.45 (2H,m), 7.5 (1H,t), 7.65 (1H,d).

g) (5-(BOC-aminomethyl)-2,3-dihydro-1,1-dioxobenzothiophene-3-yl)acetic acid

This material was formed from ethyl (5-(BOC-aminomethyl)-2,3-dihydro-1,1-dioxobenzothiophene-3-yl)-acetate (0.34g), following the procedure of Description 10(i). This gave the title compound (0.3g).

NMR (δ) ($DMSOd_6$): 1.4 (9H,s), 2.65 (1H,dd), 2.9 (1H,dd), 3.4 (1H,dd), 3.7-3.9 (2H,m), 4.2 (2H,d), 7.3-7.5 (3H,m), 7.7 (1H,d), 12.5 (1H,bs).

Description 31

a) (5-Formyl-2,3-dihydrobenzofuran-3-yl)acetic acid

This material was formed from ethyl (5-formyl-2,3-dihydrobenzofuran-3-yl)acetate (1.52g) (Description 11(a)), following the procedure of Description 10(i). This gave the title compound contaminated with ca.25% of an unidentified, dihydrobenzofuran-derived impurity, as a light yellow powder (1.24g).

NMR (δ) ($DMSOd_6$): 2.6-2.9 (2H,ABX), 3.85 (1H,m), 4.4 (1H,dd), 4.85 (1H,t), 6.95 (1H,d), 7.75 (1H,dd), 7.8 (1H,s), 9.8 (1H,s), 12.3 (1H,b).

b) tert-Butyl (5-formyl-2,3-dihydrobenzofuran-3-yl)acetate

A mixture of (5-formyl-2,3-dihydrobenzofuran-3-yl)acetic acid (3g) and concentrated sulphuric acid (0.25 ml) in dioxan (20 ml) was cooled in an acetone/solid carbon dioxide bath as isobutylene (60g) was added as

a liquid. The mixture was sealed in a pressure bomb, allowed to warm to room temperature and stirred for 72h. After cooling, the pressure was released and the excess isobutylene allowed to gradually boil off as the reactants reached room temperature. The reaction mixture was poured into ice cold ethyl acetate/10% aqueous sodium hydroxide (1:1). The organic layer was separated, washed with cold 10% aqueous sodium hydroxide, and brine, dried ($Na_2SO_4$) and the solvent evaporated in vacuo to afford the title compound (0.66g) as a light brown oil.

NMR (δ) ($CDCl_3$): 1.5 (9H,s), 2.4-2.9 (2H,m), 3.8 (1H,m), 4.4 (1H,dd), 4.9 (1H,t), 6.9 (1H,d), 7.7 (1H,m), 9.8 (1H,s).

c) tert-Butyl (5-(methoxycarbonylmethylaminomethyl)-2,3-dihydrobenzofuran-3-yl acetate

To a stirred mixture of tert-butyl (5-formyl-2,3-dihydrobenzofuran-3-yl)acetate (0.75g), triethylamine (0.4 ml) and predried powdered 4A molecular sieves (6g) in dry ethanol (30 ml) was added methyl glycinate .HCl (0.36g). After stirring for 3h, sodium cyanoborohydride (0.18g) was added portionwise over 1h and stirring continued at room temperature for 72h. Filtration and evaporation of the filtrate in vacuo afforded a yellow residue which was taken up in ethyl acetate and washed with water and brine. The organic layer was dried ($Na_2SO_4$) and evaporated to dryness in vacuo. Purification by chromatography on silica gel using ether afforded the title compound (0.26g) as a light green oil.

NMR (δ) ($CDCl_3$): 1.45 (9H,s), 2.4-2.8 (3H,m), 3.4 (2H,s), 3.7-3.9 (6H,m), 4.3 (1H,dd), 4.75 (1H,t), 6.7 (1H,d), 7.01-7.2 (2H,m).

d) (5-(Methoxycarbonylmethylaminomethyl)-2,3-dihydrobenzofuran-3-yl)acetic acid .$CF_3CO_2H$

A mixture of trifluoroacetic acid (8 ml) and glacial acetic acid (2 ml) at 0°C was added to tert-butyl (5-(methoxycarbonylmethylaminomethyl)-2,3-dihydrobenzo furan-3-yl)acetate (0.175g) and the resultant solution stirred at 0°C for 1.5h. Evaporation to dryness in vacuo afforded the title compound (0.18g).

NMR (δ) ($CD_3OD$): 2.5-2.9 (2H,m), 3.75-4.0 (6H,m), 4.2 (2H,s), 4.3 (1H,dd), 4.75 (1H,t), 6.8 (1H,d), 7.25 (1H,dd), 7.35 (1H,d).

M.S. (m/z) $C_{14}H_{17}NO_5$ requires 279.1107. Found: 279.1107.

e) (5-(N-BOC-N-(Methoxycarbonylmethyl)aminomethyl]-2,3-dihydrobenzofuran-3-yl)acetic acid

To an ice-cooled mixture of (5-(methoxycarbonylmethylaminomethyl)-2,3-dihydrobenzofuran-3-yl)acetic acid .$CF_3CO_2H$ (0.18g) and triethylamine (0.19 ml) in chloroform (10 ml) was added di-tert-butyl dicarbonate (0.11g). After stirring at room temperature for 18h, chloroform (100 ml) was added and the mixture washed with water and 10% citric acid. The chloroform layer was extracted with saturated sodium carbonate (3x10 ml). Combined basic extracts were acidified with solid citric acid and extracted into ethyl acetate. The ethyl acetate extracts were dried ($Na_2SO_4$) and evaporated in vacuo to afford the title compound (0.09g) as a light green semi-solid.

NMR (δ) ($CD_3OD$): 1.4 (9H,d), 2.4-2.75 (2H,m), 3.6 (3H,s), 3.65-3.9 (4H,m), 4.1 (1H,dd), 4.3 (2H,s), 4.6 (1H,t), 6.6 (1H,d), 6.9 (1H,bd), 7.05 (1H,bs).

M.S. (m/z) $C_{19}H_{25}NO_7$ requires 379.1631. Found: 379.1629.

f) (5-(N-BOC-N-(Methoxycarbonylmethyl)aminomethyl)-2,3-dihydrobenzofuran-3-yl)acetyl-Phe-Leu-ACHPA 3-(1-imidazolyl)propylamide

This material was formed from (5-(N-BOC-N-(methoxycarbonylmethyl) aminomethyl)-2,3-dihydrobenzofuran-3-yl)acetic acid (0.077g), following the procedure of Example 33. The crude product was chromatographed on silica gel using methanol/chloroform (0-12% methanol, gradient). This gave the title compound (0.15g) as a light green foam.

NMR (δ) ($CD_3OD$): 0.8-2.1 (33H,m), 2.3-2.5 (3H,m), 2.5-2.7 (1H,m), 2.8-3.0 (1H,m), 3.1-3.3 (3H,m), 3.6-3.75 (4H,m), 3.75-4.2 (7H,m), 4.3-4.5 (4H,m), 4.7 (1H,m), 6.65 (1H,m), 6.95 (1H,s), 7.0 (2H,m), 7.15 (1H,s), 7.25 (5H,m), 7.7 (1H,s).

M.S. (m/z) (FAB) (M+1) 944 (consistent with m.w. = 943).

g) (5-(N-BOC-N-(Carboxymethyl)aminomethyl)-2,3-dihydrobenzofuran-3-yl)acetyl-Phe-Leu-ACHPA 3-(1-imidazolyl)propylamide

This material was formed from (5-(N-BOC-N-(methoxycarbonylmethyl)aminomethyl)-2,3-dihydrobenzofuran-3-yl)acetyl-Phe-Leu-ACHPA 3-(1-imidazolyl)propylamide (0.131g), following the procedure of Description 10(i), except that the mixture was neutralised with 2M hydrochloric acid and then extracted into ethyl acetate. This gave the title compound (0.1g) as a light green foam.

NMR (δ) ($DMSOd_6$): 0.7-1.9 (33H,m), 2.0-2.3 (3H,m), 2.55-2.8 (3H,m), 3.0 (3H,m), 3.5-3.9 (6H,m), 3.95 (2H,t), 4.1 (1H,m), 4.3 (4H,m), 4.6 (1H,m), 6.7 (1H,m), 6.9 (1H,s), 7.0 (2H,m), 7.1-7.4 (7H,m), 7.6 (1H,s), 7.75 (1H,m), 8.2 (1H,m).

M.S. (m/z) (FAB) (M+1) = 930 (consistent with m.w. = 929).

Description 32

a) (5-(BOC-aminomethyl)-2,3-dihydro-1,1-dioxobenzothiophene-3-yl)acetyl-Phe-His methyl ester

This material was formed from (5-(BOC-aminomethyl)-2,3-dihydro-1,1-dioxobenzothiophene-3-yl)acetic acid (0.34g) (Description 30(g)) and Phe-His methyl ester .2($CF_3CO_2H$) (0.52g), following the coupling procedure of Description 1(b). The crude product was chromatographed on silica gel using methanol/chloroform (0-20% methanol, gradient). This gave the title compound (0.60g) as a white foam.

NMR (δ) ($DMSOd_6$): 1.4 (9H, 2xs), 2.45 (1H,m), 2.8 (2H,m), 2.9-4.0 (m), 4.25 (2H,m), 4.7 (2H,m), 6.85 (1H,d), 7.25 (6H,m), 7.3-7.45 (2H,m), 7.55-7.65 (2H,m), 8.4 (2H,m).

b) (5-(BOC-aminomethyl)-2,3-dihydro-1,1-dioxobenzothiophene-3-yl)acetyl-Phe-His-OH, and the corresponding hydrochloride salt

(5-(BOC-aminomethyl)-2,3-dihydro-1,1-dioxobenzothiophene-3-yl)acetyl-Phe-His methyl ester (0.48g) was hydrolysed following the procedure in Description 10(i). After dilution with water and neutralisation (5M hydrochloric acid), the mixture was extracted with ethyl acetate. The extract was dried ($Na_2SO_4$) and evaporated in vacuo to give the title free base (0.22g) as a white solid.

NMR (δ) ($DMSOd_6$): 1.4 (9H,s), 2.4 (m), 2.6-3.5 (m), 4.15 (m), 4.35 (2H,m), 4.6 (2H,m), 6.8 (1H,d), 7.1-7.3 (5H,m), 7.35 (2H,m), 7.5 (2H,m), 7.65 (1H,d), 8.4 (2H,m).

Acidification of the aqueous fraction (5M hydrochloric acid) and extraction as above gave the title hydrochloride salt (0.17g) as a white solid.

NMR (δ) ($DMSOd_6$): 1.4 (9H,s), 2.4 (m), 2.6-3.8 (m), 4.15 (2H,d), 4.55 (2H,m), 7.15-7.4 (8H,m), 7.5 (1H,m), 7.65 (1H,d), 8.45 (1H,d), 8.6 (1H,t), 8.8 (1H,d).

Description 33

a) Ethyl 4-phthalimidobutanoate

This material was formed from phthalimide (10.0g) and ethyl 4-bromobutyrate (9.7 ml) following the procedure of Description 8(a). The title compound (16.6g) was obtained as an off-white solid.

NMR (δ) ($CDCl_3$): 1.25 (3H,t), 2.0 (2H,m), 2.4 (2H,t), 3.75 (2H,t), 4.1 (2H,q), 7.7 (2H,m), 7.85 (2H,m).

b) Ethyl 4-(1,3-dihydro-1-oxo-2H-isoindol-2-yl)butanoate

Ethyl 4-phthalimidobutanoate (2.0g) was stirred at 0°C in methanol (120 ml). Sodium borohydride (0.58g) was added, followed after 20 min by 5M hydrochloric acid (1 ml). The reaction mixture was evaporated, and the residue partitioned between chloroform (100 ml) and water (20 ml). The organic phase was dried ($Na_2SO_4$) and evaporated to give the intermediate hydroxylactam, which was hydrogenated following the procedure of Description 22(b). The title compound (1.8g) was obtained as a clear oil.

NMR (δ) ($CDCl_3$): 1.2 (3H,t), 2.0 (2H, quint), 2.4 (2H,t), 3.55 (2H,t), 4.05 (2H,q), 4.4 (2H,s), 7.4-7.55 (3H,m), 7.85 (1H,m).

c) 4-(1,3-Dihydro-1-oxo-2H-isoindol-2-yl)butanoic acid

This material was formed from ethyl 4-(1,3-dihydro-1-oxo-2H-isoindol-2-yl)butanoate (0.26g) following the procedure of Description 10(i). This gave the title compound (0.23g) as a clear oil which crystallised on standing.

NMR (δ) ($CDCl_3$): 2.0 (2H, quint), 2.45 (2H,t), 3.7 (2H,t), 4.4 (2H,s), 7.4-7.6 (3H,m), 7.85 (1H,m), 8.9-10.7 (1H,b).

Description 34

a) N-(3-Phthalimidopropyl)-Pro methyl ester

This material was formed from N-(3-bromopropyl)phthalimide (1.45g) and Pro methyl ester .HCl (0.89g), following the procedure of Description 18(a). The crude product was purified by chromatography on silica gel using methanol/chloroform (0-2% methanol, gradient). This gave the title compound (0.88g) as a yellow oil.

NMR (δ) ($CDCl_3$): 1.7-2.0 (5H,m), 2.05 (1H,m), 2.3-2.55 (2H,m), 2.8 (1H,m), 3.2 (2H,m), 3.65-3.9 (2H,m), 3.7 (3H,s), 7.7 (2H,m), 7.85 (2H,m).

b) N-(3-Aminopropyl)-Pro methyl ester.2HCl

This material was formed from N-(3-phthalimidopropyl)-Pro methyl ester (0.88g), following the procedure of Description 14(a). This gave the title compound (0.83g) as a yellow solid.

NMR (δ) ($CD_3OD$): 2.0-2.45 (6H,m), 2.6 (2H,m), 3.05 (2H,t), 3.4-3.6 (2H,m), 3.8 (3H,s), 4.55 (1H,t).

c) BOC-ACHPA-NH($CH_2$)$_3$-Pro methyl ester

This material was formed from N-(3-aminopropyl)-Pro methyl ester .2HCl (0.38g) following the procedure of Description 14(c). The crude product was purified by chromatography on silica gel using methanol/chloroform (0-5% methanol, gradient). This gave the title compound (0.25g).

NMR (δ) ($CDCl_3$): 0.8-1.05 (2H,m), 1.05-1.35 (6H,m), 1.45 (9H,s), 1.5-1.8 (7H,m), 1.8-2.0 (5H,m), 2.0-2.55 (5H,m), 2.8 (1H,m), 3.2 (2H,m), 3.4 (2H,m), 3.6 (1H,m), 3.75 (3H,m), 4.0 (1H,d), 4.85 (1H,d).

Description 35

a) 3-(2-Aminoethyl)pyridine

3-Pyridylacetonitrile (0.5ml) was hydrogenated in ethanol (15 ml) in the presence of ammonia (sp.gr.0.88, 3 ml) over Raney Nickel (settled aqueous suspension, 1.5 ml) for 70 h. The catalyst was filtered off, the filtrate evaporated and the residue partitioned between saturated potassium carbonate solution and chloroform. The organic layer was dried dried ($K_2CO_3$) and evaporated to give the title compound (0.56g) as a yellow oil.

NMR (δ) ($CDCl_3$): 1.5 (2H,m), 2.75 (2H,m), 2.95 (2H,m), 7.25 (1H,m), 7.55 (1H,m), 8.45 (2H,m).

b) BOC-ACHPA 2-(3-pyridyl)ethylamide

This material was formed from BOC-ACHPA-OH (0.38g) and 3-(2-aminoethyl)pyridine (0.13g), following

the procedure of Description 1(a). The crude product was chromatographed on silica gel using methanol/chloroform (0-5% methanol, gradient). This gave the title compound (0.32g) as a yellow foam.

NMR (δ) ($CD_3OD$): 0.8-1.1 (1H,m), 1.15-1.6 (5H,m), 1.45 (9H,s), 1.6-1.9 (7H,m), 2.25 (2H,m), 2.85 (2H,t), 3.45 (2H,m), 3.65 (1H,m), 3.95 (1H,m), 6.2 (m), 7.4 (1H,m), 7.75 (1H,m), 8.15 (b), 8.4 (2H,m).

Description 36

a) 4-(2-Aminoethyl)pyridine.$2CF_3CO_2H$

This material was formed from 4-pyridylacetonitrile (0.5g), following the procedure of Description 35(a). The crude product was dissolved in dichloromethane (40 ml). Di-tert-butyl dicarbonate (0.88g) was added and the mixture stirred at room temperature for 16h. The reaction mixture was evaporated and chromatographed on silica gel using methanol/chloroform (0-5-methanol, gradient). The 4-(2-(BOC-amino)ethyl)-pyridine so isolated was converted to the title compound (0.45g) following the procedure of Description 23-(a).

NMR (δ) ($CD_3OD$): 3.35 (4H,m), 8.05 (2H,m), 8.85 (2H,m).

b) BOC-ACHPA 2-(4-pyridyl)ethylamide

This material was formed from BOC-ACHPA-OH (0.38g) and 4-(2-aminoethyl)pyridine.$2CF_3CO_2H$ (0.45g), following the procedure of Description 1(b). The crude product was chromatographed on silica gel using methanol/chloroform (0-5% methanol, gradient). This gave the title compound (0.38g) as a brown foam.

NMR (δ) ($CDCl_3$): 0.75-1.05 (2H,m), 1.05-1.4 (5H,m), 1.45 (9H,s), 1.5-1.8 (6H,m), 2.2-2.45 (2H,m), 2.85 (2H,t), 3.55 (3H,m), 3.9 (1H,m), 4.75 (1H,d), 6.7 (1H,m), 7.15 (2H,d), 8.45 (2H,d).

Example 1a

(2,3-Dihydro-1,1-dioxobenzothiophene-3-yl)acetyl-Phe-Leu-ACHPA 3-(1-imidazolyl)propylamide .$H_2O$

CBZ-Phe-Leu-ACHPA 3-(1-imidazolyl)propylamide (0.146g) (Description 1(b)) was hydrogenated over 10% palladium on charcoal (60% aqueous paste, 0.07g) in ethanol (20 ml) for 3h. The mixture was filtered, and the filtrate was evaporated. The product was dissolved in dry dimethylformamide (DMF) (5 ml), and (2,3-dihydro-1,1-dioxobenzothiophene-3-yl)acetic acid (0.051g) (Description 10(b)) and 1-hydroxybenzotriazole (HOBT) (0.030g) were added. The solution was cooled in ice, and 1-(3-dimethylaminopropyl)-3-ethylcarbodiimide hydrochloride (DEC) (0.043g) was added, stirring thereafter for 16h, and warming to ambient temperature. The solution was diluted with ethyl acetate (50 ml), washed with 5% sodium hydrogen carbonate solution, water, and brine, dried ($Na_2SO_4$) and evaporated. The crude product was chromatographed on silica gel using methanol/chloroform (0-10% methanol, gradient). This gave the title compound (0.080g).

NMR (δ) ($CDCl_3$): 0.7-1.05 (7H, m), 1.05-2.5 (21H, m), 2.5-3.8 (8H, m), 3.8-4.15 (4H, m), 4.15-4.35 (1H, m), 4.65-4.85 (1H, m), 6.5 and 6.6 (1H, 2xd), 6.8-7.4 (11H, m), 7.4-7.6 (2H, m), 7.7 (1H, d), 7.9 (1H, m), 8.0 and 8.25 (1H, 2xd).

Analysis: $C_{42}H_{58}N_6O_7S.H_2O$ requuires C,62.3; H,7.5; N,10.4%. Found: C,62.4; H,7.5; N,10.0%.

Example 1b

(2,3-Dihydro-1,1-dioxobenzothiophene-3-yl)acetyl-Phe-Leu-ACHPA 3-(1-imidazolyl)propylamide .HCl

The corresponding free base (Example 1(a)) (0.026g) was dissolved in methanol (2 ml), and acidified with excess hydrochloric acid (0.5 M). The solution was diluted with water (30 ml) and immediately freeze-dried. This gave the title compound (0.028g) as a fluffy white solid.

NMR (δ) ($DMSOd_6$): 0.7-1.0 (7H, m), 1.0-1.8 (17H, m), 1.9 (2H, m), 2.05-2.3 (2H, m), 2.6-2.9 (2H, m), 2.9-3.15 (4H, m), 3.7-3.95 (3H, m), 4.15 (2H, m), 4.35 (1H, m), 4.65 (1H, m), 4.9 (1H, m), 7.15-7.5 (7H, m), 7.5-7.9 (6H, m), 8.25-8.55 (2H, m), 8.9 (1H, s).

M.S. (m/z) (FAB) (M+1) = 791 (consistent with m.w. of free base = 790).

Example 2

(2,3-Dihydro-1,1-dioxobenzothiophene-3-yl)acetyl-Phe-Leu-ACHPA 2-(4-morpholinyl)ethylamide .$H_2O$

This material was formed from CBZ-Phe-Leu-ACHPA 2-(4-morpholinyl)ethylamide (0.163g) (Description 2(b)), following the procedure of Example 1(a). The crude product was chromatographed on silica gel using methanol/chloroform (0-10% methanol, gradient). This gave the title compound (0.074g).

NMR (δ) ($CDCl_3$): 0.7-1.0 (7H, m), 2.2-3.5 (16H, m), 3.5-3.8 (5H, m), 3.85-4.1 (3H, m), 4.3 (1H, m), 4.7 (1H, m).

Analysis: $C_{42}N_{61}N_5O_8S.H_2O$ requires C,62.0; H,7.8; N,8.6%. Found: C,61.8; H,7.6; N,8.5%.

Additional Example

The corresponding compound to that of Example 2, but wherein s is 3 is prepared analogously.

Example 3

(2,3-Dihydro-1,1-dioxobenzothiophene-3-yl)acetyl-Phe-Leu-ACHPA 3-(2-oxopyrrolidin-1-yl) propylamide

This material was formed from CBZ-Phe-Leu-ACHPA 3-(2-oxopyrrolidin-1-yl)propylamide (0.176g) (Description 3(b)), following the procedure of Example 1(a). The crude product was chromatographed on silica gel using methanol/chloroform (0-5% methanol, gradient). This gave the title compound (0.155g) as a white solid.

NMR (δ) ($CD_3OD$): 0.85-1.25 (8H, m), 1.25-1.6 (7H, m), 1.6-1.9 (9H, m), 1.9-2.25 (4H, m), 2.3-2.55 (4H, m), 2.6-3.05 (4H, m), 3.05-3.2 (3H, m), 3.25-3.5 (m), 3.5-3.65 (3H, m), 3.7-4.25 (6H, m), 4.3-4.8 (2H, m), 4.8-5.15 (m), 7.3-7.5 (5H, m), 7.55-7.85 (4H, m).

Analysis: $C_{43}H_{61}N_5O_8S.1.5\ H_2O$ requires C,61.9; H,7.7; N,8.4%. Found: C,61.7; H,7.5; N,8.0%.

M.S. (m/z) (FAB) (M+1) = 808 (consistent with m.w. = 807).

Example 4

(2,3-Dihydro-1,1-dioxobenzothiophene-3-yl)acetyl-Phe-Leu-ACHPA 2-(2-pyridyl)ethylamide .$H_2O$

This material was formed from CBZ-Phe-Leu-ACHPA 2-(2-pyridyl)ethylamide (0.141g) (Description 4(b)-), following the procedure of Example 1(a). The crude product was chromatographed on silica gel using methanol/chloroform (0-5% methanol, gradient). This gave the title compound (0.066g) as a white solid.

NMR (δ) ($CD_3OD$): 0.8-1.15 (8H, m), 1.2-1.55 (7H, m), 1.55-2.0 (9H, m), 2.25-2.4 (2H, m), 2.45-2.75 (1H, m), 2.8-3.2 (6H, m), 3.2-3.5 (m), 3.55-3.8 (3H, m), 3.8-4.2 (3H, m), 4.65-5.15 (m), 7.25-7.5 (9H, m), 7.5-7.95 (5H, m), 8.45-8.6 (1H, m).

Analysis: $C_{43}H_{57}N_5O_7S.H_2O$ requires C,64.1; H,7.4; N,8.7%. Found: C,64.5; H,7.3; N,8.6%.

M.S. (m/z) (FAB) (M+1) = 788 (consistent with m.w. = 787).

Example 5

(2,3-Dihydro-1,1-dioxobenzothiophene-3-yl)acetyl-Phe-Leu-ACHPA 2-hydroxyethylamide $.H_2O$

This material was formed from CBZ-Phe-Leu-ACHPA 2-hydroxyethylamide (0.046g) (Description 5(b)), following the procedure of Example 1(a). The crude product was chromatographed on silica gel using methanol/chloroform (0-10% methanol, gradient). This gave the title compound (0.027g) as a white solid.

NMR (δ) ($CD_3OD$): 0.65-1.05 (8H, m), 1.1-1.4 (6H, m), 1.45-1.80 (7H, m), 1 8-1.9 (1H, m), 2.15-2.4 (2H, m), 2.4-2.65 (1H, m), 2.7-3.1 (3H, m), 3.5-3.7 (2H, m), 3.75-3.9 (1H, m), 3.9-4.05 (2H, m), 4.3-4.45 (1H, m), 4.5-4.65 (1H, m), 7.1-7.45 (6H, m), 7.45-7.75 (3H, m).

Analysis: $C_{38}H_{57}N_4O_8S.H_2O$ requires C,61.3; H,7.6; N,7.5%. Found: C,61.0; H,7.4; N,7.5%.

M.S. (m/z) (FAB) (M+1) = 727 (consistent with m.w. = 726).

Example 6

(2,3-Dihydro-1,1-dioxobenzothiophene-3-yl acetyl-Phe-Leu-ACHPA 2-(acetylamino)ethylamide $.2H_2O$

This material was formed from BOC-ACHPA 2-(acetylamino)ethylamide (0.182g) (Description 6) and 2,3-dihydro-1,1-dioxobenzothiophene-3-yl)acetyl-Phe-Leu-ACHPA (0.205g) (Description 27) following the procedure of Description 1(b). The crude product was chromatographed on silica gel using methanol/chloroform (0-10% methanol, gradient). This gave the title compound (0.188g) as a yellow solid.

NMR (δ) ($CD_3OD$): 0.7-0.8 (3H, m), 0.8-1.05 (4H, m), 1.05-1.45 (7H, m), 1.5-1.9 (8H, m), 1.9-2.0 (3H, m), 2.2-2.4 (2H, m), 2.45-2.65 (1H, m), 2.75-2.9 (2H, m), 2.95-3.1 (1H, m), 3.1-3.4 (m), 3.45-3.6 (1H, m), 3.6-3.75 (0.5H, m), 3.8-3.9 (0.5H, m), 3.9-4.15 (2H, m), 4.2-4.3 (0.5H, m), 4.3-4.4 (0.5H, m), 4.5-4.6 (1H, m), 4.7-4.9 (m), 7.2-7.35 (5H,m), 7.35-7.7 (4H, m).

Analysis: $C_{40}H_{57}N_5O_8S.2H_2O$ requires C,59.8; H,7.6; N,8.7%. Found: C,59.9; H,7.2; N,8.5%.

M.S. (m/z) (FAB) (M+1) = 768 (consistent with m.w. = 767).

Example 7

(2,3-Dihydro-1,1-dioxobenzothiophene-3-yl acetyl-Phe-Leu-ACHPA-$NH(CH_2)_3CO_2Et$

This material was formed from BOC-ACHPA-$NH(CH_2)_3CO_2Et$ (0.25g) (Description 7) following the procedure of Example 6. The crude product was chromatographed on silica gel using methanol/chloroform (0-5% methanol, gradient). This gave the title compound (0.257g) as a yellow solid.

NMR (δ) ($CD_3OD$): 0.7-0.85 (4H, m), 0.9-1.0 (4H, m), 1.1-1.4 (9H, m), 1.45-1.85 (10H, m), 2.2-2.4 (4H, m), 2.45-2.65 (1H, m), 2.75-2.9 (2H, m), 2.95-3.05 (1H, m), 3.1-3.25 (3H, m), 3.4-3.65 (1H, m), 3.8-3.9 (0.5H, m), 3.9-4.0 (2H, m), 4.05-4.15 (2H, m), 4.25-4.3 (0.5H, dd), 4.35-4.4 (0.5H, m), 4.5-4.55 (0.5H, m), 7.2-7.35 (6H, m), 7.35-7.4 (0.5H, m), 7.4-7.45 (0.5H, m), 7.45-7.6 (2H, m), 7.6-7.7 (2H, m).

Analysis: $C_{42}H_{60}N_4O_9S$ requires C,63.3; H,7.6; N,7.0%. Found: C,63.2; H,7.6; N,7.0%.

M.S. (m/z) (FAB) (M+1) = 797 (consistent with m.w. = 796).

Example 8a

(2,3-Dihydro-1,1-dioxobenzothiophene-3-yl)acetyl-Phe-Leu-ACHPA-$NH(CH_2)_3CO_2H$ $.1.5H_2O$

(2,3-Dihydro-1,1-dioxobenzothiophene-3-yl)acetyl-Phe-Leu-ACHPA-$NH(CH_2)_3CO_2Et$ (0.118g) (Example 7) in ethanol (5 ml) and sodium hydroxide (10% aqueous solution, 0.08 ml) were stirred for 19 h. The solution was diluted with water (60 ml), washed with chloroform, acidified with 5 M hydrochloric acid, and extracted with chloroform. The extract was dried ($MgSO_4$), and evaporated to give the title compound (0.08g) as a yellow foam.

NMR (δ) ($CD_3OD$): 0.60-0.85 (3H, m), 0.85-1.05 (5H, m), 1.1-1.4 (6H, m), 1.45-1.9 (10H, m), 2.15-2.4 (4H, m), 2.4-2.65 (1H, m), 2.75-2.9 (2H, m), 2.95-3.1 (1H, m), 3.4-3.7 (1H, m), 3.8-3.9 (0.5H, m), 3.9-4.05 (2H, m), 4.2-4.3 (0.5H, m), 4.35-4.45 (0.5H, m), 4.45-4.6 (1H, m), 7.15-7.35 (5H, m), 7.35-7.4 (1H, m), 7.4-7.45 (1H, m), 7.5-7.6 (2H, m), 7.6-7.75 (2H, m).

Analysis: $C_{40}H_{56}N_4O_9S.1.5H_2O$ requires C,60.4; H,7.5; N,7.0%. Found: C,60.3; H,7.2; N,6.9%.

M.S. (m/z) (FAB) (M+1) 769 (consistent with m.w. = 768).

## Example 8b

(2,3-Dihydro-1,1-dioxobenzothiophene-3-yl acetyl-Phe-Leu-ACHPA-NH$(CH_2)_3CO_2Na$

The corresponding free acid (Example 8(a)) (0.44g) was dissolved in methanol (2 ml), and sodium hydroxide (1.15 mg/ml aqueous solution, 2.0 ml) was added. The solution was diluted with water (30 ml) and freeze-dried immediately giving the title compound (0.045g) as a fluffy white solid.

NMR (δ) ($CD_3OD$): 0.5-0.95 (8.5H, m), 1.0-1.35 (6.5H, m), 1.4-1.8 (9H, m), 2.05-2.25 (4H, m), 2.3-2.6 (1H, m), 2.65-2.85 (2H, m), 2.85-3.0 (1H, m), 3.0-3.15 (3H, m), 3.25-3.3 (0.25H, m), 3.3-3.4 (0.25H, m), 3.4-3.55 (0.25H, m), 3.7-3.8 (0.5H, m), 3.8-3.95 (2H, m), 4.15-4.2 (0.25H, m), 4.25-4.35 (0.5H, m), 4.4-4.5 (0.5H, m), 7.1-7.25 (5H, m), 7.25-7.3 (0.25H, d), 7.3-7.4 (0.25H, d), 7.4-7.5 (2H, m), 7.5-7.6 (2H, m).

## Example 9

(2,3-Dihydro-1,1-dioxobenzothiophene-3-yl)acetyl-Phe-Leu-ACHPA-NH$(CH_2)_3$-(S)-CH$(NH_2]CO_2H$ .HCl.$3H_2O$

(2,3-Dihydro-1,1-dioxobenzothiophene-3-yl) acetyl-Phe-Leu-ACHPA-NH$(CH_2)_3$-(S)-CH(NH-CBZ)-$CO_2CH_2Ph$ (0.203g) (Description 8(c)) was hydrogenated over 10% palladium on charcoal (60% aqueous paste, 0.28g) in ethanol (30 ml) for 4 h. The mixture was filtered and evaporated. The product was dissolved in methanol (2 ml) and acidified with a slight excess of dilute hydrochloric acid. The solution was diluted with water (30 ml) and immediately freeze-dried. This gave the title compound (0.046g) as a white solid.

NMR (δ) ($CD_3OD$): 0.7-1.0 (8H, m), 1.05-1.45 (8H, m), 1.5-1.8 (8H, m), 1.85-2.0 (2H, m), 2.2-2.35 (2H, m), 2.45-2.65 (1H, m), 2.75-2.9 (2H, m), 2.95-3.1 (2H, m), 3.1-3.15 (1H, m), 3.15-3.25 (m), 3.35-3.45 (1H, m), 3.45-3.5 (0.25H, m), 3.5-3.7 (0.5H, m), 3.8-3.9 (1H, m), 3.9-4.05 (2H, m), 4.2-4.3 (0.5H, m), 4.3-4.4 (0.5H, m), 4.45-4.55 (1H, m), 7.2-7.35 (5H, m), 7.35-7.75 (4H, m).

Analysis: $C_{41}H_{59}N_5O_9S.HCl.3H_2O$ requires C,55.4; H,7.5; N,7.9%. Found: C,55.2; H,7.1; N,7.9%.

M.S. (m/z) (FAB) (M+1) = 798 (consistent with m.w. of free base = 797).

## Example 10

(2,3-Dihydro-1,1-dioxobenzothiophene-3-yl)acetyl-Phe-Leu-ACHPA-NH$(CH_2)_4$-(S)-CH$(NH_2)CO_2H$ .HCl.$2H_2O$

This material was formed from (2,3-dihydro-1,1-dioxobenzothiophene-3-yl)acetyl-Phe-Leu-ACHPA-NH-$(CH_2)_4$-(S)-CH(NH-CBZ)$CO_2CH_2Ph$ (0.22g) (Description 9(c)) following the procedure of Example 9. This gave the title compound (0.114g) as a pale yellow solid.

NMR (δ) ($CD_3OD$): 0.75-0.95 (4H, m), 1.0-1.15 (5H, m), 1.15-1.85 (17H, m), 1.85-2.1 (2H, m), 2.3-2.45 (2H, m), 2.5-2.75 (1H, m), 2.8-3.05 (3H, m), 3.05-3.35 (3H, m), 3.5-3.8 (0.5H, m), 3.85-4.0 (1H, m), 4.0-4.15 (2H, m), 4.25-4.4 (0.5H, m), 4.4-4.55 (0.5H, m), 4.55-4.7 (1H, m), 7.3-7.5 (6H, m), 7.5-7.85 (4H, m).

Analysis: $C_{42}H_{61}N_5O_9S.HCl.2H_2O$ requires C,57.0; H,7.5; N,7.9%. Found: C,56.7; H,7.3; N,8.1%.

M.S. (m/z) (FAB) (M+1) = 812 (consistent with m.w. of free base = 811).

## Example 11

(6-(BOC-aminomethyl)-2,3-dihydro-1,1-dioxobenzothiophene-3-yl)acetyl-Phe-Leu-ACHPA-NH$(CH_2)_3CO_2Et$

This material was formed from (6-(BOC-aminomethyl)-2,3-dihydro-1,1-dioxobenzothiophene-3-yl)acetic

acid (0.16g) (Description 10(i)) and Phe-Leu-ACHPA-NH$(CN_2)_3$-$CO_2Et$ .HCl (0.25g) (Description 12(b)), following the procedure of Description 14(c). The crude product was chromatographed on silica gel using methanol/chloroform (0-5% methanol, gradient). This gave the title compound (0.23g) as an orange-brown gum.

NMR (δ) ($CD_3OD$): 0.7-1.05 (9H, m), 1.05-1.6 (16H, m), 1.6-1.9 (8H, m), 2.25 (2H, m), 2.35 (2H, m), 2.7-3.1 (m), 3.2 (2H, m), 3.6-3.9 (m), 3.95 (2H, m), 4.1 (2H, m), 4.3 (1H, m), 4.4-4.7 (m), 7.0-7.9 (m).

M.S. (m/z) (FAB) (M+1) = 926 (consistent with m.w. = 925).

## Example 12

(5-(BOC-aminomethyl-2,3-dihydrobenzofuran-3-yl acetyl-Phe-Leu-ACHPA 3-(1-imidazolyl)propylamide .0.5$H_2O$

This material was formed from (5-(BOC-aminomethyl)-2,3-dihydrobenzofuran-3-yl)acetic acid (0.096g) (Description 11(d)) and Phe-Leu-ACHPA 3-(1-imidazolyl)propylamide .2($CF_3CO_2H$) (0.21g) (Description 13-(b)), following the coupling procedure of Description 1(b). The crude product was chromatographed on silica gel using methanol/chloroform (0-10% methanol, gradient). This gave the title compound (0.15g) as a white powder.

NMR (δ) (DMSO$d_6$): 0.6-1.0 (9H, m), 1.0-1.85 (24H, m), 2.0-2.3 (3H, m), 2.55 (m), 2.65-2.9 (1H, m), 2.9-3.1 (3H, m), 3.6 (1H, m), 3.7-4.1 (6H, m), 4.2-4.4 (2H, m), 4.6 (1H, m), 4.8-4.9 (1H, m), 6.65 (1H, 2xd), 6.85 (1H, 5), 6.9-7.05 (2H, m), 7.1-7.4 (8H, m), 7.6 (1H, m), 7.75 (1H, m), 7.9-8.4 (2H, m).

Analysis: $C_{48}H_{69}N_7O_8$.0.5$N_2O$ requires C,65.4; H,8.0; N,11.1%. Found: C,65.4; H,8.0; N,10.9%.

M.S. (m/z) (FAB) (M+1) = 872 (consistent with m.w. = 871).

## Example 13

(5-(Aminomethyl)-2,3-dihydrobenzofuran-3-yl)acetyl-Phe-Leu-ACHPA 3-(1-imidazolyl)propylamide .2-($CF_3CO_2H$).$H_2O$

This material was formed from (5-(BOC-aminomethyl)-2,3-dihydrobenzofuran-3-yl) acetyl-Phe-Leu-ACHPA 3-(1-imidazolyl)propylamide (0.12g) (Example 12), following the procedure of Description 13(b). Trituration with ether, and filtration, gave the title compound (0.12g) as a white powder.

NMR (δ) (DMSO$d_6$): 0.6-1.0 (8H, m), 1.0-1.8 (14H, m), 1.9 (2H, m), 2.05-2.4 (3H, m), 2.6-2.85 (1H, m), 2.9-3.1 (3H, m), 3.6 (m), 3.9 (4H, m), 4.1-4.5 (5H, m), 4.6 (1H, m), 4.9 (1H, b), 6.8 (1H, 2xd), 7.1-7.4 (8H, m), 7.6 (1H, s), 7.7 (1H, s), 7.8 (1H, s), 7.9-8.15 (3H, m), 8.15-8.4 (2H, m), 9.0 (1H, s), 14.5 (vb).

Analysis: $C_{43}H_{61}N_7O_6$.2($C_2HO_2F_3$).$H_2O$ requires C,55.5; H,6.4; N,9.6%. Found: C,55.6; H,6.4; N,9.6%.

M.S. (m/z) (FAB) (M+1) = 772 (consistent with m.w. of free base = 771).

## Example 14

(2,3-Dihydro-1,1-dioxobenzothiophene-3-yl)acetyl-Phe-Leu-ACHPA 4-(1-imidazolyl)butylamide .HCl.2$H_2O$

This material was formed from BOC-ACHPA 4-(1-imidazolyl)butylamide (0.346g) (Description 15(c)), following the procedure of Example 6. The crude product was chromatographed on silica gel using methanol/chloroform (0-5% methanol, gradient). The product was dissolved in methanol (2 ml), and acidified with a slight excess of dilute hydrochloric acid. The solution was diluted with water (30 ml) and immediately freeze-dried. This gave the title compound (0.27g) as a white solid.

NMR (δ) ($CD_3OD$): 0.75-0.95 (4H, m), 0.95-1.1 (5H,m), 1.15-1.5 (6H, m), 1.5-2.05 (11H, m), 2.3-2.4 (2H, m), 2.4-2.75 (1H, m), 2.8-3.0 (2H, m), 3.0-3.15 (1H, m), 3.2-3.35 (3H, m), 3.85-4.1 (3H, m), 4.25-4.5 (3H, m), 4.5-4.65 (1H, m), 7.25-7.35 (5H, m), 7.35-7.8 (6H, m), 9.0-9.1 (1H, m).

Analysis: $C_{43}H_{60}N_6O_7S$.HCl.2$H_2$0 requires C,58.9; H,7.5; N,9.6%. Found: C,59.3; H,7.2; N,9.2%.

M.S. (m/z) (FAB) (M+1) = 805 (consistent with m.w. of free base = 804).

Example 15

(2,3-Dihydro-1,1-dioxobenzothiophene-3-yl)acetyl-Phe-Leu-ACHPA 2-(1-imidazolyl)ethylamide .HCl.$2H_2O$

This material was formed from BOC-ACHPA 2-(1-imidazolyl)ethylamide (0.18g) (Description 14(c)), following the procedure of Example 6. The crude product was chromatographed on silica gel using methanol/chloroform (0-20% methanol, gradient), and converted to the hydrochloride salt as described in Example 14. This gave the title compound (0.153g) as a white solid.

NMR (δ) ($CD_3OD$): 0.8-1.0 (3H, m), 1.0-1.2 (5H, m), 1.2-1.55 (6H, m), 1.55-2.0 (8H, m), 2.3-2.8 (3H, m), 2.85-3.05 (2H, m), 3.1-3.2 (1H, m), 3.55-3.85 (2H, m), 3.9-4.25 (2H, m), 4.3-4.55 (2H, m), 7.3-7.5 (5H, m), 7.5-7.85 (6H, m), 9.0-9.1 (1H, m).

Analysis: $C_{41}N_{56}N_6O_7S$.HCl.$2H_2O$ requires C,58.0; H,7.2; N,9.9%. Found: N,57.6; H,6.9; C,9.7%.

M.S. (m/z) (FAB) (M+1) = 777 (consistent with m.w. of free base = 776).

Example 16

(5-(Benzyloxycarbonyl-2,3-dihydrobenzofuran-3-yl)acetyl-Phe-Leu-ACHPA 3-(1-imidazolyl)propylamide

This material was formed from (5-(benzyloxycarbonyl)-2,3-dihydrobenzofuran-3-yl)acetic acid (0.15g) (Description 16(c)), following the procedure of Example 12. The crude product was chromatographed on silica gel using methanol/chloroform (0-10% methanol, gradient). This gave the title compound (0.25g).

NMR (δ) ($CD_3OD$): 0.75-1.15 (9H,m), 1.2-1.55 (5H,m), 1.6-2.15 (10H,m), 2.3-2.7 (3H,m), 2.7-3.4 (5H,m), 3.65-4.0 (1H,m), 4.05-4.25 (4H,m), 4.3-4.55 (2H,m), 4.55-4.85 (3H,m), 5.3-5.5 (2H,m), 6.9 (1H,m), 7.1 (1H,s), 7.2-7.6 (11H,m), 7.8 (1H,m), 7.9-8.1 (2H,m).

Example 17

(5-Carboxy-2,3-dihydrobenzofuran-3-yl acetyl-Phe-Leu-ACHPA 3-(1-imidazolyl)propylamide .HCl.$3H_2O$

This material was formed from (5-(benzyloxycarbonyl)-2,3-dihydrobenzofuran-3-yl)acetyl-Phe-Leu-ACHPA 3-(1-imidazolyl)propylamide (0.25g) (Example 16) following the procedure of Example 9. This gave the title compound (0.19g) as a white solid.

NMR (δ) ($CD_3OD$): 0.6-1.05 (9H,m), 1.1-1.45 (6H,m), 1.5-1.9 (9H,m), 2.05 (2H,m), 2.2-2.55 (3H,m), 2.6-2.75 (0.5H,m), 2.8-2.85 (0.5H,m), 2.9-3.05 (1H,m), 3.05-3.2 (2H,m), 3.6-3.7 (1H,m), 3.7-3.9 (1H,m), 4.0 (2H,m), 4.2-4.4 (4H,m), 4.45-4.7 (2H,m), 6.8 (1H,m), 7.1-7.4 (6H,m), 7.55 (1H,s), 7.7 (1H,m), 7.75-7.9 (2H,m), 8.95 (1H,m).

Analysis: $C_{43}H_{58}N_6O_8$.HCl.$3H_2O$ requires C,58.9; H,7.5; N,9.6%. Found: C,58.5; H,7.2; N,9.4%.

M.S. (m/z) (FAB) (M+1) = 787 (consistent with m.w. of free base = 786).

Example 18

(2,3-Dihydro-1,1-dioxobenzothiophene-3-yl)acetyl-Phe-Leu-ACHPA-NH($CH_2$)$CO_2$Me.0.5$H_2O$

This material was formed from BOC-ACHPA-NH$(CH_2)_4CO_2$Me (0.31g) (Description 17(b)), following the procedure of Example 6. The crude product was chromatographed on silica gel using ethyl acetate/petroleum ether (b.p. 60-80°C) (60-100% ethyl acetate, gradient). This gave the title compound (0.34g) as a pale yellow solid.

NMR (δ) ($CD_3OD$): 0.75-0.9 (4H,m), 0.9-1.05 (4H,m), 1.1-1.45 (6H,m), 1.45-1.9 (12H,m), 2.2-2.4 (4H,m), 2.4-2.65 (1H,m), 2.75-2.9 (2H,m), 2.9-3.1 (1H,m), 3.1-3.25 (3H,m), 3.35-3.4 (0.5H,m), 3.4-3.6 (1H,m), 3.65 (3H,m), 3.8-3.9 (0.5H,m), 3.9-4.0 (2.5H,m), 4.25 (0.5H,m), 4.4 (0.5H,m), 4.55 (0.5H,m), 7.2-7.35 (5H,m), 7.35-7.4 (0.25H,m), 7.45 (0.25H,d), 7.5-7.6 (1.75H,m), 7.6-7.7 (1.75H,m).

Analysis: $C_{42}H_{60}N_4O_9S.0.5H_2O$ requires C,62.6; H,7.6; N,6.9%. Found: C,62.4; H,7.5; N,6.8%.
M.S. (m/z) (FAB) (M+1) = 797 (consistent with m.w. = 796).

Example 19

(2,3-Dihydro-1,1-dioxobenzothiophene-3-yl)acetyl-Phe-Leu-ACHPA-NH$(CH_2)_4CO_2H$

This material was formed from (2,3-dihydro-1,1-dioxo benzothiophene-3-yl) acetyl-Phe-Leu-ACHPA-NH-$(CH_2)_4CO_2Me$. $0.5H_2O$ (0.16g) (Example 18), following the procedure of Example 8(a). The crude product was chromatographed on silica gel using methanol/chloroform (5-40% methanol, gradient). This gave the title compound (0.10g) as a white solid.

NMR (δ) ($CD_3OD$): 0.65-1.05 (8H,m), 1.05-1.45 (8H,m), 1.45-1.9 (10H,m), 2.15-2.4 (4H,m), 2.4-2.7 (1H,m), 2.8-3.1 (3H,m), 3.1-3.25 (3H,m), 3.4-3.75 (1H,m), 3.8-4.1 (3H,m), 4.25 (0.5H,m), 4.4 (0.5H,m), 4.5 (0.5H,m), 4.7-4.8 (0.5H,m), 7.2-7.35 (5H,m), 7.35-7.4 (0.25H,m), 7.4-7.45 (0.25H,m), 7.45-7.6 (1.75H,m), 7.6-7.7 (1.75H,m).

M.S. (m/z) (FAB) (M+1) = 783 (consistent with m.w. = 782).

Example 20

(2,3-Dihydro-1,1-dioxobenzothiophene-3-yl)acetyl-Phe-Leu-ACHPA 4-(4-methylpiperazin-1-yl)-butylamide.2HCl

This material was formed from BOC-ACHPA 4-(4-methylpiperazin-1-yl)butylamide (0.33g) (Description 18(c)), following the procedure of Example 6. The crude product was chromatographed on silica gel using methanol/chloroform (0-20% methanol, gradient), and converted to the hydrochloride salt as described in Example 14. This gave the title compound (0.17g) as a white solid.

NMR (δ) ($CD_3OD$): 0.7-0.9 (4H,m), 0.9-1.05 (4H,m), 1.1-1.45 (7H,m), 1.45-1.75 (8H,m), 1.75-1.9 (3H,m), 2.25-2.4 (2H,m), 2.45-2.7 (1H,m), 2.9 (2H,m), 2.95-3.15 (4H,m), 3.15-3.3 (4H,m), 3.4-3.45 (0.5H,m), 3.45-3.9 (8H,m), 3.9-4.1 (3H,m), 4.25 (0.5H,m), 4.35 (0.5H,m), 4.55 (0.5H,m), 4.7-4.8 (1H,m), 7.2-7.35 (5H,m), 7.35-7.7 (4H,m).

M.S. (m/z) (FAB) (M+1) = 837 (consistent with m.w. of free base = 836).

Example 21

(2,3-Dihydro-1,1-dioxobenzothiophene-3-yl)acetyl-Phe-Leu-ACHPA 3-(4-methylpiperazin-1-yl)-propylamide.2HCl

This material was formed from BOC-ACHPA 3-(4-methylpiperazin-1-yl)propylamide (0.28g) (Description 19(c)), following the procedure of Example 6. The crude product was chromatographed on silica gel using methanol/chloroform (0-30% methanol, gradient), and converted to the hydrochoride salt as described in Example 14. This gave the title compound (0.19g) as a white solid.

NMR (δ) ($CD_3OD$): 0.75-1.05 (8H,m), 1.1-1.45 (7H,m), 1.5-1.9 (9H,m), 1.95 (2H,m), 2.3 (2H,m), 2.45-2.7 (1H,m), 2.9 (2H,m), 3.0 (3H,m), 3.05-3.15 (1H,m), 3.35-3.45 (1H,m), 3.45-3.8 (7H,m), 3.9 (1H,m), 4.0 (2H,m), 4.25 (0.5H,m), 4.35 (0.5H,m), 4.55-4.65 (0.5H,m), 4.65-4.75 (1H,m), 7.2-7.35 (5H,m), 7.35-7.45 (1H,m), 7.45-7.7 (3H,m).

M.S. (m/z) (FAB) (M+1) = 823 (consistent with m.w. of free base = 822.

Example 22

(2,3-Dihydro-1,1-dioxobenzothiophene-3-yl)acetyl-Phe-Leu-ACHPA 2-(4-methylpiperazin-1-yl)ethylamide

.2.5$H_2O$

This material was formed from BOC-ACHPA 2-(4-methylpiperazin-1-yl)ethylamide (0.23g) (Description 20(c)), following the procedure of Example 6. The crude product was chromatographed on silica gel using methanol/chloroform (0-20% methanol, gradient). This gave the title compound (0.14g) as a white solid.

NMR (δ) ($CD_3OD$): 0.7-0.9 (4H,m), 0.9-1.05 (4H,m), 1.1-1.4 (6H,m), 1.45-1.9 (8H,m), 2.2-2.4 (5H,m), 2.4-2.7 (9H,m), 2.75-2.9 (2H,m), 2.95-3.1 (1H,m), 3.1-3.25 (1H,m), 3.4-3.65 (1H,m), 3.85 (0.5H,m), 3.95 (2H,m), 4.25 (0.5H,m), 4.4 (0.5H,m), 4.55 (0.5H,m), 4.7-4.8 (0.5H,m), 7.2-7.35 (5H,m), 7.35-7.75 (4H,m).

Analysis: $C_{43}H_{64}N_6O_7S.2.5H_2O$ requires C,60.5; H,8.1; N,9.8%. Found: C,60.1; H,7.6; N,9.6%.

M.S. (m/z) (FAB) (M+1) 809 (consistent with m.w. = 808).

## Example 23

(2,3-Dihydro-1,1-dioxobenzothiophene-3-yl)acetyl-Phe-Leu-ACHPA 3-(dimethylamino)propylamide .1.5$H_2O$

This material was formed from BOC-ACHPA 3-(dimethylamino)propylamide (0.27g) (Description 21), following the procedure of Example 6. The crude product was chromatographed on silica gel using methanol/chloroform (0-30% methanol, gradient). This gave the title compound (0.32g) as a pale yellow solid.

NMR (δ) (CD3OD): 0.7-0.9 (4H,m), 0.9-1.05 (4H,m), 1.1-1.4 (6H,m), 1.45-1.9 (10H,m), 2.2-2.35 (8H,m), 2.45 (2H,m), 2.5-2.65 (1H,m), 2.85 (2H,m), 3.0 (1H,m), 3.2 (3H,m), 3.35-3.65 (1H,m), 3.85 (0.5H,m), 3.95 (2H,m), 4.25 (0.5H,quintet), 4.4 (0.5H,m), 4.55 (0.5H, q), 4.7-4.8 (0.5H,m), 7.2-7.35 (5H,m), 7.35-7.7 (4H,m).

Analysis: $C_{41}H_{61}N_5O_7S.1.5H_2O$ requires C,61.9; H,8.1; N,8.8%. Found: C,61.8; H,7.8; N,8.8%.

M.S. (m/z) (FAB) (M+1) = 768 (consistent with m.w. = 767).

## Example 24

(2,3-Dihydro-3-oxo-1H-isoindole-1-acetyl)-Phe-Leu-ACHPA-NH$(CH_2)_3CO_2CH_2$Ph

This material was formed from (2,3-dihydro-3-oxo-1H-isoindole-1-acetyl)-Phe-Leu-OH (0.41g) (Description 22(e)) and BOC-ACHPA-NH$(CH_2)_3CO_2CH_2$Ph (0.52g) (Description 23(c)), following the procedure of Description 1(b). This gave the title compound (0.45g) as a yellow solid.

NMR (δ) ($DMSOd_6$): 0.6-0.8 (3H,m), 0.8-0.95 (4H,m), 0.95-1.4 (7H,m), 1.4-1.8 (8H,m), 2.0-2.2 (2H,m), 2.3-2.6 (4H,m), 2.65-3.1 (5H,m), 3.3 (1H,s), 3.85 (2H,m), 4.2-4.4 (1H,m), 4.5-4.9 (3H,m), 5.05 (2H,m), 7.1-7.3 (6H,m), 7.3-7.4 (5H,m), 7.4-7.55 (3H,m), 7.6 (1H,m), 7.7 (1H,m), 8.2-8.5 (3H,m). M.S. (m/z) (FAB) (M+1) 824 (consistent with m.w. = 823).

## Example 25a

(2,3-Dihydro-3-oxo-1H-isoindole-1-acetyl-Phe-Leu-ACHPA-NH$(CH_2)_3CO_2$H.

This material was formed from (2,3-dihydro-3-oxo-1H-isoindole-1-acetyl)-Phe-Leu-ACHPA-NH$(CH_2)_3CO_2CH_2$Ph (0.21g) (Example 24), following the procedure of Description 22(b). This gave the title compound (0.13g) as a pale yellow solid.

NMR (δ) ($CD_3OD$): 0.7-1.05 (8H,m), 1.05-1.45 (6H,m), 1.5-1.9 (10H,m), 2.3 (4H,m), 2.5 (1H,m), 2.6-2.8 (1H,m), 2.9 (1H,m), 3.0 (1H,m), 3.1-3.3 (2.5H,m), 3.95 (2H,m), 4.25 (0.5H,m), 4.4 (0.5H,m), 4.45-4.6 (0.5H,m), 4.7-4.75 (0.5H,m), 4.85-4.95 (1H,m), 4.95-5.05 (0.5H,m), 7.25 (6H,m), 7.4-7.7 (3H,m), 7.7-7.8 (1H,m).

M.S. (m/z) (FAB) (M+1) 734 (consistent with m.w. = 733).

## Example 25b

(2,3-Dihydro-3-oxo-1H-isoindole-1-acetyl)-Phe-Leu-ACHPA-NH$(CH_2)_3CO_2$Na

This material was formed from the corresponding acid (0.12g) (Example 25(a)), following the procedure of Example 8(b). This gave the title compound (0.1g) as a white solid.

NMR (δ) ($CD_3OD$): 0.7-1.05 (8H,m), 1.05-1.45 (6H,m), 1.45-1.9 (10H,m), 2.15-2.35 (4H,m), 2.4-2.7 (1H,m), 2.7-2.85 (1H,m), 2.9 (0.5H,m), 3.0 (1H,m), 3.2 (3H,m), 4.0 (2H,m), 4.3 (0.5H,m), 4.4 (0.5H,m), 4.5 (0.5H,m), 4.7-4.75 (0.5H,m), 4.85-4.95 (1H,m), 5.05 (0.5H,m), 7.2-7.35 (5H,m), 7.4-7.7 (3H,m), 7.7 (1H,m),.

M.S. (m/z) (FAB) (M+1) = 734 (consistent with m.w. of free acid 733).

Example 26a

(2,3-Dihydro-3-oxo-1H-isoindole-1-acetyl-Phe-Leu-ACHPA 3-(1-imidazolyl)propylamide

This material was formed from 2,3-dihydro-3-oxo-1H-isoindole-1-acetic acid (0.27g) (Description 22(c)), following the procedure of Example 12. The crude product was purified by chromatography on silica gel using methanol/chloroform (0-15% methanol, gradient). The title compound (0.19g) was isolated as a white solid.

NMR (δ) ($DMSOd_6$): 0.6-2.0 (24H,m), 2.0-2.2 (2H,m), 2.4-2.5 (2H,m), 2.7-2.9 (1H,m), 2.9-3.1 (3H,m), 3.8-4.0 (4H,m), 4.2-4.4 (1H,m), 4.6-4.9 (3H,m), 6.8-6.9 (1H,bs), 7.1-7.5 (10H,m), 7.5-7.6 (2H,m), 7.7-7.8 (1H,m), 8.2-8.5 (3H,m).

M.S. (m/z) (FAB) (M+1) 756 (consistent with m.w. = 755).

Example 26b

(2,3-Dihydro-3-oxo-1H-isoindole-1-acetyl-Phe-Leu-ACHPA 3-(1-imidazolyl)propylamide .HCl.$2H_2O$

This material was formed from the corresponding free base (Example 26(a)) (0.19g) following the procedure described in Example 14. The title compound (0.19g) was isolated as a white solid.

NMR (δ) ($DMSOd_6$): 0.6-2.0 (26H,m), 2.0-3.2 (7H,m), 3.8-5.0 (9H,m), 7.0-7.8 (14H,m), 8.2-8.6 (3H,m), 9.1 (1H,s), 14.5 (1H,bs).

Analysis: $C_{42}H_{57}N_7O_6$.HCl.$2H_2O$ requires C,60.9; H,7.5; N,11.8%. Found: C,61.1; H,7.3; N,11.6%.

Example 27

(2,3-Dihydro-1,1-dioxobenzothiophene-3-yl)acetyl-Phe-Leu-ACHPA-NH$(CH_2)_2CO_2CH_2$Ph

This material was formed from BOC-ACHPA-NH$(CH_2)_2CO_2CH_2$-Ph (0.38g) (Description 24(c)), following the procedure of Description 8(c). The crude product was chromatographed on silica gel using methanol/chloroform (0-2% methanol, gradient). This gave the title compound (0.30g) as a pale yellow solid.

NMR (δ) ($CD_3OD$): 0.85 (3H,m), 1.0 (5H,m), 1.1-1.5 (6H,m), 1.5-1.95 (8H,m), 2.3 (1H,m), 2.45-2.7 (2H,m), 2.75-2.95 (2H,m), 3.0-3.1 (1H,m), 3.15-3.3 (1H,m), 3.4-3.7 (3H,m), 3.85-4.1 (3H,m), 4.5-4.8 (2H,m), 5.15 (2H,m), 7.2-7.45 (10H,m), 7.45-7.75 (4H,m).

M.S. (m/z) (FAB) (M+1) = 845 (consistent with m.w. = 844).

Example 28

(2,3-Dihydro-1,1-dioxobenzothiophene-3-yl)acetyl-Phe-Leu-ACHPA-NH$(CH_2)_2CO_2H$ .1.5$H_2O$

(2,3-Dihydro-1,1-dioxobenzothiophene-3-yl)acetyl-Phe-Leu-ACHPA-NH$(CH_2)_2CO_2CH_2$Ph (0.14g) (Example 27) was hydrogenated over 10% palladium on charcoal (60% aqueous paste, 0.1g) in ethanol (30

ml) for 4h. The mixture was filtered, and the filtate evaporated. The product was dissolved in 5% sodium hydrogen carbonate solution, washed with ethyl acetate, acidified (5M hydrochloric acid), and extracted with chloroform. The extract was dried ($Na_2SO_4$) and evaporated to give the title compound (0.094g) as a white solid.

NMR (δ) ($CD_3OD$): 0.7-0.9 (3H,m), 0.9-1.05 (5H,m), 1.1-1.4 (6H,m), 1.45-1.6 (2H,m), 1.6-1.9 (6H,m), 2.25 (2H,m), 2.45-2.65 (3H,m), 2.85 (2H,m), 3.0 (1H,m), 3.1-3.25 (1H,m), 3.35-3.65 (3H,m), 3.85 (0.5H,m), 3.95 (2H,m), 4.25 (0.5H,quintet), 4.35 (0.5H,m), 4.55 (0.5H,t), 7.2-7.35 (5H,m), 7.35-7.7 (4H,m).

Analysis: $C_{39}H_{54}N_4O_9S.1.5H_2O$ requires C,59.9; H,7.3; N,7.2%. Found: C,59.6; H,7.3; N,7.0%.

M.S. (m/z) (FAB) (M+1) = 755 (consistent with m.w. = 754).

Example 29

(2,3-Dihydro-1,1-dioxobenzothiophene-3-yl)acetyl-Phe-Leu-ACHPA-NH($CH_2$)$_2$-(S)-CH($NH_2$)$CO_2H$ .HCl.1.5$H_2O$

This material was formed from (2,3-dihydro-1,1-dioxobenzothiophene-3-yl) acetyl-Phe-Leu-ACHPA-NH-($CH_2$)$_2$-(S)-CH(NH-CBZ)$CO_2CH_2$Ph (0.25g) (Description 25(d)), following the procedure of Example 9. This gave the title compound (0.15g) as a white solid.

NMR (δ) (DMSOd$_6$): 0.7-1.0 (7H,m), 1.0-2.0 (17H,m), 2.1 (1.5H,m), 2.4-2.5 (1H,m), 2.75 (2H,m), 2.9-3.1 (2H,m), 3.1-3.3 (3H,m), 3.8 (3H,m), 4.0 (0.5H,m), 4.35 (1H,m), 4.65 (1H,m), 4.8-5.1 (1H,m), 7.15-7.35 (4H,m), 7.35-7.4 (1H,m), 7.4-7.5 (1H,m), 7.5-7.65 (2H,m), 7.65-7.75 (1H,m), 8.05 (1H,m), 8.1-8.6 (3H,m).

Analysis: $C_{40}H_{57}N_5O_9S$.HCl.1.5$H_2O$ requires C,56.7; H,7.3; N,8.3%. Found: C,56.9; H,6.9; N,7.9%.

M.S. (m/z) (FAB) (M+1) 784 (consistent with m.w. of free base = 783).

Example 30

(2,3-Dihydro-1,1-dioxobenzothiophene-3-yl)acetyl-PheLeu-ACHPA 2-(2-pyridyliethylamide N-oxide .0.5$CHCl_3$ .0.5$H_2O$

(2,3-Dihydro-1,1-dioxobenzothiophene-3-yl)acetyl-Phe-Leu-ACHPA 2-(2-pyridyl)ethylamide .$H_2O$ (0.12g) (Example 4) was dissolved in dry dichloromethane (5 ml), and cooled in ice. 3-Chloroperoxybenzoic acid (0.032g) was added and the reaction stirred for 16h, warming to ambient temperature. The mixture was evaporated and the crude product chromatographed on silica gel using methanol/chloroform (0-5% methanol, gradient). This gave the title compond (0.11g) as a white solid.

NMR (δ) ($CD_3OD$): 0.7-1.05 (8H,m), 1.05-1.4 (6H,m), 1.4-1.9 (8H,m), 2.2-2.35 (2H,m), 2.45-2.65 (1H,m), 2.85 (2H,m), 3.0 (1H,m), 3.15 (2H,m), 3.25 (1H,m), 3.4-3.65 (3H,m), 3.8-4.05 (3H,m), 4.25 (0.5H,m), 4.35 (0.5H,m), 4.5 (0.5H,t), 4.7-4.8 (0.5H,m), 7.2-7.45 (8H,m), 7.45-7.55 (4H,m), 7.55-7.7 (2H,m), 7.85-7.95 (1H,m), 8.3 (1H,m).

Analysis: $C_{43}H_{57}N_5O_8S$.0.5$CHCl_3$.0.5$H_2O$ requires C,59.9; N,6.8; H,8.0%. Found: C,59.7; H,6.8; N,7.7%.

M.S. (m/z) (FAB) (M+1) = 804 (consistent with m.w. = 803).

Example 31

(6-Nitro-2,3-dihydro-1,1-dioxobenzothiophene-3-yl)acetyl-Phe-Leu-ACHPA 3-(1-imidazolyl)propylamide

This material was formed from (6-nitro-2,3-dihydro-1,1-dioxobenzothiophene-3-yl)acetic acid (0.16g) (Description 10(c)), following the procedure of Example 12. The crude product was purified by chromatography on silica gel using methanol/chloroform (0-10% methanol, gradient) to give the title compound (0.17g).

NMR (δ) ($CD_3OD$): 0.7-2.4 (29H,m), 2.6-3.0 (3H,m), 3.1-3.3 (2H,m), 3.9-4.1 (4H,m), 6.9 (1H,s), 7.1 (1H,m), 7.2-7.3 (5H,m), 7.5-7.7 (1H,m), 8.2-8.5 (2H,m).

Example 32a

(6-Amino-2,3-dihydro-1,1-dioxobenzothiophene-3-yl)acetyl-Phe-Leu-ACHPA 3-(1-imidazolyl)propylamide

This material was formed from (6-nitro-2,3-dihydro-1,1-dioxobenzothiophene-3-yl) acetyl-Phe-Leu-ACHPA 3-(1-imidazolyl)propylamide (0.15g) (Example 31), following the procedure of Description 22(b). The title compound (0.10g) was isolated as a slightly coloured solid.

NMR (δ) ($CD_3OD$): 0.7-1.9 (29H,m), 2.0 (2H,m), 2.4-2.5 (2H,m), 6.8-7.8 (11H,m).

M.S. (m/z) (FAB) (M+1) = 806 (consistent with m.w. = 805).

Example 32b

(6-Amino-2,3-dihydro-1,1-dioxobenzothiophene-3-yl)acetyl-Phe-Leu-ACHPA 3-(1-imidazolyl)propylamide .2HCl

This material was formed from the corresponding free base (0.15g) (Example 32(a)), following the procedure of Example 14. The title compound (0.15g) was isolated as a slightly coloured solid.

NMR (δ) ($CD_3OD$): 0.7-1.9 (29H,m), 2.0-2.2 (2H,m), 2.3-2.4 (2H,m), 3.9-4.1 (2H,m), 4.2-4.4 (2H,m), 7.2-7.4 (6H,m), 7.5-7.6 (3H,m), 7.6-7.7 (1H,m), 9.0 (1H,s)

Analysis: $C_{42}H_{59}N_7O_7S.2HCl$ requires C,57.4; H,7.0; N,11.2%. Found: C,57.3; H,6,6; N,11.4%.

Example 33

4-(6-Nitro-2,3-dihydro-1,1-dioxobenzothiophene-2-yl)butanoyl-Phe-Leu-ACHPA 3-(1-imidazolyl)propylamide

This material was formed from (6-nitro-2,3-dihydro-1,1-dioxobenzothiophene-2-yl)butanoic acid (0.14g) (Description 28(c)), and Phe-Leu-ACHPA 3-(1-imidazolyl)propylamide (0.25g) (Description 26) following the procedure of Description 1(a). The crude product was purified by chromatography on silica gel using methanol/chloroform (0-10% methnol, gradient) to give the title compound (0.14g).

NMR (δ) ($CDCl_3$): 0.7-2.1 (29H,m), 2.3-2.5 (4H,m), 2.9-3.6 (10H,m), 3.9-4.7 (7H,m), 6.7 (1H,m), 7.0 (2H,m), 7.1-7.4 (7H,m), 7.5-7.6 (2H,m), 7.6-7.7 (1H,d), 8.4 (1H,m), 8.5-8.6 (1H,m).

Example 34a

4-(6-Amino-2,3-dihydro-1,1-dioxobenzothiophene-2-yl)butanoyl-Phe-Leu-ACHPA 3-(1-imidazolyl)propylamide

This material was formed from (6-nitro-2,3-dihydro-1,1-dioxobenzothiophene-2-yl)butanoyl-Phe-Leu-ACHPA 3-(1-imidazolyl)propylamide (0.14g) (Example 33), following the procedure of Description 22(b). This gave the title compound (0.083g) as a white solid.

NMR (δ) ($DMSOd_6$): 0.6-1.8 (29H,m), 1.9-2.0 (2H,m), 2.0-2.2 (4H,m), 2.5-3.6 (6H,m), 3.8 (2H,bs), 4.1 (2H,t), 4.3 (1H,m), 4.5-4.6 (1H,m), 4.9 (1H,d), 6.6-6.9 (2H,m), 7.1-7.4 (9H,m), 7.5 (1H,s), 7.8 (1H,m), 8.1-8.3 (3H,m).

M.S. (m/z) (FAB) (M+1) = 834 (consistent with m.w. = 833).

Example 34b

4-(6-Amino-2,3-dihydro-1,1-dioxobenzothiophene-2-yl)butanoyl-Phe-Leu-ACHPA 3-(1-imidazolyl)propylamide .2HCl.4$H_2O$

This material was formed from the corresponding free base (0.083g) (Example 34(a)), following the procedure of Example 14. The title compound (0.079g) was isolated as a white solid.

NMR (δ) (DMSOd$_6$): 0.7-2.2 (32H,m), 2.6-2.8 (2H,m), 3.0-3.2 (5H,m), 4.2 (2H,m), 4.3 (1H,m), 4.5 (1Hm), 6.8 (1H,s), 6.9-7.1 (2H,m), 7.1-7.3 (7H,m), 7.4 (1H,m), 7.7 (1H,s), 7.8 (1H,s), 7.9 (1H,m), 8.1-8.2 (2H,m), 9.1 (1H,s), 14.0-14.8 (b).

Analysis: $C_{44}H_{63}N_7O_7S.2HCl.4H_2O$ reguires C,54.0; N,10.0%. Found C,53.9; N,9.7%.

Example 35

(6-(BOC-aminomethyl)-2,3-dihydro-1,1-dioxobenzothiophene-3-yl)acetyl-Phe-Leu-ACHPA-NH$(CH_2)_3CO_2CH_2Ph$

This material was formed from (6-(BOC-aminomethyl)-2,3-dihydro-1,1-dioxobenzothiophene-3-yl)acetic acid (0.11g) (Description 10(i)) and BOC-Phe-Leu-ACHPA-NH$(CH_2)_3CO_2CH_2Ph$ (0.23g) (Description 23(e)), following the procedure of Description 1(b). The crude product was chromatographed on silica gel using methanol/chloroform (0-5% methanol, gradient). This gave the title compound (0.14g) as a yellow solid.

NMR (δ) ($CDCl_3$): 0.7-2.0 (33H,m), 2.2-2.7 (m), 2.9-3.4 (m), 3.95 (2H,m), 4.3 (2H,m), 5.1 (2H,s), 6.7-7.7 (17H,m).

M.S. (m/z) (FAB) (M + 1) 988 (consistent with m.w. = 987.

Example 36

(6-(BOC-aminomethyl)-2,3-dihydro-1,1-dioxobenzothiophene-3-yl)acetyl-Phe-Leu-ACHPA-NH$(CH_2)_3CO_2H$

This material was formed from (6-(BOC-aminomethyl)-2,3-dihydro-1,1-dioxobenzothiophene-3-yl)acetyl-Phe-Leu-ACHPA-NH$(CH_2)_3CO_2CH_2Ph$ (0.14g) (Example 35), following the procedure of Description 22(b). This gave the title compound (0.12g) as a yellow powder.

NMR (δ) ($CD_3OD$): 0.65-1.1 (8H,m), 1.2-1.95 (25H,m), 2.2-2.4 (4H,m), 2.7-2.95 (2H,m), 3.15-3.3 (3H,m), 4.0 (2H,m), 4.2-4.4 (2H,m), 7.1-7.7 (12H,m).

M.S. (m/z) (FAB) (M + 1) = 898 (consistent with m.w. = 897).

Example 37

(6-(Aminomethyl)-2,3-dihydro-1,1-dioxobenzothiophene-3-yl)acetyl-Phe-Leu-ACHPA-NH$(CH_2)_3CO_2H$ $.CF_3CO_2H$

This material was formed from (6-(BOC-aminomethyl)-2,3-dihydro-1,1-dioxobenzothiophene-3-yl)acetyl-Phe-Leu-ACHPA-NH$(CH_2)_3CO_2H$ (0.12g) (Example 36), following the procedure of Description 13(b). This gave the title compound (0.10g) as a buff powder.

NMR (δ) ($CD_3OD$): 0.6-1.05 (9H,m), 1.05-1.9 (15H,m), 2.2-2.4 (3H,m), 2.4-2.6 (1H,m), 2.8-3.3 (5H,m), 3.8-4.0 (2H,m), 4.2 (2H,s), 4.4 (1H,m), 7.1-7.4 (7H,m), 7.4-8.4 (5H,m).

Analysis: $C_{41}H_{59}N_5O_9S.C_2HO_2F_3$ requires C,56.6; H,6.6; N,7.7%. Found: C,56.7; H,6.7; N,7.7%.

M.S. (m/z) (FAB) (M + 1) 798 (consistent with m.w. of free base = 797).

Example 38

(5-(BOC-aminomethyl)-2,3-dihydrobenzofuran-3-yl)acetyl-Phe-Leu-ACHPA-NH$(CH_2)_3CO_2CH_2Ph$

This material was formed from (5-(BOC-aminomethyl)-2,3-dihydrobenzofuran-3-yl)acetic acid (0.10g) (Description 11(d)), following the procedure of Example 35. The crude product was chromatographed on

silica gel using methanol/chloroform (0-5% methanol, gradient). This gave the title compound (0.13g) as a yellow solid.

M.S. (m/z) (FAB) (M+1) .940 (consistent with m.w. = 939.

Example 39

(5-BOC-aminomethyl-2,3-dihydrobenzofuran-3-yl)acetyl-Phe-Leu-ACHPA-NH$(CH_2)_3CO_2H$

This material was formed from (5-(BOC-aminomethyl)-2,3- dihydrobenzofuran-3-yl)acetyl-Phe-Leu-ACHPA-NH$(CH_2)_3$-$CO_2CH_2$Ph (0.13g) (Example 38), following the procedure of Description 22(b). This gave the title compound (0.12g) as a light grey solid.

M.S. (m/z) (FAB) (M+1) = 850, (M+23) = 872 (consistent with m.w. 849).

Example 40

(5-(Aminomethyl)-2,3-dihydrobenzofuran-3-yl)acetyl-Phe-Leu-ACHPA-NH$(CH_2)_3CO_2H$ .$CF_3CO_2H$.0.5$H_2O$

This material was formed from (5-(BOC-aminomethyl)-2,3-dihydrobenzofuran-3-yl) acetyl-Phe-Leu-ACHPA-NH$(CH_2)_3CO_2H$ (0.11g) (Example 39), following the procedure of Description 13(b). This gave the title compound (0.10g) as an off-white powder.

NMR (δ) ($DMSOd_6$): 0.6-1.0 (8H,m), 1.0-1.8 (16H,m), 2.05 (1H,m), 2.15-2.35 (3H,m), 2.7 (1H,m), 2.8-3.1 (3H,m), 3.6 (1H,m), 3.75-3.95 (3H,m), 4.1 (0.5H,dd), 4.3 (1H,m), 4.4 (0.5H,t), 4.6 (1H,m), 4.9 (1H,m), 6.8 (1H,2xd), 7.1-7.4 (9H,m), 7.7 (1H,m), 7.8-8.3 (4H,m).

Analysis: $C_{41}H_{59}N_5O_8$.($C_2HO_2F_3$).0.5($H_2O$) requires C,59.2; H,7.1; N,8.0%. Found: C,58.8; H,7.0; N,7.6%.

M.S. (m/z) (FAB) (M+1) = 750 (consistent with m.w. of free base = 749).

Example 41

(2,3-Dihydro-2-oxo-1H-benzimidazole-1-propanoyl)-Phe-Leu-ACHPA 3-(1-imidazolyl)propylamide .0.5$CHCl_3$

This material was formed from 2,3-dihydro-2-oxo-1H-benzimidazole-1-propanoic acid (0.036g) (Description 29(b)), following the procedure of Example 33. The crude product was chromatographed on silica gel using methanol/chloroform (0-15% methanol, gradient). This gave the title compound (0.071g) as a white solid.

NMR (δ) ($DMSOd_6$): 0.7-1.0 (8H,m), 1.0-1.7 (13H,m), 1.7-1.9 (3H,m), 2.1 (2H,m), 2.4 (2H,t), 2.7 (1H,m), 2.8-3.1 (3H,m), 3.85 (4H,m), 3.95 (2H,t), 4.3 (1H,m), 4.55 (1H,m), 4.9 (1H,m), 6.85 (1H,m), 6.95 (3H,m), 7.05 (1H,m), 7.1-7.5 (7H,m), 7.6 (1H,m), 7.75 (1H,m), 8.2 (1H,m), 8.25-8.4 (1H,m), 10.8 (1H,s).

Analysis: $C_{42}H_{58}N_8O_6$.0.5($CHCl_3$) requires C,61.4; H,7.1; N,13.4%. Found: C,61.4; H,7.2; N,13.3%.

M.S. (m/z) (FAB) (M+1) = 771 (consistent with m.w. = 770).

Example 42

(5-(BOC-aminomethyli-2,3-dihydro-1,1-dioxobenzothiophene-3-yl)acetyl-Phe-Leu-ACHPA 3-(1-imidazolyl)-propylamide .2$H_2O$

This material was formed from (5-(BOC-aminomethyl)-2,3-dihydro-1,1-dioxobenzothiophene-3-yl)acetic acid (0.11g) (Description 30(g)), following the procedure of Example 33. The crude-product was chromatographed on silica gel using methanol/chloroform (0-10% methanol, gradient). This gave the title compound (0.15g) as a white powder.

NMR (δ) ($DMSOd_6$): 0.7-1.9 (31H,m), 2.05-2.2 (2H,m), 2.35 (1H,m), 2.7 (2H,m), 2.9-3.1 (3H,m), 3.2 (1H,m), 3.35-3.5 (1H,m), 3.7-4.0 (4H,m), 4.2 (2H,bd), 4.3 (1H,m), 4.65 (1H,m), 4.8-4.9 (1H,m), 6.85 (1H,s), 7.1-7.8 (11H,m), 8.2-8.5 (2H,m).

Analysis: $C_{48}H_{69}N_7O_9S.2H_2O$ requires C,60.3; H,7.7; N,10.3%. Found: C,60.2; H,8.1; N,10.1%.

M.S. (m/z) (FAB) (M+1) 919 (consistent with m.w. = 918).

Example 43

(5-(Aminomethyl)-2,3-dihydro-1,1-dioxobenzothiophene-3-yl)acetyl-Phe-Leu-ACHPA 3-(1-imidazolyl)-propylamide $.3CF_3CO_2H.H_2O$

This material was formed from (5-(BOC-aminomethyl)-2,3-dihydro-1,1-dioxobenzothiophene-3-yl)acetyl-Phe-Leu-ACHPA 3-(1-imidazolyl)propylamide $.2H_2O$ (0.13g) (Example 42), following the procedure of Example 13. This gave the title compound (0.12g) as a white powder.

NMR (δ) ($DMSOd_6$): 0.6-1.8 (22H,m), 1.9 (2H,m), 2.1 (2H,m), 2.4 (1H,m), 2.75 (2H,m), 2.9-3.5 (7H,m), 4.1-4.3 (4H,m), 4.35 (1H,m), 4.6 (1H,m), 7.1-7.4 (6H,m), 7.5-7.9 (6H,m), 8.3 (5H,m), 9.1 (1H,s).

Analysis: $C_{43}H_{61}N_7O_7S$ $.3(C_2HO_2F_3).H_2O$ requires C,49.9; H,5.6; N,8.3%. Found: C,49.9; H,5.5; N,8.4%.

M.S. (m/z) (FAB) (M+1) = 820 (consistent with m.w. of free base = 819).

Example 44

(5- (Carboxymethylaminomethyl)-2,3-dihydrobenzofuran-3-yl)acetyl-Phe-Leu-ACHPA 3-(1-imidazolyl)-propylamide $.3(CF_3CO_2H)$

This material was formed from (5-(N-BOC-N-(carboxymethyl)aminomethyl)-2,3-dihydrobenzofuran-3-yl) acetyl-Phe-Leu-ACHPA 3-(1-imidazolyl)propylamide (0.095g) (Description 31(g)), following the procedure of Description 23(d). The product was dissolved in the minimum quantity of methanol, diluted with distilled water and freeze-dried to afford the title compound (0.1g).

NMR (δ) ($DMSOd_6$): 0.7-1.8 (22H,m), 1.9 (2H,m), 2.05-2.3 (2H,m), 2.55 (1H,m), 2.7 (1H,m), 3.05 (4H,m), 3.5-4.7 (12H,m), 6.8 (1H,m), 7.1-7.4 (9H,m), 7.65 (1H,s), 7.7 (1H,m), 7.85 (1H,m), 8.25 (3H,m), 9.0 (1H,s).

Analysis: $C_{45}H_{63}N_7O_8.3(C_2HO_2F_3)$ requires C,52.3; H,5.8; N,8.4%. Found: C,52.3; H,5.9; N,8.1%.

M.S. (m/z) (FAB) (M+1) = 830 (consistent with m.w. of free base = 829).

Example 45

(5-BOC-aminomethyl)-2,3-dihydro-1,1-dioxobenzothiophene-3-yl)acetyl-Phe-His-ACHPA-$NH(CH_2)_3CO_2CH_2Ph$

This material was formed from (5-(BOC-aminomethyl)-2,3- dihydro-1,1-dioxobenzothiophene-3-yl) acetyl-Phe-His-OH (0.24g) (Description 32(b)), following the procedure of Description 23(e). The crude product was chromatographed on silica gel using methanol/chloroform (0-10% methanol, gradient). This gave the title compound (0.23g) as a light orange powder.

NMR (δ) ($DMSOd_6$): 0.65-0.95 (2H,m), 1.0-1.5 (15H,m), 1.5-1.8 (7H,m), 1.9-2.2 (2H,m), 2.4 (3H,m), 2.7 (3H,m), 2.9 (2H,m), 3.05 (3H,m), 3.2 (1H,m), 3.65-3.9 (3H,m), 4.2 (2H,d), 4.45-4.7 (2H,m), 4.9 (0.5H,m), 5.0 (0.5H,m), 5.05 (2H, 2xs), 6.7-6.9 (1H,m), 7.1-7.4 (13H,m), 7.4-7.55 (2H,m), 7.7 (1H,d), 7.75 (0.5H,m), 7.85 (0.5H,m), 8.4 (2H,m), 11.6-11.9 (1H,m).

M.S. (m/z) (FAB)(M+1) 1012 (consistent with m.w. = 1011).

Example 46

(5-BOC-aminomethyl)-2,3-dihydro-1,1-dioxobenzothiophene-3-yl)acetyl-Phe-His-ACHPA-NH$(CH_2)_3CO_2H$

This material was formed from (5-(BOC-aminomethyl)-2,3-dihydro-1,1-dioxobenzothiophene-3-yl)acetyl-Phe-His-ACHPA-NH$(CH_2)_3CO_2CH_2Ph$ (0.22g) (Example 45), following the procedure of Description 22(b). This gave the title compound (0.19g) as a light yellow solid.

NMR (δ) ($DMSOd_6$): 0.7-1.0 (2H,m), 1.0-1.5 (15H,m), 1.5-1.9 (7H,m), 2.1-2.35 (4H,m), 2.8 (2H,m), 2.9-3.5 (9H,m), 4.25 (3H,m), 4.65 (3H,m), 6.9-7.0 (1H,m), 7.1-7.45 (10H,m), 7.6 (1H,d), 7.8 (1H,m), 8.2 (1H,m), 8.4 (1H,m), 8.55 (1H,m).

M.S. (m/z) (FAB) (M+1) = 922, (M+23) = 944 (consistent with m.w. 921).

## Example 47

(5-Aminomethyl)-2,3-dihydro-1,1-dioxobenzothiophene-3-yl)acetyl-Phe-His-ACHPA-NH$(CH_2)_3CO_2H$.3-$(CF_3CO_2H)$.$2H_2O$

This material was formed from (5-(BOC-aminomethyl)-2,3-dihydro-1,1-dioxobenzothiophene-3-yl) acetyl-Phe-His-ACHPA-NH$(CH_2)_3CO_2H$ (0.18g) (Example 46), following the procedure of Description 23(d). The residue was dissolved in water and freeze-dried, giving the title compound (0.21g) as a fluffy white powder.

NMR (δ) ($DMSOd_6$): 0.7-1.45 (8H,m), 1.45-1.8 (7H,m), 2.0-2.15 (2H,m), 2.2 (2H,m), 2.45 (1H,m), 2.6-2.8 (2H,m), 2.8-3.2 (6H,m), 3.25 (1H,m), 4.15 (b), 4.55-4.8 (2H,m), 5.0 (1H,b), 7.2-7.35 (5H,m), 7.35-7.5 (1H,m), 7.6 (3H,m), 7.8 (2H,m), 8.3 (3H,b), 8.5 (1H,m), 8.7 (1H,m), 9.0 (1H,m), 12.1 (1H,vb), 14.25 (2H,b).

Analysis: $C_{41}H_{55}N_7O_9S$.3$(C_2HO_2F_3)$.$2H_2O$ requires C,47.0; H,5.2; N,8.2%. Found: C,46.9; H,4.9; N,8.2%.

M.S. (m/z) (FAB) (M+1) = 822 (consistent with m.w. of free base = 821).

## Example 48

(5-BOC-aminomethyl)-2,3-dihydro-1,1-dioxobenzothiophene-3-yl)acetyl-Phe-His-ACHPA 3-(1-imidazolyl)-propylamide

This material was formed from (5-(BOC-aminomethyl)-2,3-dihydro-1,1-dioxobenzothiophene-3-yl)acetyl-Phe-His-OH.HCl (0.17g) (Description 32(b)), following the procedure of Description 13(a). The crude product was chromatographed on silica gel using methanol/chloroform (0-20% methanol, gradient). This gave the title compound (0.12g) as a light yellow solid.

NMR (δ) ($DMSOd_6$): 0.65-1.5 (16H,m), 1.5-2.0 (8H,m), 2.1 (2H,m), 2.4 (1H,m), 2.7 (2H,m), 2.8-3.1 (5H,m), 3.2 (1H,m), 3.65-3.9 (3H, 2xb), 4.0 (2H,m), 4.2 (2H,d), 4.4-4.7 (2H,m), 4.9-5.1 (1H,m), 6.7-7.0 (1H,m), 7.1-7.4 (9H,m), 7.5-8.0 (5H,m), 8.45 (2H,m), 11.7-11.9 (1H,m).

M.S. (m/z) (FAB) (M+1) = 944 (consistent with m.w. = 943).

## Example 49

(5-(Aminomethyl)-2,3-dihydro-1,1-dioxobenzothiophene-3-yl)acetyl-Phe-His-ACHPA 3-(1-imidazolyl)-propylamide .3$(CF_3CO_2H)$.$CH_2Cl_2$

This material was formed from (5-BOC-aminomethyl)-2,3-dihydro-1,1-dioxobenzothiophene-3-yl) acetyl-Phe-His-ACHPA 3-(1-imidazolyl)propylamide (0.12g) (Example 48), following the procedure of Example 47. This gave the title compound (0.14g) as a fluffy white powder.

NMR (δ) ($DMSOd_6$): 0.7-1.45 (8H,m), 1.45-1.85 (5H,m), 1.9 (2H,m), 2.0-2.2 (2H,m), 2.4 (1H,m), 2.55-2.8 (2H,m), 2.8-3.9 (m), 4.1 (2H,m), 4.2 (2H,m), 4.5-4.8 (2H,m), 5.05 (1H,b), 7.15-7.3 (5H,m), 7.4 (1H,m), 7.55-7.7 (4H,m), 7.75 (1H,m), 7.8-8.0 (2H, 2xm), 8.2-8.5 (4H, m+b), 8.7 (1H,m), 8.95-9.1 (2H,m).

Analysis: $C_{43}H_{57}N_9O_7S$.3$(C_2HO_2F_3)$.$CH_2Cl_2$ requires C,47.3; H,4.9; N,9.9%. Found: C,47.7; H,4.8; N,10.2%.

M.S. (m/z) (FAB) (M+1) = 844 (consistent with m.w. of free base = 843).

Example 50a

4-(2,3-Dihydro-1,1-dioxobenzothiophene-2-yl)butanoyl-Phe-Leu-ACHPA 3-(1-imidazolyl)propylamide

This material was formed from 4-(2,3-dihydro-1,1-dioxobenzothiophene-2-yl)butanoic acid (0.20g) (Description 28(b)), following the procedure of Example 12. The crude product was purified by chromatography on silica gel using methanol/chloroform (0-5% methanol, gradient) to give the title compound (0.25g) as a white powder.

NMR (δ) ($DMSOd_6$): 0.6-1.0 (9H,m), 1.0-1.9 (21H,m), 2.0-2.4 (5H,m), 2.7-3.1 (6H,m), 3.4-3.6 (2H,m), 3.8-4.0 (4H,m), 4.2-4.4 (1H,m), 4.5-4.7 (1H,m), 4.8-5.0 (1H,m), 6.9 (1H,s), 7.1-7.4 (7H,m), 7.5-7.9 (6H,m), 8.1-8.3 (2H,m).

M.S. (m/z) (FAB) (M+1) 819 (consistent with m.w. = 818).

Example 50b

4-(2,3-Dihydro-1,1-dioxobenzothionhene-2-yl)butanoyl-Phe-Leu-ACHPA 3-(1-imidazolyl)propylamide .HCl.1.5$H_2O$

This material was formed from the corresponding free base (Example 50(a)) (0.23g), following the procedure of Example 1(b). The title compound (0.21g) was isolated as a white powder.

NMR (δ) ($DMSOd_6$): 0.7-1.8 (30H,m), 1.9-2.4 (6H,m), 2.7-3.2 (6H,m), 3.3-3.5 (2H,m), 3.7 (2H,m), 4.1-4.4 (3H,m), 4.5 (1H,m), 4.9 (1H,bs), 7.1-7.4 (6H,m), 7.5-7.9 (7H,m), 8.1-8.3 (2H,m), 9.1 (1H,s), 14.4 (1H,bs).

Analysis: $C_{44}H_{62}N_6O_7S$.HCl.1.5$H_2O$ requires C,59.9; H,7.5; N,9.5%. Found: C,59.7; H,7.3; N,9.4%.

Example 51

(6-BOC-aminomethyl)-2,3-dihydro-1,1-dioxobenzothiophene-3-yl)acetyl-Phe-Leu-ACHPA 3-(1-imidazolyl)-propylamide.2$H_2O$

This material was formed from (6-(BOC-aminomethyl)-2,3-dihydro-1,1-dioxobenzothiophene-3-yl)acetic acid (0.22g) (Description 10(i)), following the procedure of Example 33. The crude product was purified by chromatography on silica gel using methanol/chloroform (0-10% methanol, gradient), giving the title compound (0.19g) as a white solid.

NMR (δ) ($DMSOd_6$): 0.6-1.9 (33H,m), 2.05-2.2 (2H,m), 2.4 (1H,m), 2.7 (2H,m), 2.9-3.1 (4H,m), 35 (m), 3.7-4.0 (5H,m), 4.2 (2H,m), 4.35 (1H,m), 4.65 (1H,m), 4.8-4.9 (1H,m), 6.85 (1H,s), 7.1-7.8 (13H,m), 8.2-8.45 (m).

Analysis: $C_{48}H_{69}N_7O_9S$.2$H_2O$ requires C,60.3; H,7.7; N,10.2%. Found: C,60.1; H,7.3; N,10.1%.

M.S. (m/z) (FAB) (M+1) 920 (consistent with m.w. = 919).

Example 52

(6-(Aminomethyl)-2,3-dihydro-1,1-dioxobenzothiophene-3-yl)acetyl-Phe-Leu-ACHPA 3-(1-imidazolyl)-propylamide .3($CF_3CO_2H$)

This material was formed from (6-(BOC-aminomethyl)-2,3-dihydro-1,1-dioxobenzothiophene-3-yl)acetyl-Phe-Leu-ACHPA 3-(1-imidazolyl)propylamide (0.19g) (Example 51), following the procedure of Description 23(d). Trituration with ether then gave the title compound (0.21g) as a white solid.

NMR (δ) ($DMSOd_6$): 0.7-1.0 (8H,m), 1.0-1.45 (6H,m), 1.45-1.7 (7H,m), 1.75 (1H,d), 1.9 (2H,m), 2.1

(2H,m), 2.4 (1H,m), 2.75 (2H,m), 2.9-3.15 (3H,m), 3.25 (1H,m), 3.5 (m), 3.75-3.9 (m), 4.15 (4H,m), 4.35 (1H,m), 4.65 (1H,m), 4.9 (1H,b), 7.15-7.3 (5H,m), 7.35 (1H,t), 7.45 (1H,dd), 7.65 (2H,m), 7.75 (1H,s), 7.8 (2H,m), 8.2-8.4 (5H,m), 9.05 (1H,s), 14.45 (1H,b).

Analysis: $C_{43}H_{61}N_7O_7S.3(C_2HO_2F_3)$ requires C,50.6; H,5.5; N,8.4%. Found: C,50.4; H,5.5; N,8.7%.

M.S. (m/z) (FAB) (M+1) = 820 (consistent with m.w. of free base = 819).

Example 53

4-(1,3-Dihydro-1-oxo-2H-isoindol-2-yl)butanoyl-Phe-Leu-ACHPA 3-(1-imidazolyl)propylamide

This material was formed from 4-(1,3-dihydro-1-oxo-2H-isoindol-2-yl)butanoic acid (0.13g) (Description 33(c)), following the procedure of Example 33. The crude product was chromatographed on silica gel using methanol/chloroform (5-10% methanol, gradient). This gave the title compound (0.29g) as a white foam.

NMR (δ) ($DMSOd_6$): 0.6-0.95 (8H,m), 0.95-1.4 (7H,m), 1.4-1.9 (11H,m), 1.95-2.15 (4H,m), 2.55-3.5 (7H,m), 3.75-4.0 (4H,m), 4.0-4.6 (4H,m), 4.85 (1H,m), 6.85 (1H,s), 7.1-7.35 (7H,m), 7.35-7.7 (5H,m), 7.8 (1H,t), 8.1-8.35 (1H,m).

Example 54

(2,3-Dihydro-1,1-dioxobenzothiophene-3-yl)acetyl-Phe-Leu-ACHPA-NH$(CH_2)_3$-Pro methyl ester.HCl.$2H_2O$.0.5$CHCl_3$

This material was formed from BOC-ACHPA-NH$(CH_2)_3$-Pro methyl ester (0.25g) (Description 34(c)) following the procedure of Example 6. The crude product was chromatographed on silica gel using methanol/chloroform (0-10% methanol, gradient), and converted to the hydrochloride salt as described in Example 1(b). This gave the title compound (0.24g) as a white solid.

NMR (δ) ($CD_3OD$): 0.85 (4H,m), 0.95 (5H,m), 1.1-1.4 (7H,m), 1.5-1.8 (8H,m), 1.8-2.1 (3H,m), 2.2 (2H,m), 2.35 (2H,m), 2.45-2.65 (2H,m), 2.85 (2H,m), 3.05 (1H,m), 3.1-3.3 (3.5H,m), 3.4 (2H,m), 3.3-3.65 (0.5H,m), 3.8 (1H,m), 3.85 (3H, 2xs), 4.0 (2H,m), 4.25 (0.5H,m), 4.35 (0.5H,m), 4.45-4.5 (1H,m), 4.55 (0.5H,m), 4.75 (0.5H,m), 7.2-7.35 (5H,m), 7.35-7.7 (4H,m).

Analysis: $C_{45}H_{65}N_5O_9S.HCl.2H_2O.0.5(CHCl_3)$ requires C,55.5; H,7.2; N,7.1%. Found: C,55.7; H,6.9; N,7.4%.

M.S. (m/z) (FAB) (M+1) = 852 (consistent with m.w. of free base = 851).

Example 55a

(2,3-Dihydro-1,1-dioxobenzothiophene-3-yl)acetyl-Phe-Leu-ACHPA 2-(3-pyridyl)ethylamide

This material was formed from BOC-ACHPA 2-(3-pyridyl)ethylamide (0.32g) (Description 35(b)), following the procedure of Example 6. The crude product was chromatographed on silica gel using methanol/chloroform (0.5% methanol, gradient). This gave the title compound (0.36g) as a white solid.

NMR (δ) ($CD_3OD$): 0.8 (2H,m), 1.0 (5H,m), 1.1-1.4 (6H,m), 1.4-1.9 (9H,m), 2.2 (2H,m), 2.4-2.65 (1.5H,m), 2.85 (4H,m), 3.0 (1H,m), 3.1-3.3 (1H,m), 3.45 (3H,m), 3.95 (3H,m), 4.2-4.3 (0.5H,m), 4.35 (1H,m), 4.45-4.55 (0.5H,m), 4.7-4.85 (0.5H,m), 7.2-7.35 (6H,m), 7.4-7.8 (5H,m), 8.3-8.45 (2H,m).

Example 55b

2,3-Dihydro-1,1-dioxobenzothiophene-3-yl)acetyl-Phe-Leu-ACHPA 2-(3-pyridyl)ethylamide.HCl

This material was formed from the corresponding free base (Example 55(a)) (0.17g), following the

procedure of Example 1(b). This gave the title compound (0.18g) as a white solid.

Example 56

(2,3-Dihydro-1,1-dioxobenzothiophene-3-yl)acetyl-Phe-Leu-ACHPA 2-(3-pyridyl)ethylamide N-oxide

This material was formed from (2,3-dihydro-1,1-dioxobenzothiophene-3-yl)acetyl-Phe-Leu-ACHPA 2-(3-pyridyl)ethylamide (0.18g) (Example 55(a)), following the procedure of Example 30. The crude product was chromatographed on silica gel using methanol/chloroform (0-10% methanol, gradient). This gave the title compound (0.18g) as a white solid.

Biological Data

In vitro human renin inhibition

Renin inhibitory activity was estimated as the percentage change in renin activity in human plasma in the presence and absence of compound. The source of plasma was blood taken from healthy volunteers. Renin activity was defined by the difference in angiotensin I levels between two halves of a sample, one incubated at 37° and the other at 4° for 2h. Angiotensin I levels were measured using a $^{125}$I- angiotensin I radioimmunoassay kit (NEN/DuPont, Stevenage). Results were calculated as the mean of at least two, duplicate determinations and $IC_{50}$ values were calculated by linear regression analysis of at least three concentrations of compound.

The results were as follows:

| Compound | $IC_{50}$(x$10^{-9}$-M) |
|---|---|
| EI (a) | 15 |
| E3 | 27 |
| E6 | 87 |
| E13 | 32 |
| E14 | 37 |
| E18 | 30 |
| E19 | 45 |
| E22 | 95 |
| E34a | 12 |
| E37 | 44 |
| E41 | 43 |
| E43 | 12 |
| E44 | 48 |
| E50a | 14 |
| E51 | 53 |
| E52 | 20 |

**Claims**

1. A compound of formula (I), or a pharmaceutically acceptable salt thereof:

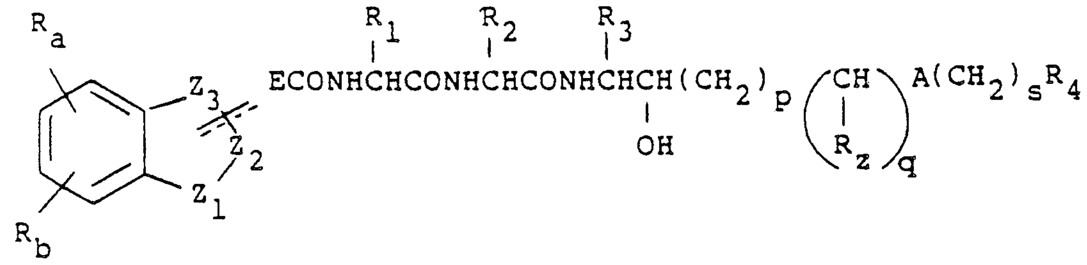

(I)

wherein

$Z_1$, $Z_2$, $Z_3$ and the carbon atoms to which $Z_1$ and $Z_3$ are attached, form a 5-membered non-aromatic heterocyclic ring;

E is absent or is $(CH_2)n$ or $CH(CH_2)_{n-1}$ wherein n is 1 to 4;

A is -CONH-, -NHCO-, -COO- $-S(O)_r-$ wherein r is 0, 1, or 2, or $-CH_2-$;

p is 0, 1 or 2;

q is 0 or 1;

s is 0, 1, 2, 3 or 4;

$R_z$ is hydrogen, $C_{1-6}$ alkyl or, when A is $-CH_2-$, hydroxy;

$R_a$ and $R_b$ are independently selected from hydrogen or a substituent;

$R_1$ is $CH_2R_9$ wherein $R_9$ is optionally substituted aryl or heteroaryl;

$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;

$R_3$ is $CH_2R_{12}$ wherein $R_{12}$ is $C_{1-6}$ alkyl, $C_{3-8}$ cycloalkyl or phenyl; and

$R_4$ is a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NMR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and

the dashed line represents an optional bond (when E is present).

2. A compound according to claim 1 wherein one of $Z_1$, $Z_2$ and $Z_3$ is attached to E and is N, CH or C (wherein the optional exocyclic bond is present); the second of $Z_1$, $Z_2$ and $Z_3$ is O, S, SO, $SO_2$ or NR wherein R is hydrogen or $C_{1-6}$ alkyl; and the third is O, S, SO, $SO_2$, NR, $CH_2$ or C=O provided that, one of $Z_1$ and $Z_2$, or one of $Z_2$ and $Z_3$, is $CH_2$, CH or C when the other is S or O, or is $CH_2$, CH, C, NR or N when the other is SO or $SO_2$; and $Z_2$ is SO, $SO_2$ or C=O when one of $Z_1$ and $Z_3$ is N and the other is O, S or NR; or $Z_1$ is CO, $Z_2$ is O and $Z_3$ is CH.

3. A compound according to claim 2, $Z_1$ is $SO_2$ or O, $Z_2$ is CH, $Z_3$ is $CH_2$ and E is attached at $Z_2$; wherein $Z_1$ is $SO_2$ or O, $Z_2$ is CH and E is attached at $Z_3$; or $Z_1$ is CO, $Z_2$ is N and $Z_3$ is CH and E is attached at $Z_2$.

4. A compound according to any one of claims 1 to 3 wherein $R_a$ and $R_b$ are selected from hydrogen, halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or $NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy.

5. A compound according to claim 4 wherein one of $R_a$ and $R_b$ is hydrogen and the other is $CH_2NH_2$, $CO_2H$, $CH_2NHCH_2CO_2H$ or $SO_2CH_2CO_2H$.

6. A compound according to any one of claims 1 to 5 wherein the amino acid residue containing $R_2$ is Leu, $\beta$-pyrazolylalanine or His.

7. A compound according to claim 1

wherein

E is $(CH_2)_n$ wherein n is 0, 1 or 2;

$Z_1$ is O, S, SO, $SO_2$ or NR wherein R is hydrogen or $C_{1-6}$ alkyl;

$Z_2$ is $CR_xR_y$ wherein $R_x$ is hydrogen and $R_y$ is hydrogen or $R_y$ is attached to E in formula (I), or $R_x$ and $R_y$ together are an oxo group;
$Z_3$ is $CH_2$ or is CH attached to E in formula (I);
$R_{11}$ is $C_{1-6}$ alkyl, $C_{3-8}$ cycloalkyl, or optionally substituted aryl or heteroaryl; and $R_4$ is a saturated or unsaturated heterocyclic ring linked through carbon, hydroxy, $C_{1-6}$ alkoxy, $C_{1-6}$ alkanoyloxy, amino, $C_{1-7}$ alkanoylamino, amino substituted by one or two $C_{1-6}$ alkyl groups, or $C_{1-6}$ alkylsulphonyl, or (when s is 2 to 4) $R_4$ is a saturated or unsaturated heterocyclic ring linked through nitrogen; and
the remaining variables are as defined in claim 1.

8. A compound according to claim 1 of formula (IA) or a pharmaceutically acceptable salt thereof:

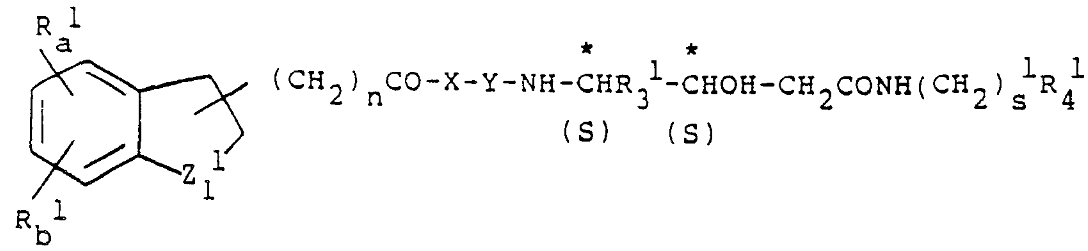

(IA)

wherein
X is Phe or NAla;
Y is Leu, Ile, Nle or His;
$Z_1^1$ is O, S, SO or $SO_2$;
n is 0, 1, 2 or 3;
$s^1$ is 2 or 3;
$R_a^1$ and $R_b^1$ are as defined for $R_a$ and $R_b$ in claim 4.

9. A compound according to any one of claims 1 to 8 wherein E is $(CH_2)_n$ wherein n is 1, 2 or 3 and E is attached at the 3-position with respect to $Z_1$.

10. A compound according to any one of claims 1 to 9 wherein the sequence from the amino acid containing $R_3'$ or $R_3^1$ to $NH(CH_2)_sR_4$, is 4(S)-amino-5-cyclohexyl-3(S)- hydroxypentanoic acid (ACHPA).

11. A compound according to any one of claims 1 to 10 wherein s is 2, 3 or 4 and $R_4$, is 1-imidazolyl, 2-imidazolyl, 4-morpholinyl, 2-oxopyrrolidin-1-yl, 2-pyridyl, 2-pyridyl-N-oxide, 3-pyridyl, hydroxyethyl, acetylamino, 4-methylpiperazin-1-yl, piperazin-1-yl or dimethylamino.

12. A compound selected from the group consisting of:
(2,3-dihydro-1,1-dioxobenzothiophene-3-yl)acetyl-Phe-Leu-ACHPA 3-(1-imidazolyl)propylamide,
(2,3-dihydro-1,1-dioxobenzothiophene-3-yl)acetyl-Phe-Leu-ACHPA 2-(4-morpholinyl)ethylamide,
(2,3-dihydro-1,1-dioxobenzothiophene-3-yl)acetyl-Phe-Leu-ACHPA 3-(2-oxopyrrolidin-1-yl)propylamide, (2,3-dihydro-1,1-dioxobenzothiophene-3-yl)acetyl-Phe-Leu-ACHPA 2-(2-pyridyl)ethylamide,
(2,3-dihydro-1,1-dioxobenzothiophene-3-yl)acetyl-Phe-Leu-ACHPA 2-hydroxyethylamide,
(2,3-dihydro-1,1-dioxobenzothiophene-3-yl)acetyl-Phe-Leu-ACHPA 2-(acetylamino)ethylamide,
(2,3-dihydro-1,1-dioxobenzothiophene-3-yl)acetyl-Phe-Leu-ACHPA-$NH(CH_2)_3CO_2Et$,
(2,3-dihydro-1,1-dioxobenzothiophene-3-yl)acetyl-Phe-Leu-ACHPA-$NH(CH_2)_3CO_2H$,
(2,3-dihydro-1,1-dioxobenzothiophene-3-yl)acetyl-Phe-Leu-ACHPA-$NH(CH_2)_3$-(S)-$CH(NH_2)CO_2H$,
(2,3-dihydro-1,1-dioxobenzothiophene-3-yl)acetyl-Phe-Leu-ACHPA-$NH(CH_2)_4$-(5)-$CH(NH_2)CO_2H$,
(6-(BOC-aminomethyl)-2,3-dihydro-1,1-dioxobenzothiophene-3-yl)acetyl-Phe-Leu-ACHPA-$NH(CH_2)_3CO_2Et$,
(5-(BOC-aminomethyl)-2,3-dihydrobenzofuran-3-yl)acetyl-Phe-Leu-ACHPA 3-(1-imidazolyl)propylamide,
(5-(aminomethyl)-2,3-dihydrobenzofuran-3-yl)acetyl-Phe-Leu-ACHPA 3-(1-imidazolyl)propylamide,
(2,3-dihydro-1,1-dioxobenzothiophene-3-yl)acetyl-Phe-Leu-ACHPA 4-(1-imidazolyl)butylamide,
(2,3-dihydro-1,1-dioxobenzothiophene-3-yl)acetyl-Phe-Leu-ACHPA 2-(1-imidazolyl)ethylamide,
(5-(benzyloxycarbonyl)-2,3-dihydrobenzofuran-3-yl)acetyl-Phe-Leu-ACHPA 3-(1-imidazolyl)propylamide,
(5-carboxy-2,3-dihydrobenzofuran-3-yl) acetyl-Phe-Leu-ACHPA 3-(1-imidazolyl)propylamide,
(2,3-dihydro-1,1-dioxobenzothiophene-3-yl)acetyl-Phe-Leu-ACHPA-$NH(CH_2)_4CO_2Me$,
(2,3-dihydro-1,1-dioxobenzothiophene-3-yl) acetyl-Phe-Leu-ACHPA-$NH(CH_2)_4CO_2H$,
(2,3-dihydro-1,1-dioxobenzothiophene-3-yl)acetyl-Phe-Leu-ACHPA 4-(4-methylpiperazin-1-yl)butylamide,
(2,3-dihydro-1,1-dioxobenzothiophene-3-yl)acetyl-Phe-Leu-ACHPA 3-(4-methylpiperazin-1-yl)propylamide,

(2,3-dihydro-1,1-dioxobenzothiophene-3-yl)acetyl-Phe-Leu-ACHPA 2-(4-methylpiperazin-1-yl)ethylamide, (2,3-dihydro-1,1-dioxobenzothiophene-3-yl)acetyl-Phe-Leu-ACHPA 3-(dimethylamino)propylamide, (2,3-dihydro-3-oxo-1H-isoindole-1-acetyl)-Phe-Leu-ACHPA-NH$(CH_2)_3CO_2CH_2$Ph, (2,3-dihydro-3-oxo-1H-isoindole-1-acetyl)-Phe-Leu-ACHPA-NH$(CH_2)_3CO_2$H, (2,3-dihydro-3-oxo-1H-isoindole-1-acetyl)-Phe-Leu-ACHPA 3-(1-imidazolyl)propylamide, (2,3-dihydro-1,1-dioxobenzothiophene-3-yl)acetyl-Phe-Leu-ACHPA-NH$(CH_2)_2CO_2CH_2$Ph, (2,3-dihydro-1,1-dioxobenzothiophene-3-yl)acetyl-Phe-Leu-ACHPA-NH$(CH_2)_2CO_2$H, (2,3-dihydro-1,1-dioxobenzothiophene-3-yl)acetyl-Phe-Leu-ACHPA-NH$(CH_2)_2$-(S)-CH$(NH_2)CO_2$H, (2,3-dihydro-1,1-dioxobenzothiophene-3-yl)acetyl-Phe-Leu-ACHPA 2-(2-pyridyl)ethylamide N-oxide, (6-nitro-2,3-dihydro-1,1-dioxobenzothiophene-3-yl)acetyl-Phe-Leu-ACHPA 3-(1-imidazolyl)propylamide, (6-amino-2,3-dihydro-1,1-dioxobenzothiophene-3-yl)acetyl-Phe-Leu-ACHPA 3-(1-imidazolyl)propylamide, 4-(6-nitro-2,3-dihydro-1,1-dioxobenzothiophene-2-yl)butanoyl-Phe-Leu-ACHPA 3-(1-imidazolyl)propylamide, 4-(6-amino-2,3-dihydro-1,1-dioxobenzothiophene-2-yl)butanoyl-Phe-Leu-ACHPA 3-(1-imidazolyl)propylamide, (6-(BOC-aminomethyl)-2,3-dihydro-1,1-dioxobenzothiophene-3-yl)acetyl-Phe-Leu-ACHPA-NH$(CH_2)_3CO_2CH_2$Ph, (6-(BOC-aminomethyl)2,3-dihydro-1,1-dioxobenzothiophene-3-yl)acetyl-Phe-Leu-ACHPA-MH$(CH_2)_3CO_2$H, (6-(aminomethyl)-2,3-dihydro-1,1-dioxobenzothiophene-3-yl)acetyl-Phe-Leu-ACHPA-NH$(CH_2)_3CO_2$H, (5-(BOC-aminomethyl)-2,3-dihydrobenzofuran-3-yl)acetyl-Phe-Leu-ACHPA-NH$(CH_2)_3CO_2CH_2$Ph, (5-BOC-aminomethyl)-2,3-dihydrobenzofuran-3-yl)acetyl-Phe-Leu-ACHPA-NH$(CH_2)_3CO_2$H, (5-(aminomethyl)-2,3-dihydrobenzofuran-3-yl)acetyl-Phe-Leu-ACHPA-NH$(CH_2)_3CO_2$H, (2,3-dihydro-2-oxo-1H-benzimidazole-1-propanoyl)-Phe-Leu-ACHPA 3-(1-imidazolyl)propylamide, (5-(BOC-aminomethyl)-2,3-dihydro-1,1-dioxobenzothiophene-3-yl)acetyl-Phe-Leu-ACHPA 3-(1-imidazolyl)-propylamide, (5-(aminomethyl)-2,3-dihydro-1,1-dioxobenzothiophene-3-yl)acetyl-Phe-Leu-ACHPA 3-(1-imidazolyl)-propylamide, (5-(carboxymethylaminomethyl)-2,3-dihydrobenzofuran-3-yl)acetyl-Phe-Leu-ACHPA 3-(1-imidazolyl)-propylamide, (5-(BOC-aminomethyl)-2,3-dihydro-1,1-dioxobenzothiophene-3-yl)acetyl-Phe-His-ACHPA-NH$(CH_2)_3CO_2CH_2$Ph, (5-(BOC-aminomethyl)-2,3-dihydro-1,1-dioxobenzothiophene-3-yl) acetyl-Phe-His-ACHPA-NH$(CH_2)_3CO_2$H, (5-(aminomethyl)-2,3-dihydro-1,1-dioxobenzothiophene-3-yl)acetyl-Phe-His-ACHPA-NH$(CH_2)_3CO_2$H, (5-(BOC-aminomethyl)-2,3-dihydro-1,1-dioxobenzothiophene-3-yl)acetyl-Phe-His-ACHPA 3-(1-imidazolyl)-propylamide, (5-(aminomethyl)-2,3-dihydro-1,1-dioxobenzothiophene-3-yl)acetyl-Phe-His-ACHPA 3-(1-imidazolyl)-propylamide, 4-(2,3-dihydro-1,1-dioxobenzothiophene-2-yl) butanoyl-Phe-Leu-ACHPA 3-(1-imidazolyl)propylamide, (6-(BOC-aminomethyl)-2,3-dihydro-1,1-dioxobenzothiophene-3-yl)acetyl-Phe-Leu-ACHPA 3-(1-imidazolyl)-propylamide, (6-(aminomethyl)-2,3-dihydro-1,1-dioxobenzothiophene-3-yl)acetyl-Phe-Leu-ACHPA 3-(1-imidazolyl)-propylamide, 4-(1,3-dihydro-1-oxo-2H-isoindol-2-yl)butanoyl-Phe-Leu-ACHPA 3-(1-imidazolyl)propylamide, (2,3-dihydro-1,1-dioxobenzothiophene-3-yl)acetyl-Phe-Leu-ACHPA-NH$(CH_2)_3$-Pro methyl ester, (2,3-dihydro-1,1-dioxobenzothiophene-3-yl)acetyl-Phe-Leu-ACHPA 2-(3-pyridyl)ethylamide, (2,3-dihydro-1,1-dioxobenzothiophene-3-yl)acetyl-Phe-Leu-ACHPA 2-(3-pyridyl)ethylamide N-oxide, (2,3-dihydro-1,1-dioxobenzothiophene-3-yl)acetyl-Phe-Leu-ACHPA 2-(4-pyridyl)ethylamide, (2,3-dihydro-1,1-dioxobenzothiophene-3-yl)acetyl-Phe-Leu-ACHPA 2-(4-pyridyl)ethylamide N-oxide, (2,3-dihydro-1,1-dioxobenzothiophene-3-yl)acetyl-Phe-Leu-ACHPA 3(2-imidazolyl)propylamide, (2,3-dihydro-1,1-dioxoben zothiophene-3-yl)acetyl-Phe-Leu-ACHPA 3-(2-pyridyl)propylamide, (2,3-dihydro-1,1-dioxobenzothiophene-3-yl)acetyl-Phe-Leu-ACHPA 3-(2-pyridyl)propylamide N-oxide, (2,3-dihydro-1,1-dioxobenzothiophene-3-yl)-acetyl-Phe-Leu-ACHPA 3-(N-prolinyl)propylamide, (2,3-dihydro-1,1-dioxobenzothiophene-3-yl)acetyl-Phe-Leu-ACHPA 4-(piperazin-1-yl)butylamide, and pharmaceutically acceptable salts of any one of the foregoing compounds.

13. A process for the preparation of a compound according to claim 1, which process comprises reacting a reagent of the formula (III):

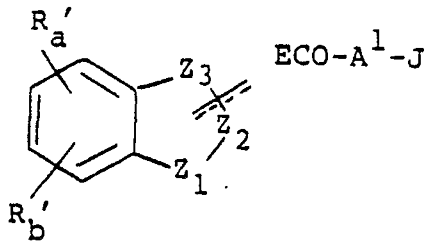

(III)

wherein $R_a'$ and $R_b'$ are $R_a$ and $R_b$ respectively or groups or atoms convertible thereto; $A^1$ is absent or represents an appropriate amino acid or dipeptide unit, J is OH or a leaving groups and the remaining variables are as hereinbefore defined; with a reagent of the formula (IV):

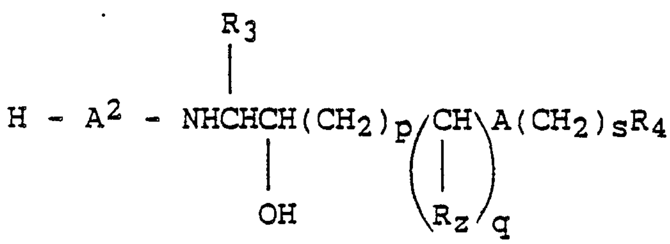

(IV)

wherein

$A^2$ is absent or represents an appropriate amino acid or dipeptide unit such that $A^1$ + $A^2$ is $-NHCHR_1CONHCHR_2CO-$and the remaining variables are as hereinbefore defined; and thereafter, if desired or necessary deprotecting (within $A^1$ or $A^2$) of the products, and/or converting $Z_1$, $Z_2$ or $Z_3$ to other $Z_1$, $Z_2$ or $Z_3$, $R_a'$ /$R_b'$ to $R_a$ and/or $R_b$ and/or forming a pharmaceutically acceptable salt thereof.

14. A pharmaceutical composition comprising a compound according to any one of claims 1 to 12, and a pharmaceutically acceptable carrier.

15. A compound according to any one of claims 1 to 12 for use as an active therapeutic substance.

16. A compound according to any one of claims 1 to 12 for use in the treatment of hypertension.

17. Use of a compound according to any one of claims 1 to 12 in the manufacture of a medicament for use in the treatment of hypertension.

Claims for the following Contracting State: ES

1. A process for the preparation of a compound of formula (I), or a pharmaceutically acceptable salt thereof:

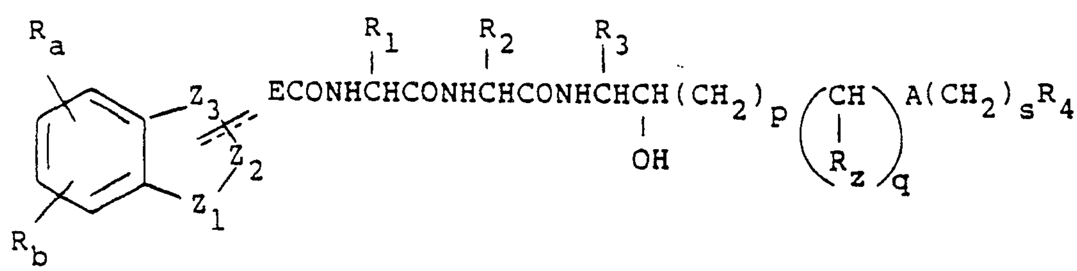

(I)

wherein

$Z_1$, $Z_2$, $Z_3$ and the carbon atoms to which $Z_1$ and $Z_3$ are attached, form a 5-membered non-aromatic heterocyclic ring;

E is absent or is $(CH_2)_n$ or $CH(CH_2)_{n-1}$ wherein n is 1 to 4;

A is -CONH-, -NHCO-, -COO- -S(O)$_r$- wherein r is 0, 1, or 2, or -$CH_2$-;
p is 0, 1 or 2;
q is 0 or 1;
s is 0, 1, 2, 3 or 4;
$R_z$ is hydrogen, $C_{1-6}$ alkyl or, when A is -$CH_2$-, hydroxy;
$R_a$ and $R_b$ are independently selected from hydrogen or a substituent;
$R_1$ is $CH_2R_9$ wherein $R_9$ is optionally substituted aryl or heteroaryl;
$R_2$ is $CHR_{10}R_{11}$ wherein $R_{10}$ is hydrogen or methyl and $R_{11}$ is $C_{1-6}$ alkyl, $C_{3-8}$ cycloalkyl, optionally substituted aryl or heteroaryl, or $R_{11}$ is amino, $C_{2-7}$ alkanoylamino, 2-oxopyrrolidinyl, 2-oxopiperidinyl or $C_{1-6}$ alkoxycarbonylamino;
$R_3$ is $CH_2R_{12}$ wherein $R_{12}$ is $C_{1-6}$ alkyl, $C_{3-8}$ cycloalkyl or phenyl; and
$R_4$ is a saturated or unsaturated heterocyclic ring linked through carbon, hydroxy, $C_{1-6}$ alkoxy, $C_{1-7}$ alkanoyloxy, amino, $C_{1-7}$ alkanoylamino, amino substituted by one or two $C_{1-6}$ alkyl groups, $C_{1-6}$ alkylsulphonyl, carboxy, $C_{1-6}$ alkoxycarbonyl, benzyloxycarbonyl, aminocarbonyl or $CH(NHR_{13})CO_2R_{14}$ wherein $R_{13}$ is hydrogen or $C_{1-6}$ alkanoyl and $R_{14}$ is hydrogen or $C_{1-6}$ alkyl; or (when s is 2 to 4) $R_4$ is a saturated or unsaturated heterocyclic ring linked through nitrogen; and
the dashed line represents an optional bond (when E is present);
which process comprises reacting a reagent of the formula (III):

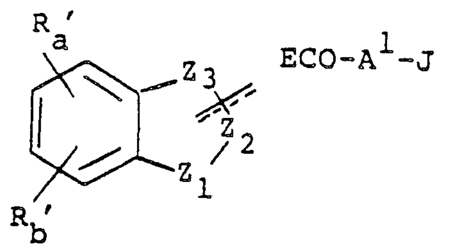

(III)

wherein $R_a'$ and $R_b'$ are $R_a$ and $R_b$ respectively or groups or atoms convertible thereto;
$A^1$ is absent or represents an appropriate amino acid or dipeptide unit, J is OH or a leaving group; and the remaining variables are as hereinbefore defined; with a reagent of the formula (IV):

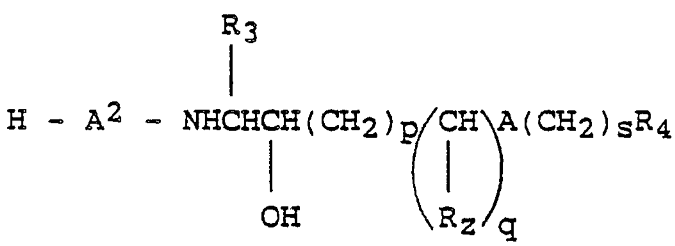

(IV)

wherein
$A^2$ is absent or represents an appropriate amino acid or dipeptide unit such that $A^1$ + $A^2$ is -$NHCHR_1CONHCHR_2CO$-and the remaining variables are as hereinbefore defined; and thereafter, if desired or necessary deprotecting (within $A^1$ or $A^2$) of the products, and/or converting $Z_1$, $Z_2$ or $Z_3$ to other $Z_1$, $Z_2$ or $Z_3$, $R_a'$/$R_b'$ to $R_a$ and/or $R_b$ and/or forming a pharmaceutically acceptable salt thereof.

2. A process according to claim 1 wherein one of $Z_1$, $Z_2$ and $Z_3$ is attached to E and is N, CH or C (wherein the optional exocyclic bond is present); the second of $Z_1$, $Z_2$ and $Z_3$ is O, S, SO, $SO_2$ or NR wherein R is hydrogen or $C_{1-6}$ alkyl; and the third is O, S, SO, $SO_2$, NR, $CH_2$ or C=O provided that, one of $Z_1$ and $Z_2$, or one of $Z_2$ and $Z_3$, is $CH_2$, CH or C when the other is S or O, or is $CH_2$, CH, C, NR or N when the other is SO or $SO_2$; and $Z_2$ is SO, $SO_2$ or C=O when one of $Z_1$ and $Z_3$ is N and the other is O, S or NR; or $Z_1$ is CO, $Z_2$ is O and $Z_3$ is CH.

3. A process according to claim 2, $Z_1$ is $SO_2$ or O, $Z_2$ is CH, $Z_3$ is $CH_2$ and E is attached at $Z_2$; wherein $Z_1$ is $SO_2$ or O, $Z_2$ is CH and E is attached at $Z_3$; or $Z_1$ is CO, $Z_2$ is N and $Z_3$ is CH and E is attached at $Z_2$.

4. A process according to any one of claims 1 to 3 wherein $R_a$ and $R_b$ are selected from hydrogen, halogen, $C_{1-6}$ alkyl, $C_{1-6}$ alkoxy, nitro, cyano, SH, $SO_3H$, CHO, $C_{1-6}$ alkyl substituted by hydroxy, $C_{1-6}$ alkanoyloxy, optionally substituted amino or by $C_{1-7}$ alkanoylamino, a group $NR_5R_6$, $SO_2NR_5R_6$, $CO_2R_5$, $NHCONR_5R_6$ or $NHCOR_5$ wherein $R_5$ is hydrogen, $C_{1-6}$ alkyl or optionally substituted benzyl and $R_6$ is hydrogen or $C_{1-6}$ alkyl, a group $S(O)_mR_7$ wherein m is 0, 1 or 2 and $R_7$ is hydrogen or $C_{1-6}$ alkyl optionally substituted by amino, acylamino, protected amino, or $CO_2H$ or a pharmaceutically acceptable ester (such as a $C_{1-6}$ alkyl ester) thereof; or a group $CH=NR_8$ wherein $R_8$ is $C_{1-6}$ alkyl optionally substituted by amino or hydroxy.

5. A process according to claim 4 wherein one of $R_a$ and $R_b$ is hydrogen and the other is $CH_2NH_2$, $CO_2H$, $CH_2NHCH_2CO_2H$ or $SO_2CH_2CO_2H$.

6. A process according to any one of claims 1 to 5 wherein the amino acid residue containing $R_2$ is Leu, β-pyrazolylalanine or His.

7. A process according to claim 1 wherein

E is $(CH_2)_n$ wherein n is 0, 1 or 2;

$Z_1$ is O, S, SO, $SO_2$ or NR wherein R is hydrogen or $C_{1-6}$ alkyl;

$Z_2$ is $CR_xR_y$ wherein $R_x$ is hydrogen and $R_y$ is hydrogen or $R_y$ is attached to E in formula (I), or $R_x$ and $R_y$ together are an oxo group;

$Z_3$ is $CH_2$ or is CH attached to E in formula (I);

$R_{11}$ is $C_{1-6}$ alkyl, $C_{3-8}$ cycloalkyl, or optionally substituted aryl or heteroaryl; and

$R_4$ is a saturated or unsaturated heterocyclic ring linked through carbon, hydroxy, $C_{1-6}$ alkoxy, $C_{1-6}$ alkanoyloxy, amino, $C_{1-7}$ alkanoylamino, amino substituted by one or two $C_{1-6}$ alkyl groups, or $C_{1-6}$ alkylsulphonyl, or (when s is 2 to 4) $R_4$ is a saturated or unsaturated heterocyclic ring linked through nitrogen; and

the remaining variables are as defined in claim 1.

8. A process according to claim 1 for the preparation of a compound of formula (IA) or a pharmaceutically acceptable salt thereof:

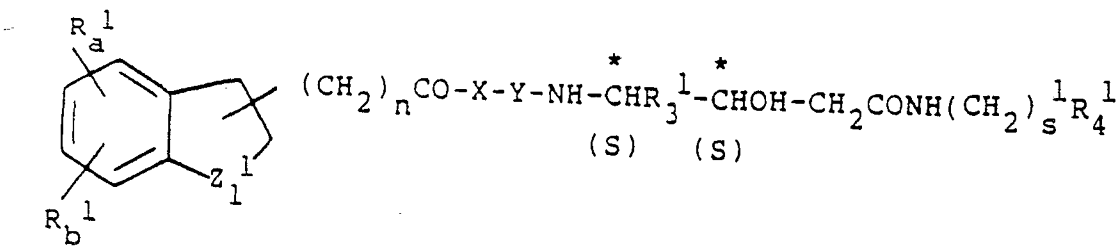

(IA)

wherein

X is Phe or NAla;

Y is Leu, Ile, Nle or His;

$Z_1^1$ is O, S, SO or $SO_2$;

n is 0, 1, 2 or 3;

$s^1$ is 2 or 3;

$R_a^1$ and $R_b^1$ are as defined for $R_a$ and $R_b$ in claim 4.

9. A process according to any one of claims 1 to 8 wherein E is $(CH_2)_n$ wherein n is 1, 2 or 3 and E is attached at the 3-position with respect to $Z_1$.

10. A process according to any one of claims 1 to 9 wherein the sequence from the amino acid containing $R_3$ or $R_3^1$ to $NH(CH_2)_sR_4$, is 4(S)-amino-5-cyclohexyl-3(S)-hydroxypentanoic acid (ACHPA).

11. A process according to any one of claims 1 to 10 wherein Ṡ is 2, 3 or 4 and $R_4$ is 1-imidazolyl, 2-imidazolyl, 4-morpholinyl, 2-oxopyrrolidin-1-yl, 2-pyridyl, 2-pyridyl-N-oxide, 3-pyridyl, hydroxyethyl, acetylamino, 4-methylpiperazin-1-yl, piperazin-1-yl or dimethylamino.

12. A process according to claim 1 for the preparation of a compound selected from the group consisting of:

(2,3-dihydro-1,1-dioxobenzothiophene-3-yl)acetyl-Phe-Leu-ACHPA 3-(1-imidazolyl)propylamide,

(2,3-dihydro-1,1-dioxobenzothiophene-3-yl)acetyl-Phe-Leu-ACHPA 2-(4-morpholinyl)ethylamide,

(2,3-dihydro-1,1-dioxobenzothiophene-3-yl)acetyl-Phe-Leu-ACHPA 3-(2-oxopyrrolidin-1-yl) propylamide,
(2,3-dihydro-1,1-dioxobenzothiophene-3-yl)acetyl-Phe-Leu-ACHPA 2-(2-pyridyl)ethylamide,
(2,3-dihydro-1,1-dioxobenzothiophene-3-yl)acetyl-Phe-Leu-ACHPA 2-hydroxyethylamide,
(2,3-dihydro-1,1-dioxobenzothiophene-3-yl)acetyl-Phe-Leu-ACHPA 2-(acetylamino)ethylamide,
(2,3-dihydro-1,1-dioxobenzothiophene-3-yl)acetyl-Phe-Leu-ACHPA-$NH(CH_2)_3CO_2Et$,
(2,3-dihydro-1,1-dioxobenzothiophene-3-yl)acetyl-Phe-Leu-ACHPA-$NH(CH_2)_3CO_2H$,
(2,3-dihydro-1,1-dioxobenzothiophene-3-yl)acetyl-Phe-Leu-ACHPA-$NH(CH_2)_3$-(S)-$CH(NH_2)CO_2H$,
(2,3-dihydro-1,1-dioxobenzothiophene-3-yl) acetyl-Phe-Leu-ACHPA-$NH(CH_2)_4$-(S)-$CH(NH_2)CO_2H$,
(6-(BOC-aminomethyl)-2,3-dihydro-1,1-dioxobenzothiophene-3-yl)acetyl-Phe-Leu-ACHPA-$NH(CH_2)_3CO_2Et$,
(5-(BOC-aminomethyl)-2,3-dihydrobenzofuran-3-yl)acetyl-Phe-Leu-ACHPA 3-(1-imidazolyl)propylamide,
(5-(aminomethyl)-2,3-dihydrobenzofuran-3-yl)acetyl-Phe-Leu-ACHPA 3-(1-imidazolyl)propylamide,
(2,3-dihydro-1,1-dioxobenzothiophene-3-yl)acetyl-Phe-Leu-ACHPA 4-(1-imidazolyl)butylamide,
(2,3-dihydro-1,1-dioxobenzothiophene-3-yl)acetyl-Phe-Leu-ACHPA 2-(1-imidazolyl)ethylamide,
(5-(benzyloxycarbonyl)-2,3-dihydrobenzofuran-3-yl)acetyl-Phe-Leu-ACHPA 3-(1-imidazolyl)propylamide,
(5-carboxy-2,3-dihydrobenzofuran-3-yl)acetyl-Phe-Leu-ACHPA 3-(1-imidazolyl)propylamide,
(2,3-dihydro-1,1-dioxobenzothiophene-3-yl)acetyl-Phe-Leu-ACHPA-$NH(CH_2)_4CO_2Me$,
(2,3-dihydro-1,1-dioxobenzothiophene-3-yl)acetyl-Phe-Leu-ACHPA-$NH(CH_2)_4CO_2H$,
(2,3-dihydro-1,1-dioxobenzothiophene-3-yl)acetyl-Phe-Leu-ACHPA 4-(4-methylpiperazin-1-yl)butylamide,
(2,3-dihydro-1,1-dioxobenzothiophene-3-yl)acetyl-Phe-Leu-ACHPA 3-(4-methylpiperazin-1-yl)propylamide,
(2,3-dihydro-1,1-dioxobenzothiophene-3-yl)acetyl-Phe-Leu-ACHPA 2-(4-methylpiperazin-1-yl)ethylamide,
(2,3-dihydro-1,1-dioxobenzothiophene-3-yl)acetyl-Phe-Leu-ACHPA 3-(dimethylamino)propylamide,
(2,3-dihydro-3-oxo-1H-isoindole-1-acetyl)-Phe-Leu-ACHPA-$NH(CH_2)_3CO_2CH_2Ph$,
(2,3-dihydro-3-oxo-1H-isoindole-1-acetyl)-Phe-Leu-ACHPA-$NH(CH_2)_3CO_2H$,
(2,3-dihydro-3-oxo-1H-isoindole-1-acetyl)-Phe-Leu-ACHPA 3-(1-imidazolyl)propylamide,
(2,3-dihydro-1,1-dioxobenzothiophene-3-yl)acetyl-Phe-Leu-ACHPA-$NH(CH_2)_2CO_2CH_2Ph$,
(2,3-dihydro-1,1-dioxobenzothiophene-3-yl)acetyl-Phe-Leu-ACHPA-$NH(CH_2)_2CO_2H$,
(2,3-dihydro-1,1-dioxobenzothiophene-3-yl)acetyl-Phe-Leu-ACHPA-$NH(CH_2)_2$-(S)-$CH(NH_2)CO_2H$,
(2,3-dihydro-1,1-dioxobenzothiophene-3-yl)acetyl-Phe-Leu-ACHPA 2-(2-pyridyl)ethylamide N-oxide,
(6-nitro-2,3-dihydro-1,1-dioxobenzothiophene-3-yl)acetyl-Phe-Leu-ACHPA 3-(1-imidazolyl)propylamide,
(6-amino-2,3-dihydro-1,1-dioxobenzothiophene-3-yl)acetyl-Phe-Leu-ACHPA 3-(1-imidazolyl)propylamide,
4′-(6-nitro-2,3-dihydro-1,1-dioxobenzothiophene-2-yl)butanoyl-Phe-Leu-ACHPA 3-(1-imidazolyl)propylamide,
4′-(6-amino-2,3-dihydro-1,1-dioxobenzothiophene-2-yl)butanoyl-Phe-Leu-ACHPA 3-(1-imidazolyl)-propylamide,
(6-(BOC-aminomethyl)-2,3-dihydro-1,1-dioxobenzothiophene-3-yl)acetyl-Phe-Leu-ACHPA-$NH(CH_2)$-$_3CO_2CH_2Ph$,
(6-(BOC-aminomethyl)-2,3-dihydro-1,1-dioxobenzothiophene-3-yl)acetyl-Phe-Leu-ACHPA-$NH(CH_2)_3CO_2H$,
(6-(aminomethyl)-2,3-dihydro-1,1-dioxobenzothiophene-3-yl)acetyl-Phe-Leu-ACHPA-$NH(CH_2)_3CO_2H$,
(5-(BOC-aminomethyl)-2,3-dihydrobenzofuran-3-yl)acetyl-Phe-Leu-ACHPA-$NH(CH_2)_3CO_2CH_2Ph$,
(5-BOC-aminomethyl)-2,3-dihydrobenzofuran-3-yl)acetyl-Phe-Leu-ACHPA-$NH(CH_2)_3CO_2H$,
(5-(aminomethyl)-2,3-dihydrobenzofuran-3-yl)acetyl-Phe-Leu-ACHPA-$NH(CH_2)_3CO_2H$,
(2,3-dihydro-2-oxo-1H-benzimidazole-1-propanoyl)-Phe-Leu-ACHPA 3-(1-imidazolyl)propylamide,
(5-(BOC-aminomethyl)-2,3-dihydro-1,1-dioxobenzothiophene-3-yl)acetyl-Phe-Leu-ACHPA 3-(1-imidazolyl)-propylamide,
(5-(aminomethyl)-2,3-dihydro-1,1-dioxobenzothiophene-3-yl)acetyl-Phe-Leu-ACHPA 3-(1-imidazolyl)-propylamide,
(5-(carboxymethylaminomethyl)-2,3-dihydrobenzofuran-3-yl)acetyl-Phe-Leu-ACHPA 3-(1-imidazolyl)-propylamide,
(5-(BOC-aminomethyl)-2,3-dihydro-1,1-dioxobenzothiophene-3-yl)acetyl-Phe-His-ACHPA-$NH(CH_2)$-$_3CO_2CH_2Ph$,
(5-(BOC-aminomethyl)-2,3-dihydro-1,1-dioxobenzothiophene-3-yl)acetyl-Phe-His-ACHPA-$NH(CH_2)_3CO_2H$,
(5-(aminomethyl)-2,3-dihydro-1,1-dioxobenzothiophene-3-yl)acetyl-Phe-His-ACHPA-$NH(CH_2)_3CO_2H$,
(5-(BOC-aminomethyl)-2,3-dihydro-1,1-dioxobenzothiophene-3-yl)acetyl-Phe-His-ACHPA 3-(1-imidazolyl)-propylamide,
(5-(aminomethyl)-2,3-dihydro-1,1-dioxobenzothiophene-3-yl)acetyl-Phe-His-ACHPA 3-(1-imidazolyl)-propylamide,
4-(2,3-dihydro-1,1-dioxobenzothiophene-2-yl)butanoyl-Phe-Leu-ACHPA 3-(1-imidazolyl)propylamide,
(6-(BOC-aminomethyl)-2,3-dihydro-1,1-dioxobenzothiophene-3-yl)acetyl-Phe-Leu-ACHPA 3-(1-imidazolyl)-propylamide,

(6-(aminomethyl)-2,3-dihydro-1,1-dioxobenzothiophene-3-yl)acetyl-Phe-Leu-ACHPA 3-(1-imidazolyl)-propylamide,
4-(1,3-dihydro-1-oxo-2H-isoindol-2-yl)butanoyl-Phe-Leu-ACHPA 3-(1-imidazolyl)propylamide,
(2,3-dihydro-1,1-dioxobenzothiophene-3-yl)acetyl-Phe-Leu-ACHPA-NH$(CH_2)_3$-Pro methyl ester,
(2,3-dihydro-1,1-dioxobenzothiophene-3-yl)acetyl-Phe-Leu-ACHPA 2-(3-pyridyl)ethylamide,
(2,3-dihydro-1,1-dioxobenzothiophene-3-yl)acetyl-Phe-Leu-ACHPA 2-(3-pyridyl)ethylamide N-oxide,
(2,3-dihydro-1,1-dioxobenzothiophene-3-yl)acetyl-Phe-Leu-ACHPA 2-(4-pyridyl)ethylamide,
(2,3-dihydro-1,1-dioxobenzothiophene-3-yl)acetyl-Phe-Leu-ACHPA 2-(4-pyridyl)ethylamide N-oxide,
(2,3-dihydro-1,1-dioxobenzothiophene-3-yl)acetyl-Phe-Leu-ACHPA 3-(2-imidazolyl)propylamide,
(2,3-dihydro-1,1-dioxobenzothiophene-3-yl)acetyl-Phe-Leu-ACHPA 3-(2-pyridyl)propylamide,
(2,3-dihydro-1,1-dioxobenzothiophene-3-yl)acetyl-Phe-Leu-ACHPA 3-(2-pyridyl)propylamide N-oxide,
(2,3-dihydro-1,1-dioxobenzothiophene-3-yl)acetyl-Phe-Leu-ACHPA 3-(N-prolinyl)propylamide,
(2,3-dihydro-1,1-dioxobenzothiophene-3-yl)acetyl-Phe-Leu-ACHPA 4-(piperazin-1-yl)butylamide,
and pharmaceutically acceptable salts of any one of the foregoing compounds.

13. Use of a compound of formula (I) as defined in any one of claims 1 to 12, or a pharmaceutically acceptable salt thereof, in the manufacture of a medicament for use in the treatment of hypertension.